# EUROPEAN PATENT SPECIFICATION

(11) **EP 1 855 696 B1**
(45) Date of publication and mention of the grant of the patent: **28.04.2010**
(21) Application number: 06727593.3
(22) Date of filing: 13.01.2006
(51) Int. Cl.: A61K 31/7052, A61P 33/06, C07H 17/08

(54) **9A-CARBAMOYL-Y-AMINOPROPYL- AND 9A-THIOCARBAMOYL-Y-AMINOPROPYL-AZALIDES WITH ANTIMALARIAL ACTIVITY**
9A-CARBAMOYL-Y-AMINOPROPYL- UND 9A-THIOCARBAMOYL-Y-AMINOPROPYL-AZALIDE MIT ANTIMALARIA-AKTIVITÄT
AZALIDES 9A-CARBAMOYL-Y-AMINOPROPYL ET 9A-THIOCARBAMOYLE-Y-AMINOPROPYL A ACTIVITE ANTIPALUDIQUE

(30) Priority: 14.01.2005 US 644360 P
(43) Date of publication of application: 21.11.2007
(73) Proprietor: GlaxoSmithKline istrazivacki centar Zagreb d.o.o., 10000 Zagreb (HR)
(72) Inventor: BUKVIC KRAJACIC, Mirjana, Zagreb 10000 (HR); KUJUNDZIC, Nedjeljko, Zagreb 10000 (HR); IVEZIC, Zrinka, 10000 Zagreb (HR); ALIHODZIC, Sulejman, 10000 Zagreb (HR); HUTINEC, Antun, Zagreb 10000 (HR); FAJDETIC, Andrea, Zagreb 10000 (HR)
(74) Representative: Crawley, Karen Anne
(86) International application number: PCT/IB2006/001227
(87) International publication number: WO 2006/085228

(56) References cited:
- WO-A-2004/052904
- WO-A-2006/075256
- ANDERSON S L ET AL: "PROPHYLAXIS OF PLASMODIUM FALCIPARUM MALARIA WITH AZITHROMYCIN ADMINISTERED TO VOLUNTEERS" ANNALS OF INTERNAL MEDICINE, NEW YORK, NY, US, vol. 123, no. 10, 15 November 1995 (1995-11-15), pages 771-773, XP008069262 ISSN: 0003-4819

## Description

### Field of the Invention

The present invention relates to novel 9a-N'-substituted-carbamoyl- and thiocarbamoyl-aminoalkyl-9a-aza-9-deoxo-9-dihydro-9a-homoerythromycin A and 3-*O*-Decladinosyl-9a-aza-9-deoxo-9-dihydro-9a-homoerythromycin A compounds having antimalarial activity. More particularly, the invention relates to 9a-N'-substituted-carbamoyl- and thiocarbamoyl-β-aminoethyl- or -γ-aminopropyl-9a-aza-9-deoxo-9-dihydro-9a-homoerythromycin A and 3-O-Decladinosyl-9a-aza-9-deoxo-9-dihydro-9a-homoerythromycin A compounds , to the method of preparation and to their use.

### Background of the Invention

Malaria is a serious infection. 200 to 300 million people are infected with malaria and two to three million people die from malaria every year. The disease is caused by a parasite (a protozoa of the Plasmodia genus), which is transmitted by the female *Anopheles* mosquito. There are four parasites hat can effect humans, *Plasmodium falciparum, P. vivax, P. ovale,* and *P. malariae.* A distinction is drawn between *Malaria tropica* (caused by *Plasmodium falciparum*), *Malaria tertiana* (caused by *Plasmodium vivax* or *Plasmodium ovale*) and *Malaria quartana* (caused *by Plasmodium malaria). Malaria tropica* is the most severe form of the disease, and is characterized by severe constitutional symptoms, and sometimes causes death.

Malaria is characterized by attacks of chills, fever, and sweating, occurring at intervals which depend on the time required for development of a new generation of parasites in the body. After recovery from the acute attack, the disease has a tendency to become chronic, with occasional relapses. The disease is prevalent in tropical and subtropical areas of the world including the Amazon region of Brazil, East and Southern Africa and Southeast Asia. The emergence of a malaria parasite resistant to chloroquine, is a drug heavily used as a panacea of malaria, has become a serious problem, and therefore, there is an urgent need to develop an effective remedy. Also, attempts to develop a malaria vaccine have failed. This compounds the urgent need to find an alternative drug-based approach to treating malaria.

Drugs of diverse chemical classes, such as chloroquine, mefloquine, halofantrine, and artemisinin, atovaquone/proguanil (Malarone™ ), doxycycline, and primaquine have been developed for the treatment of malaria. However, while marginally successful against some strains of malaria, most strains of malaria appear to have developed resistance not only to individual drugs but also to multiple combinations of drugs. Drugs which worked initially become totally ineffective after a period of time. An initial period of remission is often followed by a period during which nothing seems to be effective against the disease. This is known as multiple drug resistance, and it remains an issue in antimalarial drug development efforts. A malarial parasite which initially responds to treatment by one or more drugs becomes resistant to treatment not only using the drugs previously used, but many other antimalarial drugs. This further underscores the urgent necessity to find new compounds which show good efficacy against malaria and minimal toxicity.

In the last years several reports indicated that macrolides have potential for prophylactic as well as therapeutic use against malaria. Midecamycinin was studied in 1989 in two infectious models using *Plasmodium berghei* and *Plasmodium yoelii nigeriensis* (mouse) and *Plasmodium cynomolgi* (rhesus monkey) [S. K. Puri and G. P. Duti, Chemotherap.35 (1989) 187]. In both mouse models, the macrolide midecamycinin was active. The doses for *Plasmodium berghei* infection were significantly lower than for *Plasmodium yoelii nigeriensis*. In the monkey model, no efficacy was noted. In other investigations the animal model was challenged with azithromycin [S. K. Puri and N. Singh, Exp. Parasitol. 94 (2000) 8]. The dose regimen of 25-50 mg/kg reflects the same dose used for antibacterial treatment. Azithromycin worked in prophylactic and therapeutic dosing and in contrast to midecamycinin azithromycin was active also in the monkey model.

The efficacy of azithromycin in treating malarial infections was studied in Gambia [S. T. Sadiq et al, Lancet 1995 30, 346,881]. Children undergoing therapy for trachoma (Azithromycin is highly effective against *C. trachomatis*) were also examined for signs of malaria prophylaxis or therapeutic effects. A clear improvement of various indicators of malaria infection suggested a significant therapeutic benefit of azithromycin. The prophylactic efficacy of azithromycin was confirmed in Kenya [S. L. Anderson et al., Ann. Intern. Med. 123, 771]. A significant protection in adult volunteers was achieved with a better prophylaxis obtained through use of a daily dosing scheme of 250 mg versus a weekly regimen of 1000 mg. Also, in a double-blind, placebo-controlled trial with azithromycin in Irian Jaya in Indonesia [W. R. Taylor et al., Clin. Infect. Dis. 28 (1999) 522], the prophylactic efficacy in azithromycin treated non-immune patients was 71.6 % for *Plasmodium falciparum* and 98,9 % for *Plasmodium vivax* as compared to controls.

### Summary of the Invention

The scope of the present invention is defined by the appended claims.

New 9a-N'-substituted-carbamoyl- and thiocarbamoyl-β-aminoethyl- or -γ-aminopropyl-9a-aza-9-deoxo-9-dihydro-9a-homoerythromycin A and 3-O-Decladinosyl-9a-aza-9-deoxo-9-dihydro-9a-homoerythromycin A compounds represented by the Formula **(I):** 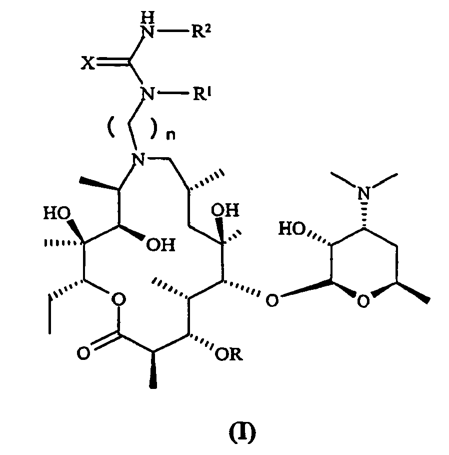 wherein
R represents H or a cladinosyl group of formula (II) 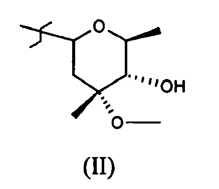
R¹ represents H, β-cyanoethyl, β-amidoethyl or β-(C₁₋₄alkoxycarbonyl)ethyl;
R² represents
   a) C₁₋₁₂ alkyl, wherein C₁₋₁₂ alkyl is
      i) uninterrupted or interrupted by 1-3 bivalent radical groups selected from -O-, -S- and -N(R³)- ; and/or
      ii) linear or branched, and unsubstituted or substituted by 1-3 groups selected from halogen (preferably fluoro, chloro or bromo); OH; NH₂; N-(C₁-C₄)alkylamino (preferably N-methylamino or N-ethylamino); N,N-di(C₁-C₄-alkyl)amino (preferably dimethylamino, diethylamino or di-isopropylamino); CN, NO₂; C(O)OC₁₋₄alkylaryl, (C₁-C₄-alkyl)thio ; a C₃-₁₄ membered carbocycle optionally substituted with one or more substituents selected from halogen, CN, C₁₋₄alkyl unsubstituted (preferably methyl or ethyl) or substituted with 1 to 3 halogen (preferably trifluoromethyl), O(C₁₋₄alkyl) optionally substituted with 1 to 3 halogen, S(C₁-C₄-alkyl) (preferably thiomethyl), O(C₃₋₆ cycloalkyl), O(C₁₋₄alkylaryl), C₁₋₄alkylcyano, C(O)C₁₋₄alkyl, C₁₋₄ alkyloxycarbonyl, optionally substituted aryl, optionally substituted heteroaryl, C(O)C₁₋₄alkylaryl, C(O)OC₁₋₄alkylaryl, NO₂, diazoaryl, sulfo- 5 or 6 membered carbocyclic or heterocyclic ring (preferably p-sulfopiperazyl), C₁₋₄alkyl-C(O)-O-C₁₋₄alkyl and C₁₋₄alkylO-C(O)-NR³; a C₃₋₁₄ membered saturated, unsaturated or aromatic heterocycle containing 1 to 3 heteroatoms selected from the group nitrogen, oxygen, sulphur optionally substituted with halogen, CN, C₁₋₄alkyl unsubstituted (preferably methyl or ethyl) or substituted with 1 to 3 halogen (preferably trifluoromethyl), O(C₁₋₄alkyl) optionally substituted with 1 to 3 halogen, S(C1-C₄-alkyl) (preferably thiomethyl), O(C₃₋₆ cycloalkyl), O(C₁₋₄alkylaryl), C₁₋₄alkylcyano, C(O)C₁₋₄alkyl, C(O)OC₁₋₄alkylaryl; C₁₋₄ alkyloxycarbonyl, optionally substituted aryl, optionally substituted heteroaryl, C(O)C₁₋₄alkylaryl, NO₂, diazoaryl, sulfo- 5 or 6 membered carbocyclic or heterocyclic ring (preferably p-sulfopiperazyl), C₁₋₄alkyl-C(O)-O-C₁₋₄alkyl and C₁₋₄alkylO-C(O)-NR³; or
   b) C₂₋₆ alkenyl containing 0, 1, 2, or 3 heteroatoms selected from O, S, or N, optionally substituted with one or more substituents selected from halogen; CN; NO₂; OH; NH₂; N-(C₁-C₄)alkylamino (preferably N-methylamino or N-ethylamino); N,N-di(C₁-C₄-alkyl)amino (preferably dimethylamino, diethylamino or di-isopropylamino); optionally substituted aryl; optionally substituted heteroaryl; or
   c) C₃₋₁₄ membered carbocycle which is unsubstituted or substituted by 1-3 groups selected from halogen; OH; CN; C₁₋₄alkyl unsubstituted (preferably methyl or ethyl) or substituted with 1 to 3 halogen (preferably trifluoromethyl) or CN group; O(C₁₋₄alkyl) optionally substituted with 1 to 3 halogen; aryloxy, S(C₁-C₄-alkyl) (preferably thiomethyl); O(C₃₋₆ cycloalkyl); O(C₁₋₄alkylaryl); C(O)C₁₋₄alkyl; C(O)OC₁₋₄alkylaryl; C₁₋₄ alkyloxycarbonyl; optionally substituted aryl; optionally substituted heteroaryl; C(O)C₁₋₄alkylaryl; NO₂; N-(C₁-C₄)alkylamino (preferably N-methylamino or N-ethylamino); N,N-di(C₁-C₄-alkyl)amino (preferably dimethylamino, diethylamino or di-isopropylamino), diazoaryl; sulfo- 5 or 6 membered carbocyclic or heterocyclic ring (preferably p-sulfopiperazyl); C₁₋₄alkyl-C(O)-O-C₁₋₄alkyl and C₁₋₄alkylO-C(O)-NR³; or
   d) C₃₋₁₄ membered saturated, unsaturated or aromatic heterocycle containing 1 to 3 heteroatoms selected from the group nitrogen, oxygen, sulphur optionally substituted by 1-3 groups selected from halogen; CN; C₁₋₄alkyl unsubstituted (preferably methyl or ethyl) or substituted with 1 to 3 halogen (preferably trifluoromethyl); O(C₁₋₄alkyl) optionally substituted with 1 to 3 halogen; aryloxy, S(C₁-C₄-alkyl) (preferably thiomethyl); O(C₃₋₆ cycloalkyl); O(C₁₋₄alkylaryl); C₁₋₄alkylcyano; C(O)C₁₋₄alkyl; C₁₋₄ alkyloxycarbonyl; optionally substituted aryl; optionally substituted heteroaryl; C(O)C₁₋₄alkylaryl; C(O)OC₁₋₄alkylaryl; NO₂; diazoaryl; 5 or 6 membered carbocyclic or heterocyclic ring; sulfo- 5 or 6 membered carbocyclic or heterocyclic ring (preferably p-sulfopiperazyl); C₁₋₄alkyl-C(O)-O-C₁₋₄alkyl and C₁₋₄alkylO-C(Q)-NR³;
   e) C(O)aryl;
      R³ represents H or C₁₋₄ alkyl;
      X represents O or S; and
      n is 2 or 3;
         wherein C₃₋₁₄ membered carbocycle means any stable 3, 4, 5, 6, 7, 8, 9, 10, 11, 12, 13 or 14-membered monocyclic, bicyclic or tricyclic ring, any of which may be saturated, unsaturated, or aromatic, fused or bridged, recognizing that rings with certain numbers of members cannot be bicyclic or tricyclic, and that when the ring is bridged, the substituents recited for the ring may also be present on the bridge;
         or a pharmaceutically acceptable salt, solvate or ester thereof;
         provided that when R¹ is H or β-cyanoethyl and n is 3, R² cannot be:
         a) uninterrupted and unsubstituted linear or branched C₁₋₄alkyl;
         b) -(CH₂)ₘ-Ar wherein m is 1-3 and wherein Ar is a C₃₋₁₀ membered monocyclic or fused bicyclic aromatic carbocycle which is optionally substituted with one or more substituents selected from halogen, unsubstituted O(C₁₋₄alkyl) or C₁₋₄alkyl unsubstituted or substituted with 1 to 3 halogen;
         c) -(CH₂)ₘ-Ar wherein m is 0 and wherein Ar is a C₃₋₁₀ membered monocyclic or fused bicyclic aromatic carbocycle which is unsubstituted or substituted by 1 to 3 substituents selected from halogen, unsubstituted O(C₁₋₄alkyl) or C₁₋₄alkyl unsubstituted or substituted with 1 to 3 halogen; or
         d) ethoxycarbonyl methyl.

The present invention also relates to pharmaceutically acceptable salts, solvates or esters of the Formula I compounds and to pharmaceutical compositions comprising the Formula I compounds.

The present invention also relates to the use of a compound of Formula I in the manufacture of a medicament for treating malarial diseases. Moreover, novel compounds of the present invention exhibit good efficacy against plasmodia, especially against multiresistent plasmodial species.

The present invention is also directed to compositions containing one or more of the foregoing compounds in an amount effective for therapeutic and/or prophylactic treatment of malaria in a subject in need of such treatment.

The present invention is also directed to the use of compounds of Formula I in the manufacture of a medicament for the prophylaxis of malaria or the treatment of subjects exposed to the malaria parasites.

This invention also discloses compositions containing one or more of the foregoing compounds in an amount effective to prevent or combat malarial disease; and

The present invention is also directed to the use of the compounds of Formula I for the manufacture of a medicament for preventing or combating malarial disease.

### Detailed Description of the Invention

In one particular embodiment, the present invention is directed to compounds represented by the formula (I), wherein
R represents H or cladinosyl group of formula (II) 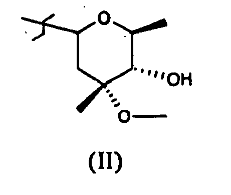
R¹ represents H, β-cyanoethyl, β-amidoethyl or β-(C₁₋₄alkoxycarbonyl)ethyl;
R² represents
   a) C₁₋₁₂ alkyl, wherein C₁₋₁₂ alkyl is linear or branched, and unsubstituted or substituted by 1-3 groups selected from halogen, N,N-di(C₁-C₄-alkyl)amino, C(O)OC₁₋₄alkylaryl, (C₁-C₄-alkyl)thio or; phenyl, naphthyl, furyl, cycloalkyl, thiophenyl, 3,4-methylenedioxyphenyl, morpholinyl, or piperidinyl each optionally substituted with one or more substituents selected from halogen, C₁₋₄alkyl unsubstituted or substituted with 1 to 3 halogen, O(C₁₋₄alkyl), O(C₃₋₆ cycloalkyl), O(C₁₋₄alkylaryl), C(O)C₁₋₄alkyl, C₁₋₄ alkyloxycarbonyl, optionally substituted aryl, optionally substituted heteroaryl, C(O)C₁₋₄alkylaryl, C(O)OC₁₋₄alkylaryl;
   b) an unsubstituted C₂₋₆ alkenyl; or
   c) C₁₋₁₂ cycloalkyl, adamantyl, norbornyl, norbornenyl, phenyl, indanyl, or naphthyl; any of which is unsubstituted or substituted by 1-3 groups selected from halogen; CN; C₁₋₄alkyl unsubstituted or substituted with 1 to 3 of halogen or CN group; O(C₁₋₄alkyl) optionally substituted with 1 to 3 halogen; S(C₁-C₄-alkyl); O(C₃₋₆ cycloalkyl); O(C₁₋₄alkylaryl); C(O)C₁₋₄alkyl; C₁₋₄ alkyloxycarbonyl; aryl; heteroaryl; NO₂; N,N-di(C₁-C₄-alkyl)amino, diazoaryl; and piperidinylsulfonamido; or
   d) dihydrobenzofuranyl, C₁₋₃ alkylenedioxyphenyl, benzopyranyl, furyl, isoxazolyl, piperidinyl, pyridinyl, thiophenyl, benzothiadiazolyl, tetrahydrobenzothiophenyl, optionally substituted by 1-3 groups selected from halogen; C₁₋₄alkyl unsubstituted or substituted with 1 to 3 halogen; C₁₋₄ alkyloxycarbonyl; optionally substituted aryl; optionally substituted heteroaryl; C(O)OC₁₋₄alkylaryl; or phenoxy;
   e) C(O)aryl;
X represents O or S; and
n is 2 or 3;
   or a pharmaceutically acceptable salt, solvate or ester thereof;
   provided that when R¹ is H or β-cyanoethyl and n is 3, R² cannot be:
   a) uninterrupted and unsubstituted linear or branched C₁₋₄alkyl;
   b) -(CH₂)ₘ-Ar wherein m is 1-3 and wherein Ar is a C₃₋₁₀ membered monocyclic or fused bicyclic aromatic carbocycle which is optionally substituted with one or more substituents selected from halogen, unsubstituted O(C₁₋₄alkyl) or C₁₋₄alkyl unsubstituted or substituted with 1 to 3 halogen; or
   c) -(CH₂)ₘ-Ar wherein m is 0 and wherein Ar is a C₃₋₁₀ membered monocyclic or fused bicyclic aromatic carbocycle which is unsubstituted or substituted by 1 to 3 substituents selected from halogen, unsubstituted O(C₁₋₄alkyl) or C₁₋₄alkyl unsubstituted or substituted with 1 to 3 halogen.

In a another particular embodiment, the present invention is directed to compounds represented by the formula (I) wherein
R represents H or cladinosyl group of formula (II) 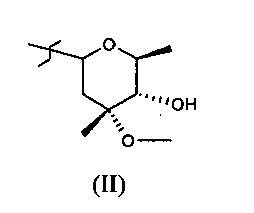
R¹ represents H, β-cyanoethyl, β-amidoethyl or β-(C₁₋₄alkoxycarbonyl)ethyl;
R² represents
   a) 3-phenylpropyl, β-phenylethyl, ethoxycarbonylmethyl, isopropyl, 1-(1-naphthyl)-ethyl, t-butyl, n-butyl, sec-butyl, benzyl, 2-furylmethyl, 4-methoxybenzyl, cyclohexylmethyl, ethyl, 2-(2-methyl-5,5-dimethyl)-pentyl, 2-(2-thophenyl)-ethyl, 3-thiomethylpropyl, 3,4-methylenedioxyphenylmethyl, N-morpholinylethyl, N-morpholinylpropyl, trityl, N-piperidinylethyl, 3-diethylaminopropyl, diphenylmethyl, 3-chloropropyl, isobutyl; or
   b) 2-propenyl; or
   c) cyclopentyl, cyclopropyl, cyclododecyl, norbornyl, norbomenyl, 2-benzyloxycyclohexyl, adamantyl, phenyl, 1-naphthyl, 4-chlorophenyl, 2-trifluoromethylphenyl, 3,4,5-trimethoxyphenyl, 2-naphthyl, 2,4-dichlorophenyl, 4-cyanophenyl, cyclohexyl, 4-ethylphenyl, 4-methoxyphenyl, 2-methyl-5-fluorophenyl, 4-cyanomethylphenyl, indanyl, 4-acetylphenyl, 2-phenylphenyl, 3-thiomethylphenyl, 3,5-dimethoxycarbonylphenyl, 4-methylphenyl, 2,4-dimethylphenyl, 3,4-difluorophenyl, 3-chlorophenyl, 3-fluorophenyl, 3-cyclopentoxy-4-methoxyphenyl, 4-benzyloxyphenyl, 2-ethylphenyl, 2,6-difluorophenyl, 4-nitrophenyl, 3,5-dichlorophenyl, 2-methoxy-4-nitrophenyl, ethoxycarbonylphenyl, 2-trifluoromethylphenyl, 4-phenylazophenyl, 4-diethylaminophenyl, 3-nitrophenyl, 3-chloro-4-trifluoromethylphenyl, 3,4-dichlorophenyl, 2,3,4-trifluorophenyl, 4-bromophenyl, 4-diazolylphenyl, 4-piperadylsulfonamidophenyl, 1-(4-dimethylamino)-naphthyl, 4-isopropylphenyl, 4-difluoromethoxyphenyl, or 2-methoxy-5-phenylphenyl; or
   d) 3,4-methylenedioxyphenyl, 6-fluorobenzo-1,3-pyranyl, dihydrobenzofuranyl, 3,4-propylenedioxyphenyl, 3-(2-trifluoromethyl-5-methyl)-furyl, 4-(3,5-dimethyl)-isoxazole, 4-(3-phenyl-5-methyl)-isoxazole, benzyloxycarbonylpiperidinyl, 4-(2,6-dichloro)-pyridinyl, 2-thiophenyl, benzothiadiazolyl, 3-(2-methoxycarbonyl)-thiophenyl, 2-(3-methoxycarbonyl)-tetrahydrobenzothiophenyl, pyridinyl, 5-(2-morpholinyl)-pyridinyl, 5-(2-phenoxy)-pyridinyl; or
   e) C(O)aryl;
X represents O or S; and
n is 2 or 3;
   or a pharmaceutically acceptable salt, solvate or ester thereof;
   provided that when R¹ is H or β-cyanoethyl and n is 3, R² cannot be:
   a) uninterrupted and unsubstituted linear or branched C₁₋₄alkyl;
   b) -(CH₂)ₘ-Ar wherein m is 1-3 and wherein Ar is a C₃₋₁₀ membered monocyclic or fused bicyclic aromatic carbocycle which is optionally substituted with one or more substituents selected from halogen, unsubstituted O(C₁₋₄alkyl) or C₁₋₄alkyl unsubstituted or substituted with 1 to 3 halogen;
   c) -(CH₂)ₘ-Ar wherein m is 0 and wherein Ar is a C₃₋₁₀ membered monocyclic or fused bicyclic aromatic carbocycle which is unsubstituted or substituted by 1 to 3 substituents selected from halogen, unsubstituted O(C₁₋₄alkyl) or C₁₋₄alkyl unsubstituted or substituted with 1 to 3 halogen; or
   d) ethoxycarbonylmethyl.
Preferred compounds of the invention are the compounds of Examples 1 to 203 and pharmaceutically acceptable salts, solvates or esters thereof.

The phrase "pharmaceutically acceptable", as used in connection with compositions of the invention, refers to molecular entities and other ingredients of such compositions that are physiologically tolerable and do not typically produce untoward reactions when administered to a mammal (e.g., human). Preferably, as used herein, the term "pharmaceutically acceptable" means approved by a regulatory agency of the Federal or a state government or listed in the U.S. Pharmacopoeia or other generally recognized pharmacopoeia for use in mammals, and more particularly in humans.

The term "carrier" applied to pharmaceutical compositions of the invention refers to a diluent, excipient, or vehicle with which an active compound is administered. Such pharmaceutical carriers can be sterile liquids, such as water, saline solutions, aqueous dextrose solutions, aqueous glycerol solutions, and oils, including those of petroleum, animal, vegetable or synthetic origin, such as peanut oil, soybean oil, mineral oil, sesame oil. However, since memantine is highly soluble, aqueous solutions are preferred. Suitable pharmaceutical carriers are described in "Remington's Pharmaceutical Sciences" by E.W. Martin, 18th Edition. Particularly preferred for the present invention are carriers suitable for immediate-release, i.e., release of most or all of the active ingredient over a short period of time, such as 60 minutes or less, and make rapid absorption of the drug possible.

The term "pharmaceutically acceptable derivative" as used herein means any pharmaceutically acceptable salt, solvate or prodrug, e.g. ester, of a compound of the invention, which upon administration to the recipient is capable of providing (directly or indirectly) a compound of the invention, or an active metabolite or residue thereof. Such derivatives are recognizable to those skilled in the art, without undue experimentation. Nevertheless, reference is made to the teaching of Burger's Medicinal Chemistry and Drug Discovery, 5th Edition, Vol 1: Principles and Practice, which is incorporated herein by reference to the extent of teaching such derivatives. Preferred pharmaceutically acceptable derivatives are salts, solvates, esters, carbamates and phosphate esters. Particularly preferred pharmaceutically acceptable derivatives are salts, solvates and esters. Most preferred pharmaceutically acceptable derivatives are salts and esters.

The compounds of the present invention may be in the form of and/or may be administered as a pharmaceutically acceptable salt. For a review on suitable salts see Berge et al., J. Pharm. Sci., 1977, 66, 1-19.

Typically, a pharmaceutical acceptable salt may be readily prepared by using a desired acid. The salt may precipitate from solution and be collected by filtration or may be recovered by evaporation of the solvent. For example, an aqueous solution of an acid such as hydrochloric acid may be added to an aqueous suspension of a compound of formula **(I)** and the resulting mixture evaporated to dryness (lyophilised) to obtain the acid addition salt as a solid. Alternatively, a compound of formula (I) may be dissolved in a suitable solvent, for example an alcohol such as isopropanol, and the acid may be added in the same solvent or another suitable solvent. The resulting acid addition salt may then be precipitated directly, or by addition of a less polar solvent such as diisopropyl ether or hexane, and isolated by filtration.

Suitable addition salts are formed from inorganic or organic acids which form non-toxic salts and examples are hydrochloride, hydrobromide, hydroiodide, sulphate, bisulphate, nitrate, phosphate, hydrogen phosphate, acetate, trifluoroacetate, maleate, malate, fumarate, lactate, tartrate, citrate, formate, gluconate, succinate, pyruvate, oxalate, oxaloacetate, trifluoroacetate, saccharate, benzoate, alkyl or aryl sulphonates (eg methanesulphonate, ethanesulphonate, benzenesulphonate or p-toluenesulphonate) and isethionate. Representative examples include trifluoroacetate and formate salts, for example the bis or tris trifluoroacetate salts and the mono or diformate salts, in particular the tris or bis trifluoroacetate salt and the monoformate salt.

Compounds of the invention may have both a basic and an acidic centre may therefore be in the form of zwitterions.

Those skilled in the art of organic chemistry will appreciate that many organic compounds can form complexes with solvents in which they are reacted or from which they are precipitated or crystallized. These complexes are known as "solvates". For example, a complex with water is known as a "hydrate". Solvates of the compound of the invention are within the scope of the invention. The salts of the compound of formula (I) may form solvates (e.g. hydrates) and the invention also includes all such solvates.

References hereinafter to a compound according to the invention include both compounds of Formula **(I)** and their pharmaceutically acceptable salts, solvates and esters.

With regard to stereoisomers, the compounds of Formula **(I)** have more than one asymmetric carbon atom. In the general Formula **(I)** as drawn, the solid wedge shaped bond indicates that the bond is above the plane of the paper. The broken bond indicates that the bond is below the plane of the paper.

It will be appreciated that the substituents on the macrolide may also have one or more asymmetric carbon atoms. Thus, the compounds of Formula **(I)** may occur as individual enantiomers or diastereomers. All such isomeric forms are included within the present invention, including mixtures thereof.

Separation of diastereoisomers may be achieved by conventional techniques, e.g. by fractional crystallisation, chromatography or H.P.L.C. A stereoisomeric mixture of the agent may also be prepared from a corresponding optically pure intermediate or by resolution, such as H.P.L.C., of the corresponding mixture using a suitable chiral support or by fractional crystallisation of the diastereoisomeric salts formed by reaction of the corresponding mixture with a suitable optically active acid or base, as appropriate.

The compounds of Formula **(I)** may be in crystalline or amorphous form. Furthermore, some of the crystalline forms of the compounds of Formula **(I)** may exist as polymorphs, which are included in the present invention.

The terms "C₁-C₄ alkyl" or "C₁-C₁₂ alkyl" as used herein, refer to saturated, straight or branched-chain hydrocarbon radicals containing between one and four, orone and twelve carbon atoms, respectively. Examples of "C₁-C₄ alkyl radicals include; methyl, ethyl, propyl, isopropyl, n-butyl, isobutyl, tert-butyl; and examples of C₁-C₁₂alkyl radicals include methyl, ethyl, propyl, isopropyl, n-butyl, isobutyl, tert-butyl, n-hexyl, neopentyl, octyl, decyl, dodecyl radicals.

The term "substituted alkyl" as used herein, refers to a "C₁-C₄ alkyl" or C₁-C₁₂ alkyl" group as previously defined, substituted by independent replacement of one, two, or three of the hydrogen atoms thereon with substituents including halogen (preferably fluoro, chloro or bromo); OH; NH₂; N-(C₁-C₄)alkylamino (preferably N-methylamino or N-ethylamino); N,N-di(C₁-C₄-alkyl)amino (preferably dimethylamino, diethylamino or di-isopropylamino); CN, NO₂; C(O)OC₁₋₄alkylaryl; a C₃₋₁₄ membered saturated, unsaturated or aromatic carbocycle optionally substituted with one or more substituents selected from halogen, CN, C₁₋₄alkyl unsubstituted (preferably methyl or ethyl) or substituted with 1 to 3 halogen (preferably trifluoromethyl), O(C₁₋₄alkyl) optionally substituted with 1 to 3 halogen, S(C₁-C₄-alkyl) (preferably thiomethyl), O(C₃₋₆ cycloalkyl), O(C₁₋₄alkylaryl), C₁₋₄alkylcyano, C(O)C₁₋₄alkyl, C₁₋₄ alkyloxycarbonyl, optionally substituted aryl, optionally substituted heteroaryl, C(O)C₁₋₄alkylaryl, C(O)OC₁₋₄alkylaryl, NO₂, diazoaryl, sulfo- 5 or 6 membered carbocyclic or heterocyclic ring (preferably p-sulfopiperazyl), C₁₋₄alkyl-C(O)-O-C₁₋₄alkyl and C₁₋₄alkylO-C(O)-NR³; a C₃₋₁₄ membered saturated, unsaturated or aromatic heterocycle containing 1 to 3 heteroatoms selected from the group nitrogen, oxygen, sulphur optionally substituted with halogen, CN, C₁₋₄alkyl unsubstituted (preferably methyl or ethyl) or substituted with 1 to 3 halogen (preferably trifluoromethyl), O(C₁₋₄alkyl) optionally substituted with 1 to 3 halogen, S(C₁-C₄-alkyl) (preferably thiomethyl), O(C₃₋₆ cycloalkyl), O(C₁₋₄alkylaryl), C₁₋₄alkylcyano, C(O)C₁₋₄alkyl, C(O)OC₁₋₄alkylaryl; C₁₋₄ alkyloxycarbonyl, optionally substituted aryl, optionally substituted heteroaryl, C(O)C₁₋₄alkylaryl, NO₂, diazoaryl, sulfo- 5 or 6 membered carbocyclic or heterocyclic ring (preferably p-sulfopiperazyl), C₁₋₄alkyl-C(O)-O-C₁₋₄alkyl and C₁₋₄alkylO-C(O)-NR³.

The term "C₂₋₆alkenyl" as used herein, denote a monovalent group derived from hydrocarbon moiety containing from 2 to 6 carbon atoms having at least one carbon-carbon double bond by the removal of a single hydrogen atom. Alkenyl groups include for example, ethenyl, propenyl, butenyl, 1-metyl-2-buten-1-yl.

The term "substituted C₂₋₆alkenyl", as used herein, refers to a "C₂₋₆alkenyl" group as previously defined, substituted by independent replacement of one, two or three of the hydrogen atoms thereon with substituents including halogen; CN; NO₂; OH; NH₂; N-(C₁-C₄)alkylamino (preferably N-methylamino or N-ethylamino); N,N-di(C₁-C₄-alkyl)amino (preferably dimethylamino, diethylamino or di-isopropylamino); optionally substituted aryl; optionally substituted heteroaryl.

The term "alkoxy", as used herein, refers to a straight or branched chain C₁₋₅ alkyl group, as previously defined, attached to the parent molecular moiety through an oxygen atom containing the specified number of carbon atoms. For example, C₁₋₄ alkoxy means a straight or branched alkoxy containing at least 1, and at most 4, carbon atoms. Examples of "alkoxy" as used herein include methoxy, ethoxy, propoxy, prop-2-oxy, butoxy, but-2-oxy, 2-methylprop-1-oxy and 2-methylprop-2-oxy.

The term "halogen" refers to a fluorine, chlorine, bromine or iodine atom.

As used herein, "carbocycle" or "carbocyclic ring" is intended to mean, unless otherwise specified, any stable 3, 4, 5, 6, 7, 8, 9, 10, 11, 12, 13 or 14-membered monocyclic, bicyclic or tricyclic ring, any of which may be saturated, unsaturated, or aromatic, recognizing that rings with certain numbers of members cannot be bicyclic or tricyclic, e.g. , a 3-membered ring can only be a monocyclic ring. Examples of such carbocycles include cyclopropyl, cyclobutyl, cyclobutenyl, cyclopentyl, cyclopentenyl, cyclohexyl, cycloheptenyl, cycloheptyl, cycloheptenyl, adamantyl, cyclooctyl, cyclooctenyl, cyclododecanyl, cyclooctadienyl, [3.3. 0] bicyclooctane, [4.3. 0] bicyclononane, [4.4. 0] bicyclodecane, [2.2. 2] bicyclooctane, phenyl, naphthyl, indanyl, adamantyl, and tetrahydronaphthyl. As shown above, bridged rings (e.g., adamantane, bicyclo[2.2.1]heptane) and fused rings (e. g., tetrahydronaphthalene, naphthalene, indane, anthracene, fluorene) are also included in the definition of carbocycle. A bridged ring occurs when one or more carbon atoms link two non-adjacent carbon atoms. Preferred bridges are one or two carbon atoms. It is noted that a bridge always converts a monocyclic ring into a tricyclic ring. When a ring is bridged, the substituents recited for the ring may also be present on the bridge.

The term "substituted carbocycle", as used herein, refers to an carbocycle group, as previously defined, substituted by independent replacement of one, two or three of the hydrogen atoms thereon with substituents including halogen; OH; CN; C₁₋₄alkyl unsubstituted (preferably methyl or ethyl) or substituted with 1 to 3 halogen (preferably trifluoromethyl) or CN group; O(C₁₋₄alkyl) optionally substituted with 1 to 3 halogen; S(C₁-C₄-alkyl) (preferably thiomethyl); O(C₃₋₆ cycloalkyl); O(C₁₋₄alkylaryl); C(O)C₁₋₄alkyl; C(O)OC₁₋₄alkylaryl; C₁₋₄ alkyloxycarbonyl; optionally substituted aryl; optionally substituted heteroaryl; C(O)C₁₋₄alkylaryl; NO₂; diazoaryl; sulfo- 5 or 6 membered carbocyclic or heterocyclic ring (preferably p-sulfopiperazyl); C₁₋₄alkyl-C(O)-O-C₁₋₄alkyl and C₁₋₄alkylO-C(O)-NR³.

The term "aryl", as used herein, refers to a mono-, bicyclic or tricyclic carbocyclic ring system having one, two or three aromatic rings including phenyl, naphthyl, tetrahydronaphthyl, indanyl, idenyl and anthracenyl.

The term "alkylaryl," as used herein, refers to a C₁₋₄ alkyl substituted with an aryl ring. Examples include benzyl and phenethyl.

As used herein, the term "heterocycle" means, unless otherwise stated, a stable 3, 4, 5, 6, 7, 8, 9, 10, 11, 12, 13, or 14-membered monocyclic, bicyclic or tricyclic ring (recognizing that rings with certain numbers of members cannot be bicyclic or tricyclic, e.g., a 3-membered ring can only be a monocyclic ring), any of which is saturated, unsaturated, or aromatic, and consists of carbon atoms and one or more ring heteroatoms, e.g., 1 or 1-2 or 1-3 heteroatoms, independently selected from the group consisting of nitrogen, oxygen, and sulfur, and including any bicyclic or tricyclic group in which any of the above-defined heterocyclic rings is fused to a second ring (e. g., a benzene ring). When a nitrogen atom is included in the ring it is either N or NH, depending on whether or not it is attached to a double bond in the ring (i.e., a hydrogen is present if needed to maintain the tri-valency of the nitrogen atom). The nitrogen atom may be substituted or unsubstituted (i. e., N or NR³ wherein R³ is H or C₁₋₄ alkyl as defined above). The heterocyclic ring may be attached to its pendant group at any heteroatom or carbon atom that results in a stable structure. The heterocyclic rings described herein may be substituted on carbon or on a nitrogen atom if the resulting compound is stable. A nitrogen in the heterocycle may optionally be quatemized. Bridged rings are also included in the definition of heterocycle. A bridged ring occurs when one or more atoms (i. e. , C, O, N, or S) link two non-adjacent carbon or nitrogen atoms. Preferred bridges include one carbon atom, two carbon atoms, one nitrogen atom, two nitrogen atoms, and a carbon-nitrogen group (e.g. quinuclidinyl). It is noted that a bridge always converts a monocyclic ring into a tricyclic ring. When a ring is bridged, the substituents recited for the ring may also be present on the bridge. Fused rings are also included (e.g. quinolinyl, iso quinolinyl, phenothiazinyl, acridinyl or phenoxazinyl).

The term "substituted heterocycle", as used herein, refers to an heterocycle group, as previously defined, substituted by independent replacement of one, two or three of the hydrogen atoms thereon with substituents including halogen; CN; C₁₋₄alkyl unsubstituted (preferably methyl or ethyl) or substituted with I to 3 halogen (preferably trifluoromethyl); O(C₁₋₄alkyl) optionally substituted with I to 3 halogen; S(C₁-C₄-alkyl) (preferably thiomethyl); O(C₃-₆ cycloalkyl); O(C₁₋₄alkylaryl); C₁₋₄alkylcyano; C(O)C₁₋₄alkyl; C₁₋₄ alkyloxycarbonyl; optionally substituted aryl; optionally substituted heteroaryl; C(O)C₁₋₄alkylaryl; C(O)OC₁₋₄alkylaryl; NO₂; diazoaryl; 5 or 6 membered carbocyclic or heterocyclic ring; sulfo- 5 or 6 membered carbocyclic or heterocyclic ring (preferably p-sulfopiperazyl); C₁₋₄alkyl-C(O)-O-C₁₋₄alkyl and C₁₋₄alkylO-C(O)-NR³.

As used herein, the term "aromatic heterocycle" or "heteroaryl" is intended to mean a stable 5,6, 7, 8, 9,10, 11, 12, 13 or 14-membered monocyclic or bicyclic aromatic ring (recognizing that rings with certain numbers of members cannot be a bicyclic aromatic, e. g., a 5-membered ring can only be a monocyclic aromatic ring), which consists of carbon atoms and one or more heteroatoms, e. g., 1 or 1-2 or 1-3 heteroatoms, independently selected from the group consisting of nitrogen, oxygen, and sulfur. In the case of bicyclic heterocyclic aromatic rings, only one of the two rings needs to be aromatic (e. g., 2,3-dihydroindole), though both may be (e.g., quinoline). The second ring can also be fused or bridged as defined above for heterocycles. The nitrogen atom may be substituted or unsubstituted (i. e., N or NR³ wherein R³ is H or C₁₋₄ alkyl as defined above).

Examples of heterocycles include acridinyl, benzimidazolyl, benzofuranyl, 2,3-dihydrobenzofuranyl, benzothiofuranyl, benzothiophenyl, benzoxazolyl, benzo[1,3]dioxolyl, benzo[1,3]dioxanyl benzoxazolinyl, benzthiazolyl, benztriazolyl, benzisoxazolyl, benzisothiazolyl, benzo[1,2,5]thiadiazolyl, benzimidazolinyl, 3,4-dihydro-2H-benzo[b][1,4]dioxepinyl, 4,5,6,7-tetrahydro-benzo[b]thiophenyl, carbazolyl, 4aH-carbazolyl, cinnolinyl, decahydroquinolinyl, dihydrofuro[2,3-b] tetrahydrofuran, furanyl, furazanyl, imidazolidinyl, imidazolinyl, imidazolyl, 1H-indazolyl, indolenyl, indolinyl, indolyl, 3H-indolyl, isatinoyl, isobenzofuranyl, isoindazolyl, isoindolinyl, isoindolyl, isoquinolinyl, isothiazolyl, isoxazolyl, methylenedioxyphenyl, morpholinyl, naphthyridinyl, octahydroisoquinolinyl, oxadiazolyl, 1,2,3-oxadiazolyl, 1,2,4-oxadiazolyl, 1,2,5-oxadiazolyl, 1,3,4-oxadiazolyl, oxazolidinyl, oxazolyl, oxindolyl, pyrimidinyl, phenanthridinyl, phenanthrolinyl, phenazinyl, phenothiazinyl, phenoxathinyl, phenoxazinyl, phthalazinyl, piperazinyl, piperidinyl, piperidonyl, 4-piperidonyl, piperonyl, pteridinyl, purinyl, pyranyl, pyrazinyl, pyrazolidinyl, pyrazolinyl, pyrazolyl, pyridazinyl, pyridooxazole, pyridoimidazole, pyridothiazole, pyridinyl, pyridyl, pyrimidinyl, pyrrolidinyl, pyrrolinyl, 2H-pyrrolyl, pyrrolyl, quinazolinyl, quinolinyl, 4H-quinolizinyl, quinoxalinyl, quinuclidinyl, tetrahydrofuranyl, tetrahydroisoquinolinyl, tetrahydroquinolinyl, tetrazolyl,6H-1,2,5-thiadiazinyl, 1,2,3-thiadiazolyl, 1,2,4-thiadiazolyl, 1,2,5-thiadiazolyl, 1,3,4-thiadiazolyl, thianthrenyl, thiazolyl, thienyl, thienothiazolyl, thienooxazolyl, thienoimidazolyl, thiophenyl, triazinyl, 1,2,3-triazolyl, 1,2,4-triazolyl, 1,2,5-triazolyl, 1,3,4-triazolyl, and xanthenyl.

The term "alkoxycarbonyl" represents an ester group, i.e., an alkoxy group, attached to the parent molecular moiety through a carbonyl group such as methoxycarbonyl and ethoxycarbonyl.

The term "lower alcohol", as used herein, refers to a C₁₋₄alcohol, such as for example, methanol, ethanol, propanol, isopropanol, butanol and t-butanol.

"Treating" or "treatment" of malaria includes
i. preventing or delaying the appearance of clinical symptoms of malaria developing in a mammal that has been in contact with the parasite.
ii. inhibiting the malaria, i.e., arresting reducing or delaying the development of malaria or a relapse thereof or at least one clinical or subclinical symptom thereof, or
iii. relieving or attenuating one or more of the clinical or subclinical symptoms of malaria.

The benefit to a subject to be treated is either statistically significant or at least perceptible to the patient or to the physician.

"Prophylactic treatment" of malaria includes treating subjects who are at risk of developing malaria. This includes the treatment of subjects who have been exposed to malaria-bearing mosquitoes, the treatment of subjects who intend to travels to a country where malaria is endemic and the treatment of subjects who otherwise risk exposure to malaria-bearing mosquitoes.

"Maintenance therapy" is therapy during a phase of malaria following the acute phase, where the parasite achievement of remission (total or partial) of one or more symptoms of the disease until the next flare-up of the disease. The *Plasmodium vivax* and *P. ovale* parasites have dormant liver stages that can remain silent for years. Maintenance therapy for these strains is particularly important. The hallmarks of the acute phase include symptoms like chills, fever,

"Subject" refers to an animal, which is preferably a mammal and more preferably a human or a domestic animal or an animal serving as a model for a disease (*e.g.*, mouse, monkey, etc.). Most preferably, the subject is a human. As used herein, the term patient is used synonymously with subject.

A "therapeutically effective amount" means the amount of a compound that, when administered to a mammal for treating a state, disorder or condition, is sufficient to effect such treatment. The "therapeutically effective amount" will vary depending on the compound, the disease and its severity and the age, weight, physical condition and responsiveness of the mammal to be treated.

### Pharmaceutical Compositions

While it is possible that, for use in the methods of the invention, a compound of formula I may be administered as the bulk substance, it is preferable to present the active ingredient in a pharmaceutical formulation, *e.g.,* wherein the agent is in admixture with a pharmaceutically acceptable carrier selected with regard to the intended route of administration and standard pharmaceutical practice.

The term "carrier" refers to a diluent, excipient, and/or vehicle with which an active compound is administered. The pharmaceutical compositions of the invention may contain combinations of more than one carrier. Such pharmaceutical carriers can be sterile liquids, such as water, saline solutions, aqueous dextrose solutions, aqueous glycerol solutions, and oils, including those of petroleum, animal, vegetable or synthetic origin, such as peanut oil, soybean oil, mineral oil and sesame oil. Water or aqueous solution saline solutions and aqueous dextrose and glycerol solutions are preferably employed as carriers, particularly for injectable solutions. Suitable pharmaceutical carriers are described in "Remington's Pharmaceutical Sciences" by E.W. Martin, 18th Edition. The choice of pharmaceutical carrier can be selected with regard to the intended route of administration and standard pharmaceutical practice. The pharmaceutical compositions may comprise as, in addition to, the carrier any suitable binder(s), lubricant(s), suspending agent(s), coating agent(s), and/or solubilizing agent(s).

A "pharmaceutically acceptable excipient" means an excipient that is useful in preparing a pharmaceutical composition that is generally safe, non-toxic and neither biologically nor otherwise undesirable, and includes an excipient that is acceptable for veterinary use as well as human pharmaceutical use. A "pharmaceutically acceptable excipient" as used in the present application includes both one and more than one such excipient.

It will be appreciated that pharmaceutical compositions for use in accordance with the present invention may be in the form of oral, parenteral, transdermal, inhalation, sublingual, topical, implant, nasal, or enterally administered (or other mucosally administered) suspensions, capsules or tablets, which may be formulated in conventional manner using one or more pharmaceutically acceptable carriers or excipients.

There may be different composition/formulation requirements depending on the different delivery systems. It is to be understood that not all of the compounds need to be administered by the same route. Likewise, if the composition comprises more than one active component, then those components may be administered by the same or different routes. By way of example, the pharmaceutical composition of the present invention may be formulated to be delivered using a mini-pump or by a mucosal route, for example, as a nasal spray or aerosol for inhalation or ingestible solution, or parenterally in which the composition is formulated by an injectable form, for delivery, by, for example, an intravenous, intramuscular or subcutaneous route. Alternatively, the formulation may be designed to be delivered by multiple routes.

The present invention further relates to pharmaceutical formulations containing a therapeutically effective quantity of a compound of formula I or one of its salts mixed with a pharmaceutically acceptable vehicle. The pharmaceutical formulations of the present invention can be liquids that are suitable for oral, mucosal and/or parenteral administration, for example, drops, syrups, solutions, injectable solutions that are ready for use or are prepared by the dilution of a freeze-dried product but are preferably solid or semisolid as tablets, capsules, granules, powders, pellets, pessaries, suppositories, creams, salves, gels, ointments; or solutions, suspensions, emulsions, or other forms suitable for administration by the transdermal route or by inhalation.

The compounds of the invention can be administered for immediate-, delayed-, modified-, sustained-, pulsed-or controlled-release applications.

The most preferred oral compositions are slow, delayed or positioned release (e.g., enteric especially colonic release) tablets or capsules. This release profile can be achieved by use of a coating resistant to conditions within the stomach but releasing the contents in the colon or other portion of the GI tract wherein a lesion or inflammation site has been identified. Or a delayed release can be achieved by a coating that is simply slow to disintegrate. Or the two (delayed and positioned release) profiles can be combined in a single formulation by choice of one or more appropriate coatings and other excipients. Such formulations constitute a further feature of the present invention.

Suitable compositions for delayed or positioned release and/or enteric coated oral formulations include tablet formulations film coated with materials that are water resistant, pH sensitive, digested or emulsified by intestinal juices or sloughed off at a slow but regular rate when moistened. Suitable coating materials include hydroxypropyl methylcellulose, ethyl cellulose, cellulose acetate phthalate, polyvinyl acetate phthalate, hydroxypropyl methylcellulose phthalate, polymers of metacrylic acid and its esters, and combinations thereof. Plasticizers such as polyethylene glycol, dibutylphthalate, triacetin and castor oil may be used. A pigment may also be used to color the film. Suppositories are be prepared by using carriers like cocoa butter, suppository bases such as Suppocire C, and Suppocire NA50 (supplied by Gattefossé Deutschland GmbH, D-Weil am Rhein, Germany) and other Suppocire type excipients obtained by interesterification of hydrogenated palm oil and palm kernel oil (C8-C18 triglycerides), esterification of glycerol and specific fatty acids, or polyglycosylated glycerides, and whitepsol (hydrogenated plant oils derivatives with additives). Enemas are formulated by using the appropriate active compound according to the present invention and solvents or excipients for suspensions. Suspensions are produced by using micronized compounds, and appropriate vehicle containing suspension stabilizing agents, thickeners and emulsifiers like carboxymethylcellulose and salts thereof, polyacrylic acid and salts thereof, carboxyvinyl polymers and salts thereof, alginic acid and salts thereof, propylene glycol alginate, chitosan, hydroxypropylcellulose, hydroxypropyl methylcellulose, hydroxyethylcellulose, ethylcellulose, methylcellulose, polyvinyl alcohol, polyvinyl pyrrolidone, N-vinylacetamide polymer, polyvinyl methacrylate, polyethylene glycol, pluronic, gelatin, methyl vinyl ether-maleic anhydride copolymer, soluble starch, pullulan and a copolymer of methyl acrylate and 2-ethylhexyl acrylate lecithin, lecithin derivatives, propylene glycol fatty acid esters, glycerin fatty acid esters, sorbitan fatty acid esters, polyoxyethylene sorbitan fatty acid esters, polyethylene glycol fatty acid esters, polyoxyethylene hydrated caster oil, polyoxyethylene alkyl ethers, and pluronic and appropriate buffer system in pH range of 6.5 to 8. The use of preservatives, masking agents is suitable. The average diameter of micronized particles can be between 1 and 20 micrometers, or can be less than 1 micrometer. Compounds can also be incorporated in the formulation by using their water-soluble salt forms.

Alternatively, materials may be incorporated into the matrix of the tablet e.g. hydroxypropyl methylcellulose, ethyl cellulose or polymers of acrylic and metacrylic acid esters. These latter materials may also be applied to tablets by compression coating.

Pharmaceutical compositions can be prepared by mixing a therapeutically effective amount of the active substance with a pharmaceutically acceptable carrier that can have different forms, depending on the way of administration. Pharmaceutical compositions can be prepared by using conventional pharmaceutical excipients and methods of preparation. The forms for oral administration can be capsules, powders or tablets where usual solid vehicles including lactose, starch, glucose, methylcellulose, magnesium stearate, di-calcium phosphate, mannitol may be added, as well as usual liquid oral excipients including ethanol, glycerol, and water. All excipients may be mixed with disintegrating agents, solvents, granulating agents, moisturizers and binders. When a solid carrier is used for preparation of oral compositions (e.g., starch, sugar, kaolin, binders disintegrating agents) preparation can be in the form of powder, capsules containing granules or coated particles, tablets, hard gelatin capsules, or granules, and the amount of the solid carrier can vary (between 1 mg to 1 g). Tablets and capsules are the preferred oral composition forms.

Pharmaceutical compositions containing compounds of the present invention may be in any form suitable for the intended method of administration, including, for example, a solution, a suspension, or an emulsion. Liquid carriers are typically used in preparing solutions, suspensions, and emulsions. Liquid carriers contemplated for use in the practice of the present invention include, for example, water, saline, pharmaceutically acceptable organic solvent(s), pharmaceutically acceptable oils or fats, as well as mixtures of two or more thereof. The liquid carrier may contain other suitable pharmaceutically acceptable additives such as solubilizers, emulsifiers, nutrients, buffers, preservatives, suspending agents, thickening agents, viscosity regulators and stabilizers. Suitable organic solvents include, for example, monohydric alcohols, such as ethanol, and polyhydric alcohols, such as glycols. Suitable oils include, for example, soybean oil, coconut oil, olive oil, safflower oil and cottonseed oil. For parenteral administration, the carrier can also be an oily ester such as ethyl oleate and isopropyl myristate. Compositions of the present invention may also be in the form of microparticles, microcapsules and liposomal encapsulates as well as combinations of any two or more thereof.

Examples of pharmaceutically acceptable disintegrants for oral compositions useful in the present invention include starch, pre-gelatinized starch, sodium starch glycolate, sodium carboxymethylcellulose, croscarmellose sodium, microcrystalline cellulose, alginates, resins, surfactants, effervescent compositions, aqueous aluminum silicates and crosslinked polyvinylpyrrolidone.

Examples of pharmaceutically acceptable binders for oral compositions useful herein include acacia; cellulose derivatives, such as methylcellulose, carboxymethylcellulose, hydroxypropylmethylcellulose, hydroxypropylcellulose or hydroxyethylcellulose; gelatin, glucose, dextrose, xylitol, polymethacrylates, polyvinylpyrrolidone, sorbitol, starch, pre-gelatinized starch, tragacanth, xanthane resin, alginates, magnesium-aluminum silicate, polyethylene glycol or bentonite.

Examples of pharmaceutically acceptable fillers for oral compositions include lactose, anhydrolactose, lactose monohydrate, sucrose, dextrose, mannitol, sorbitol, starch, cellulose (particularly microcrystalline cellulose), dihydro- or anhydro-calcium phosphate, calcium carbonate and calcium sulfate.

Examples of pharmaceutically acceptable lubricants useful in the compositions of the invention include magnesium stearate, talc, polyethylene glycol, polymers of ethylene oxide, sodium lauryl sulfate, magnesium lauryl sulfate, sodium oleate, sodium stearyl fumarate, and colloidal silicon dioxide.

Examples of suitable pharmaceutically acceptable odorants for the oral compositions include synthetic aromas and natural aromatic oils such as extracts of oils, flowers, fruits (*e.g.*, banana, apple, sour cherry, peach) and combinations thereof, and similar aromas. Their use depends on many factors, the most important being the organoleptic acceptability for the population that will be taking the pharmaceutical compositions.

Examples of suitable pharmaceutically acceptable dyes for the oral compositions include synthetic and natural dyes such as titanium dioxide, beta-carotene and extracts of grapefruit peel.

Suitable examples of pharmaceutically acceptable sweeteners for the oral compositions include aspartame, saccharin, saccharin sodium, sodium cyclamate, xylitol, mannitol, sorbitol, lactose and sucrose.

Suitable examples of pharmaceutically acceptable buffers include citric acid, sodium citrate, sodium bicarbonate, dibasic sodium phosphate, magnesium oxide, calcium carbonate and magnesium hydroxide.

Suitable examples of pharmaceutically acceptable surfactants include sodium lauryl sulfate and polysorbates.

Suitable examples of pharmaceutically acceptable preservatives include various antibacterial and antifungal agents such as solvents, for example ethanol, propylene glycol, benzyl alcohol, chlorobutanol, quaternary ammonium salts, and parabens (such as methyl paraben, ethyl paraben, propyl paraben, etc.).

Suitable examples of pharmaceutically acceptable stabilizers and antioxidants include ethylenediaminetetriacetic acid (EDTA), thiourea, tocopherol and butyl hydroxyanisole.

The compounds of the invention may also, for example, be formulated as suppositories *e*.*g*., containing conventional suppository bases for use in human or veterinary medicine or as pessaries *e.g.,* containing conventional pessary bases.

The compounds according to the invention may be formulated for topical administration, for use in human and veterinary medicine, in the form of ointments, creams, gels, hydrogels, lotions, solutions, shampoos, powders (including spray or dusting powders), pessaries, tampons, sprays, dips, aerosols, drops (*e*.*g*., eye ear or nose drops) or pour-ons.

For application topically to the skin, the agent of the present invention can be formulated as a suitable ointment containing the active compound suspended or dissolved in, for example, a mixture with one or more of the following: mineral oil, liquid petrolatum, white petrolatum, propylene glycol, polyoxyethylene polyoxypropylene compound, emulsifying wax, sorbitan monostearate, a polyethylene glycol, liquid paraffin, polysorbate 60, cetyl esters wax, cetearyl alcohol, 2-octyldodecanol, benzyl alcohol, and water. Such compositions may also contain other pharmaceutically acceptable excipients, such as polymers, oils, liquid carriers, surfactants, buffers, preservatives, stabilizers, antioxidants, moisturizers, emollients, colorants, and odorants.

Examples of pharmaceutically acceptable polymers suitable for such topical compositions include acrylic polymers; cellulose derivatives, such as carboxymethylcellulose sodium, methylcellulose or hydroxypropylcellulose; natural polymers, such as alginates, tragacanth, pectin, xanthan and cytosan.

As indicated, the compound of the present invention can be administered intranasally or by inhalation and is conveniently delivered in the form of a dry powder inhaler or an aerosol spray presentation from a pressurized container, pump, spray or nebulizer with the use of a suitable propellant, *e.g.,* dichlorodifluoromethane, trichlorofluoromethane, dichlorotetrafluoroethane, a hydrofluoroalkane such as 1,1,1,2-tetrafluoroethane (HFA 134AT"") or 1,1,1,2,3,3,3-heptafluoropropane (HFA 227EA), carbon dioxide or other suitable gas. In the case of a pressurized aerosol, the dosage unit may be determined by providing a valve to deliver a metered amount. The pressurized container, pump, spray or nebulizer may contain a solution or suspension of the active compound, *e.g.,* using a mixture of ethanol and the propellant as the solvent, which may additionally contain a lubricant, *e.g.,* sorbitan trioleate.

Capsules and cartridges (made, for example, from gelatin) for use in an inhaler or insufflator may be formulated to contain a powder mix of the compound and a suitable powder base such as lactose or starch.

For topical administration by inhalation the compounds according to the invention may be delivered for use in human or veterinary medicine via a nebulizer.

The pharmaceutical compositions of the invention may contain from 0.01 to 99% weight per volume of the active material. For topical administration, for example, the composition will generally contain from 0.01-10%, more preferably 0.01-1% of the active material.

A therapeutically effective amount of the compound of the present invention can be determined by methods known in the art. The therapeutically effective quantities will depend on the age and on the general physiological condition of the patient, the route of administration and the pharmaceutical formulation used. It will also be determine by the strain of malaria parasite that has infected the subject. The therapeutic doses will generally be between about 10 and 2000 mg/day and preferably between about 30 and 1500 mg/day. Other ranges may be used, including, for example, 50-500 mg/day, 50-300 mg/day, 100-200 mg/day. The amount of the compound required for prophylactic treatment, referred to as a prophylactically-effective dosage, is generally the same as described for therapeutic treatment.

Administration may be once a day, twice a day, or more often, and may be decreased during a maintenance phase of the disease or disorder, e.g. once every second or third day instead of every day or twice a day. The dose and the administration frequency will depend on the clinical signs, which confirm maintenance of the remission phase, with the reduction or absence of at least one or more preferably more than one clinical signs of the acute phase known to the person skilled in the art.

### Method of preparation:

Compounds of Formula **(I)** and pharmaceutically acceptable salts, solvates and esters thereof may be prepared by the general methods outlined hereinafter, said methods constituting a further aspect of the invention. In the following description, the groups X, R, R¹, R², R³ and n have the meaning defined for the compounds of Formula (I) unless otherwise stated.

It will be appreciated by those skilled in the art that it may be desirable to use protected derivatives of intermediates used in the preparation of the compounds of Formula **(I)**. Protection and deprotection of functional groups may be performed by methods known in the art. Hydroxyl or amino groups may be protected with any hydroxyl or amino protecting group (for example, as described in Green and Wuts. Protective Groups in Organic Synthesis. John Wiley and Sons, New York, 1999). The protecting groups may be removed by conventional techniques. For example, acyl groups (such as alkanoyl, alkoxycarbonyl and aryloyl groups) may be removed by solvolysis (*e.g.*, by hydrolysis under acidic or basic conditions). Arylmethoxycarbonyl groups (*e.g.*, benzyloxycarbonyl) may be cleaved by hydrogenolysis in the presence of a catalyst such as palladium-on-carbon.

The synthesis of the target compound is completed by removing any protecting groups, which are present in the penultimate intermediate using standard techniques, which are well-known to those skilled in the art. The final product is then purified, as necessary, using standard techniques such as silica gel chromatography, HPLC on silica gel or by recrystallization.
The compounds of Formula **(I)** as described herein and pharmaceutically acceptable salts, solvates and esters thereof can be prepared by reacting the compound of Formula **(III)** 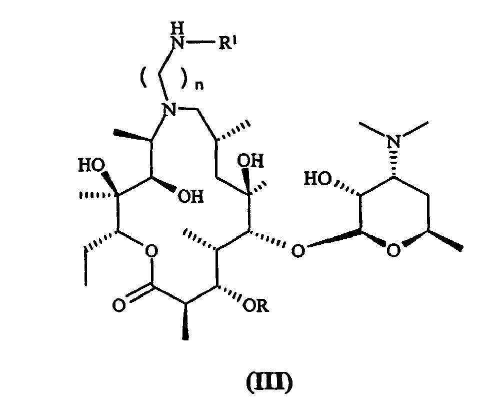 with isocyanates or thioisocyanates of general Formula **(IV)**,

R²-N=C=X **(IV)**

in toluene, xylene, dichloromethane or some other aprotic solvent, at a temperature from 0° to 110°C.
The compound of Formula **(III)**, wherein R¹ represents β-cyanoethyl group as described herein can be prepared by reacting the compound of Formula **(V)** with acrylonitrile in an inert solvent, preferably lower alcohol (such as ethanol or methanol). 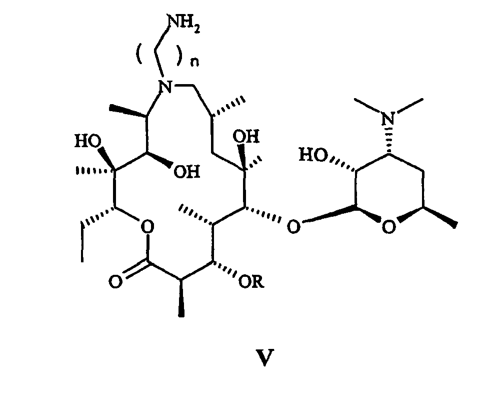 The compound of Formula **(III)**, wherein R¹ represents β-(C₁₋₄alkoxycarbonyl)ethyl group as described herein can be prepared by reacting the compound of Formula **(V)** with C₁₋₄alkylacrylate in an inert solvent, preferably lower alcohol (such as methanol).
The compound of Formula **(III)**, wherein R¹ represents β-amidoethyl group as described herein can be prepared by reacting the compound of Formula **(III)**, wherein R¹ represents β-(C₁₋₄akoxycarbonyl)ethyl group with ammonia in an inert solvent, preferably lower alcohol (such as ethanol or methanol).
Yet another means by which compound of Formula **(I)**, wherein R¹ represents β-amidoethyl group and R represents hydrogen as described herein, can be prepared is by mild acid hydrolysis of a compound of Formula **(I)**, wherein R¹ represents β-(C₁₋₄alkoxycarbonyl)ethyl group and R represents cladinosyl group of Formula **(II)**, in diluted hydrochloric acid.

Pharmaceutically acceptable acid addition salts, which also represent an object of present invention, were obtained by reaction compound of Formula **(I)** with an at least equimolar amount of the corresponding inorganic or organic acid such as hydrochloric acid, hydroiodic acid, sulfuric acid, phosphoric acid, acetic acid, trifluoroacetic acid, propionic acid, benzoic acid, benzenesulfonic acid, methane sulfonic acid, laurylsulfonic acid, stearic acid, palmitic acid, succinic acid, ethylsuccinic acid, lactobionic acid, oxalic acid, salicylic acid and similar acid, in a solvent inert to the reaction. Addition salts are isolated by evaporating the solvent or, alternatively, by filtration after a spontaneous precipitation or a precipitation by the addition of a non-polar cosolvent.

Compounds of the Formula **(I)** and pharmaceutically acceptable addition salts with inorganic or organic acids thereof possess an antimalarial activity *in vitro.*

### Biological Assays

The therapeutic effect of compounds of the present invention was determined in experiments provided in the examples.

### In vitro Screening Protocols

The *in vitro* screens for intrinsic antimalarial activity were based on modifications of the procedures described by Desjardins RE, Canfield CJ, Haynes JD, Chulay JD. (Quantitative assessment of antimalarial activity in vitro by a semiautomated microdilution technique. Antimicrob Agents Chemother. 1979 Dec;16(6):710-8.), Chulay JD, Haynes JD, Diggs CL. (Plasmodium falciparum: Assessment of in vitro growth by [3H]hypoxanthine incorporation. Exp. Parasitol. 1983 55:138-146.), and Milhous WK, Weatherly NF, Bowdre JH, Desjardins RE. (In vitro activities of and mechanisms of resistance to antifol antimalarial drugs. Antimicrob Agents Chemother. 1985 Apr;27(4):525-30.). The system was limited to the assessment of the intrinsic activity against the erythrocytic asexual life cycle (blood schizontocides). Two *Plasmodium falciparum* clones from CDC/Indochina III (W-2) and CDC/Sierra Leone I (D-6) (Oduola AM, Weatherly NF, Bowdre JH, Desjardins RE. Plasmodium falciparum: cloning by single-erythrocyte micromanipulation and heterogeneity in vitro. Exp Parasitol. 1988 66(1):86-95.) were used for all assays. TM91C235, a multiple drug resistant isolate from Thailand, was used for the prescreening assay. W-2 is resistant to chloroquine, quinine, and pyrimethamine and susceptible to mefloquine. D-6 tends to be more resistant to mefloquine and susceptible to chloroquine, quinine, and pyrimethamine.

All parasites were maintained in continuous long term cultures in RPMI-1640 medium supplemented with 6% washed human A positive (A+)(erythrocytes, 25 mM Hepes, 32 nM NaHCO₃, and 10% heat inactivated A+ human plasma or ALBUMAX® (lipid-rich bovine serum albumin; Invitrogen, Carlsbad, CA). All cultures and assays were conducted at 37°C under an atmosphere of 5% CO₂ and 5% O₂, with a balance of N₂.

### PreScreening Assay

The prescreening assay uses TM91C235 diluted at a 0.4% parasitemia in a 1% hematocrit in folic acid free and p-aminobenzoic acid free media RPMI-1640 and ALBUMAX®. One mg of the compound is typically dissolved in 100 µl of dimethyl sulfoxide (DMSO). The compound is further diluted in culture medium (FF) with ALBUMAX® for the first initial starting concentration. The rest of the stock drug solution was kept at -70°C. The isolate was preexposed, in duplicate, at three concentrations (25,000 ng/ml, 2,500 ng/ml, and 25 ng/ml) of the test compound for 48 hr in a 96-well microtiter plate (MTP) using the BIOMEK® 2000 automated laboratory workstation. Each MTP contains chloroquine as control to assess the relative activity of the compound and to monitor the response ofTM91C235.

After the preincubation, [³H]-hypoxanthine was added to each well of the MTP. (The assay relies on the incorporation of radiolabeled hypoxanthine by the parasites, which indicates reproduction, and inhibition of isotope incorporation was attributed to activity of known or candidate antimalarial drugs). After 72 hr of total incubation time, the MTP were frozen to lyse the erythrocytes and parasites. The parasite DNA was recovered by harvesting the lysate onto glass-fiber filter plates using a Packard FilterMate™ Cell Harvester. The radioactivity was counted on a Packard TopCount™ microplate scintillation counter. The results were recorded as counts per minutes (CPM) per well at each drug concentration divided by the arithmetic mean of the CPM from the three untreated infection parasite control wells.

### Serial Dilution Assay

If a compound did not affect parasite growth at 25,000 ng/ml, it was classified as inactive. If a compound suppressed greater than two standard deviations from the arithmetic mean of the untreated infection controls at 25,000 ng/ml, but less than 50% at 2,500 ng/ml, the compound was designated as partially active. However, if a compound suppressed greater than 50% of the incorporation of [³H]- hypoxanthine relative to untreated infection control parasites at 2,500 ng/ml, the compound was classified as fully active and was further evaluated by two-fold serial dilutions to determine the IC₅₀ value (50% inhibitory concentration).

The serial dilution assay was conducted using the same assay conditions and stock solution of the compound used for the preliminary screen, described above. Both the D-6 and the W-2 clones were used. The compounds were diluted two-fold over 11 different concentration ranges with a starting concentration that was based on the preliminary screen. For each drug, the concentration response profile was determined and 50% inhibitory concentrations (IC₅₀) were determined by using a non-linear, logistic dose response analysis program. If the results from this assay did not agree with the concentration ranges of the preliminary screen, the assay was repeated. For each assay, the IC₅₀ for each clone was determined against the known antimalarials chloroquine and mefloquine. These control values established the compound's relative parasite susceptibility profile compared to known antimalarials.

IC₅₀s can be similarly determined for drug-resistant isolates/clones from a wide variety of geographic locations by using different isolates/clones in the assays described herein. The assays described above can be repeated using both samples according to Formula I and isolates/clones from different malaria strains to determine the antilmalarial activity of the compounds. For Example, the above assays can be used to determine the IC₅₀ values for malarial strains TM91C235 (reported to be resistant to mefloquine, chloroquine, and quinine), D6 (reported to be resistant to mefloquine), and W2 (reported to be resistant to chloroquine).

### Presumptive Causal Prophylactic Test

The presumptive causal prophylactic test determines if test compounds have activity against either the sporozoite or exoerythrocytic (EE) stages of *Plasmodium yoelii* in mice. If all of the sporozoites or EE stages are killed, then blood stream parasites will not appear. If some numbers of these asexual tissue stages are killed then there will be a reduction in parasitemia. The mice eventually self cure and most of the mice survive. The compounds will be listed as either active or inactive based on observed parasitemias.

The presumptive causal prophylactic test may yield false positive results because of the relatively short preerythrocytic stage of the parasite (two days) and the unknown biological half-life of the test compound. It is, therefore, considered to be a test for presumptive activity and a positive result must be confirmed in another system such as *Plasmodium cynomolgi* in rhesus monkeys.

The presumptive causal prophylactic test is performed as follows: Four to five week old male CD-1 mice weighing 16-17 g, purchased from Charles River, are placed 5 per cage and allowed to acclimate for 4-7 days before being treated and infected. The animals are maintained at 75°F with a 12 hr light and 12 hr darkness cycle. The mice are fed a standard Ralston Purina™ mouse chow and given water adlibidum. The cages, corncob bedding, and water bottles are changed biweekly.

Each test compound is ground with a mortar & pestle. Compounds to be administered orally (PO) are suspended in 0.5% hydroxyethylcellulose-0.1% Tween S0. Those given subcutaneously (SC) are suspended in peanut oil. Each compound is prepared at 3 different dose levels.

*Plasmodium yoelii* 17XNL strain, is used to infect mice that will be used to infect the mosquitoes from which the sporozoites are isolated. An inoculum of 2.5 x 105 sporozoites per 0.1 mL are used to inoculate the test mice described above, 4 hours after administration of the test compound. The EE stage in the liver exists for only two days and the hypnozoite stage does not exist. Mice often self-cure from blood stage infections.

Infected, non-drug-treated controls are run with every experiment to validate the viability of the sporozoites. While these sporozoites usually produce patent infections, some mice may remain blood negative. Caution must be taken when judging a compound as prophylactic when the patency rate in the negative controls is less than 100%. The patency rate must be >80% to consider the test successful. Positive controls groups are included occasionally. Additional control mice are treated with Primaquine or Tafenoquine, which are prophylactically effective against the sporozoite and exoerythrocytic (EE) stages of *Plasmodium yoelii*.

### In Vivo Malaria Rhesus Presumptive Causal Prophylactic Test

Note: The Rhesus Causal Prophylactic Test, CP, was not implemented until 2001. Neither Sweeney (1991) nor Davidson et al. (1981) mention it.

The Rhesus Presumptive Causal Prophylactic Test is used to determine if test compounds have activity against either the sporozoite and/or exoerythrocytic (EE) stages of Plasmodium cynomolgi in Rhesus monkeys.

Briefly, healthy Indian Rhesus monkeys, *Macaca mulatta,* weighing 2-4 kg of either sex are used. Efforts are made to obtain an equal sex distribution and to keep animals in each test as uniform as possible. Prior to use, each animal undergoes a quarantine for at least five weeks during which time they are tuberculin-tested and treated with thiabendazole. Only malaria free monkeys are used. Usually, two monkeys are used for each dose.

Monkeys are often used in multiple experiments. If a monkey relapses, its infection is cleared with a radical treatment of primaquine and chloroquine before enrollment into a subsequent experiment.

Prior to administration compounds are dissolved in distilled water. If a compound is insoluble in distilled water it is solubilized in methylcellose, DMSO or HEC Tween. Drug concentrations are based on the body weight of each monkey which is determined the day before the first treatment.

Sporozoites of *Plasmodium cynomolgi bastianelli* isolated from laboratory infected mosquitoes are used for infection (see method below). Monkeys are infected with 0.5-1.5 X 106 sporozoites intravenously on day 0. Experimental monkeys are treated with compound on days -1, 0, and 1 relative to the day of infection. Negative control monkeys are given vehicle on the same days. To determine parasitemia, thin films are made from peripheral blood and stained with Giemsa. The number of infected RBCs per 500 RBCs is determined and converted to a percentage. If parasites are not found in 500 RBCs, then 1,000 RBCs are counted.

In non-treated monkeys, a rapidly rising parasitemia develops after a 7-9 day prepatent period. The test is considered valid when the controls develop parasitemia. All monkeys that become parasitemic are given a radical treatment with primaquine in combination with chloroquine. Blood smears to determine parasitemia are taken daily through day 20 post infection, and every 2-3 days thereafter.

The criteria for compound activity and toxicity are as follows. A compound is labeled as prophylactic if the monkeys show negative blood films for 30 days after splenectomy, or for100 days in intact monkeys. The lowest total dose, mg/kg body weight, resulting in negative blood films is reported as the MCD, Minimum Curative Dose. If the MCD is equal to the lowest total dose tested, then it is reported at '<=' the value of the lowest dose. If the highest dose tested is not prophylactic, then the MCD is reported as '>' the value of the highest total dose.

A compound is not considered prophylactic if parasitemia appears within 30 days post-infection in splenectomized monkeys, or within 100 days post infection in intact monkeys.

A compound is toxic if the investigator records a death, sign or symptom from compound toxicity rather than from malaria or an accident. The lowest total dose that causes toxicity is the MTD, Minimum Tolerated Dose. If the MTD is equal to the lowest total dose tested, then it is reported as '<=' the value of the lowest dose. If the highest dose tested is non-toxic, then the MTD is reported as '>' the value of the highest dose.

### Raising and Infecting the Mosquitoes

Cages of non-infected *Anopheles dirus* are kept in a room maintained at 80°F. They are allowed to feed on non-infected mice to obtain enough blood needed to produce eggs. Jars with wet cotton and moist paper towels are placed in the mosquito cages. Female mosquitoes lay their eggs on the moist paper towels. The eggs are collected and placed in enamel pans containing water. The eggs hatch and develop into larvae. The larvae are fed a liver powder suspension (2.5% liver powder in water). When the pupae have fully developed, they are placed in empty jars, which are then placed in empty mosquito cages. After the adult mosquitoes emerge from the pupal stage the jars are removed. The mosquito cages containing mosquitoes to be infected are transferred to a room maintained at 70°F. The female mosquitoes are allowed to feed on an anesthetized Rhesus monkey with circulating gametocytes of *P. cynomolgi*.

The mosquitoes are maintained in this cool room for 17 days when then are taken for sporozoite isolation. During the last 4 days a solution of PenStrep is fed to the mosquitoes to kill as many bacteria in their guts. These mosquitoes are as aspirated into a plastic baggie that is heat-sealed. This bag is placed on a freezing table to immobilize the mosquitoes. The bag is opened and the female mosquitoes are collected while the males are discarded.

The infected females are ground with a mortar and pestle in a 1:1 monkey serum-saline solution. Twenty more ml of saline is added to the mortar and the suspension is filtered to remove large pieces of mosquitoes. The sporozoites in the saline suspension are then counted and diluted to get an inoculum of 2.5 X 105 sporozoites per 0.1 ml. This is then inoculated intravenously into the test monkeys on day 0.

### In Vitro Inhibition of Liver-Stage Development Assay

The Inhibition of Liver-Stage Development Assay (ILSDA) is an *in vitro* model for evaluating the efficacy of drugs against the exoerythrocytic stages of *Plasmodium* sp. in the liver. We are using a modification of the method described by Sacci JB, 2002, Methods in Molecular Medicine, Vol 72, Malaria Methods and Protocols, p. 517-520. To enhance visualization of exoerythrocytic liver stage parasites, we use a clonal line of *P. berghei* (PbFluspo) that was stably transformed with green fluorescent protein (GFP), an autonomously fluorescent marker [Natarajan et al., Cellular Microbiology, 2001, 3(6): 371-379]. Sporozoites obtained from mosquitoes infected with the *P.berghei*-GFP were used to infect a human hepatocellular carcinoma cell line, HepG2, at a 1:1 ratio in 8-well LabTek chamber slides. After a three-hour incubation to allow for invasion, the HepG2 cells are washed to remove sporozoites that have not invaded. The cultures are then treated with test compounds at 3 doses (ten-fold serial dilutions) for 48 hrs, and liver stage parasites are counted by fluorescence microscopy. Percent parasite inhibition is determined as follows: (control GFP count - experimental GFP count/control GFP count) x 100. Primaquine, a known causal prophylactic drug, is run simultaneously as a positive control.

### Examples

The following abbreviations are used in the text: DCM for dichloromethane, DMSO for dimethyl sulfoxide, EtOAc for ethyl acetate, MeOH for methanol, EtOH for ethanol and THF for tetrahydrofuran.

The compounds and process of the present invention will be better understood in connection with the following examples. Various changes and modifications to the disclosed embodiments will be apparent to those skilled in the art and such changes and modifications including those relating to the chemical structures, substituents, derivatives formulations and/or methods of the invention may be made without departing from the scope of the appended claims.

### EXAMPLES

9-deoxo-9-dihydro-9a-aza-9a-homoerythromicin A may be prepared by procedure as described in J. Chem. Soc. Perkin Trans. I (1986) pages 1881-1890. 9a-(γ-aminopropyl)-9a-aza-9-deoxo-9-dihydro-9a-homoerythromycin A, 9a-(y-aminopropyl)-9a-aza-9-deoxo-9-dihydro-3-O-decladinosyl-9a-homoerythromycin A may be prepared by procedure as described in international patent application WO 02/055531 A1.

### INTERMEDIATES:

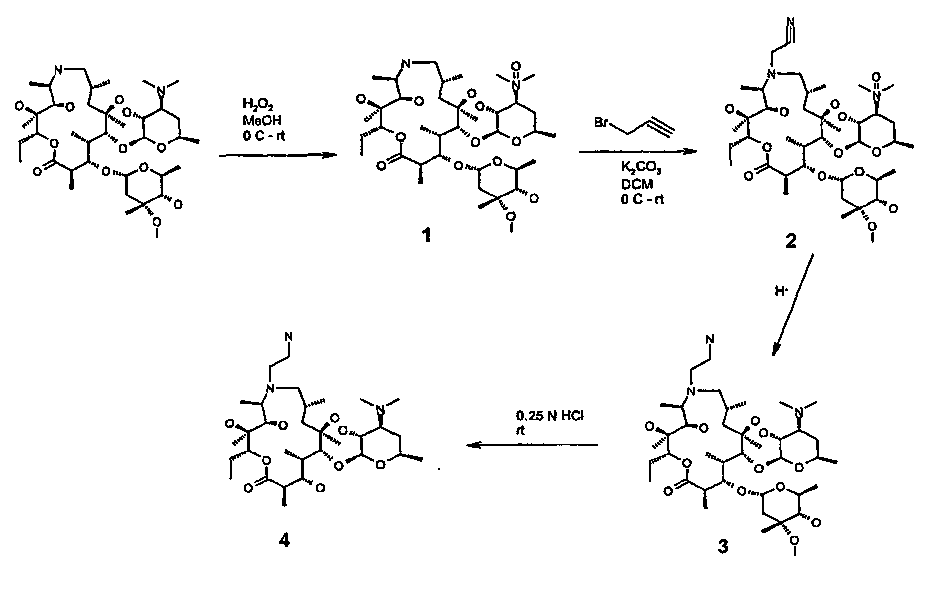

### Intermediate 1

### 9-Deoxo-9-dihydro-3'-N-oxide-9a-aza-9a-homoerythromycin A

To a solution of 9-deoxo-9-dihydro-9a-aza-9a-homoerythromycin A (20 g, 27.21 mmol) in MeOH (80 ml) at 0°C, a 30% water solution of H₂O₂ (30 ml) was added dropwise over 30 min. The reaction mixture was stirred for an additional 1.5 hour at room temperature. After detection of complete transformation the reaction mixture was poured into ice water (400 ml) and DCM (200 ml). A saturated water solution of Na₂S₂O₃ (150 ml) was added to remove excess of H₂O₂. The layers were separated and the water layer extracted with DCM (2 x 200 ml). Combined organic layers were evaporated under reduced pressure and the residue was precipitated from DCM-diisopropylether yielding intermediate 1 (21.5 g, 94.3 % yield).
**MS m/z: (ES): MH**⁺=751.6
**¹³C NMR (125 MHz, pyridine)/**δ**:** 177.3, 101.9, 96.2, 82.8, 77.6, 77.4, 76.9, 75.8, 73.2, 73.0, 72.8, 72.2, 71.9, 65.5, 65.0, 56.2, 55.8, 50.8, 48.6, 44.7, 42.3, 41.9, 34.2, 33.8, 29.1, 27.2, 21.3, 20.8, 20.5, 20.4, 18.3, 16.6, 14.1, 13.5, 10.4, 8.8.

### Intermediate 2

### 9-Deoxo-9-dihydro-3'-N-oxide-9a-cyanomethyl-9a-aza-9a-homoerythromycin A

To a DCM (200 ml) solution of **Intermediate 1,** (20 g, 26.63 mmol) K₂CO₃ (7.35 g, 53.26 mmol) was added and the reaction mixture was stirred for 10 minutes at room temperature. Then bromoacetonitrile (3.71 ml, 53.26 mmol) was added and the reaction mixture was stirred overnight at room temperature. The reaction mixture was washed with brine yielding after evaporation 20 g of the crude product. Precipitation from water yielded intermediate 2 (7.1 g, 31.31 % yield).
**MS m/z: (ES): MH**⁺=790.6
**¹³C NMR (125 MHz, pyridine)/**δ**:** 176.7, 116.9, 101.7, 94.5, 83.3, 77.5, 77.3, 76.7, 75.6, 74.9, 73.8, 73.0, 72.6, 71.4, 65.8, 65.0, 63.2, 60.8, 50.9, 48.6, 44.0, 41.7, 41.5, 36.8, 34.2, 33.7, 30.8, 25.6, 21.2, 20.7, 20.5, 20.4, 20.4, 18.1, 17.1, 13.7, 10.4, 9.1.

### Intermediate 3

### 9-Deoxo-9-dihydro-9a-(β-aminoethyl)-9a-aza-9a-homoerythromycin A

To the solution of **Intermediate 2** (3 g, 3.80 mmol) in THF (25 ml), LiB(OEt)₃H (10 ml, 1 M THF solution) was added dropwise over 20 minutes at -20°C. The reaction was stirred for 10 minutes at -20°C to complete conversion. To the reaction mixture water (50 ml) and DCM (50 ml) were added and gradient extraction was performed at pH 4.5 and 10. Evaporation of the combined organic extracts at pH 10 yielded 1.6 g of the crude product. Column chromatography using elution system DCM/MeOH/NH₄OH=90:9:0.5 yielded intermediate 3 (0.87 g, 29.5 % yield).
**MS m/z: (ES): MH**⁺=778.5.
**¹³C NMR (125 MHz, pyridine)/**δ**:** 177.8, 103.8, 96.9, 84.6, 80.2, 79.2, 78.4, 75.5, 75.4, 75.1, 74.1, 72.3, 70.4, 68.7, 66.6, 66.2, 62.9, 55.0, 50.1, 46.1, 41.9, 41.8, 41.0, 30.9, 30.4, 36.1, 27.6, 23.2, 22.3, 22.1, 20.1, 20.0, 17.7, 16.5, 11.8, 10.9, 9.0.

### Intermediate 4

### 3-O-Decladinosyl-9-deoxo-9-dihydro-9a-(β-aminoethyl)-9a-aza-9a-homoerythromycin A

A solution of **Intermediate 3** (1.5 g, 1.93 mmol) in 0.25 N HCl (50 ml) was stirred for 20 hours at room temperature. To the reaction mixture DCM (50 ml) was added and gradient extraction was performed at pH 1.1 and 9.5. Evaporation of the combined organic extracts at pH 9.5 yielded 0.98 g of crude product. Column chromatography using elution systemDCM/MeOH/NH₄OH=90:9:1.5 yielded intermediate 4 (0.76 g, 62.71 % yield).
**MS m/z: (ES): MH**⁺= 620.6.
**¹³C NMR (75 Mhz, DMSO)/δ:** 174.61, 102.24, 83.30, 76.15, 75.86, 75.20, 73.66, 73.30, 70.16, 67.82, 6 4.36, 43.63, 40.14, 37.39, 35.84, 30.31, 29.38, 25.67, 21.03, 20.87, 20.66, 16.53, 15.87, 10.27, 8.16.

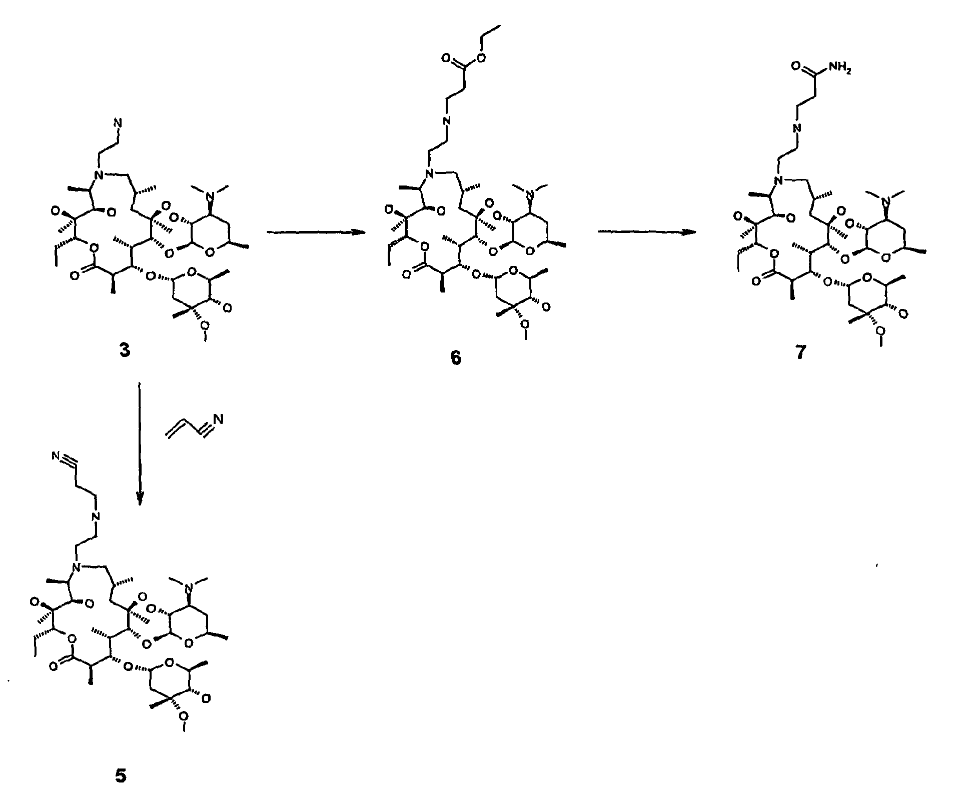

### Intermediate 5

### 9-Deoxo-9-dihydro-9a-[N'-(β-cyanoethyl)-β-aminoethyl]-9a-aza-9a-homoerythromycin A

To the MeOH (15 ml) solution of **Intermediate 3** (0.916 g, 1.177 mmol) solution of acrylonitrile (0.08 ml, 1.221 mmol) in MeOH (2 ml) was added dropwise. The reaction mixture was refluxed for 22 hours. After completing the reaction the solvent was evaporated and the residue extracted with DCM (3 x 30 ml). The crude product was purified by solid phase extraction technique (SPE 10 g) yielding title compound (0.632 g).
**MS m/z: (ES): MH⁺**= 831.4

### Intermediate 6

### 9-Deoxo-9-dihydro-9a-{N'-[β-(ethoxycarbonyl)ethyl]-β-amwoethyl}-9a-aza-9a-homoerythromycin A

To the EtOH (10 ml) solution of **Intermediate 3** (1.221 g, 1.569 mmol) ethyl acrylate (0.08 ml, 1.221 mmol) was added and the reaction mixture was stirred for 19 hours at room temperature. After completing the reaction, the solvent was evaporated and the residue was extracted with DCM (3 x 30 ml). The crude product was purified by solid phase extraction technique (SPE 10 g) yielding the title compound (0.685 g). **MS m/z: (ES): MH⁺**= 878.5.

### Intermediate 7

### 9-Deoxo-9-dihydro-9a-[N'-(β-amidoethyl)-β-aminoethyl]-9a-aza-9a-homoerythromycin A

To the EtOH (10 ml) solution of **Intermediate 6** (440 mg, 0.5 mmol) 25% ammonia solution (8 ml) was added and the reaction mixture was stirred at room temperature for three days. After evaporation of the solvent the crude residue was dissolved in DCM (30 ml), water (30 ml) was added and pH adjusted to 9.6. After extraction with DCM (2 x 30 ml) combined organic layers were dried over K₂CO₃, solvent evaporated yielding the title compound (300 mg).
**MS m/z: (ES): MH⁺**= 850.6

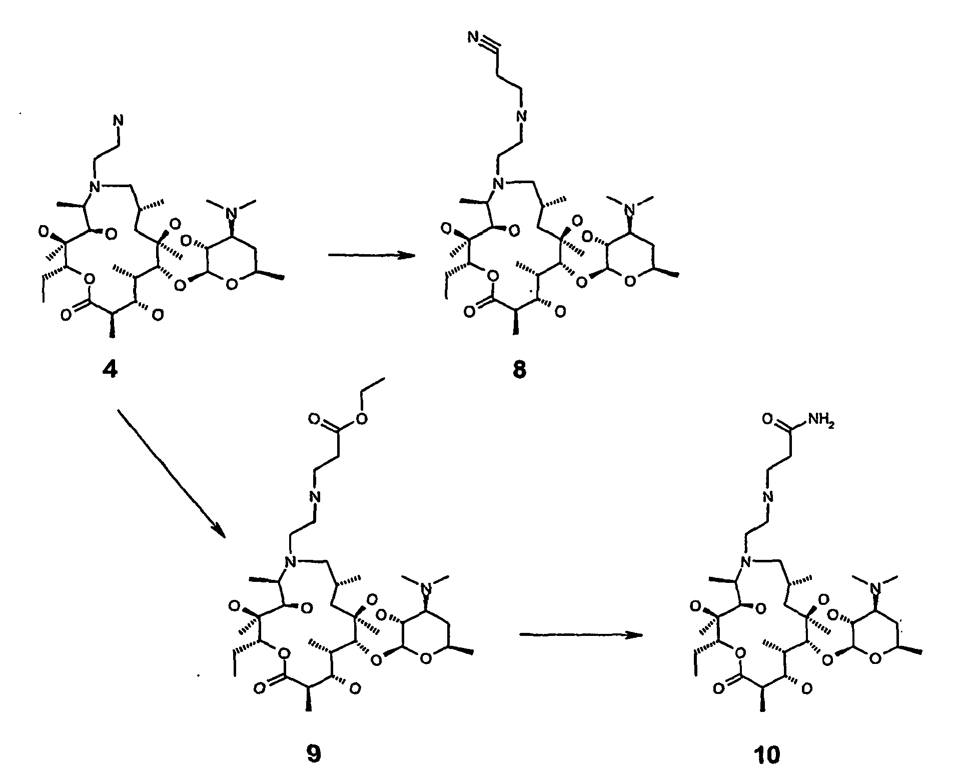

### Intermediate 8

### 3-O-Decladinosyl-9-deoxo-9-dihydro-9a-[N'-(β-cyanoethyl)-β-aminoethyl]-9a-aza-9a-homoerythromycin A

Starting from **Intermediate 4** and acrylonitrile and using the methods described for intermediate 5, the title compound is prepared.

### Intermediate 9

### 3-O-Decladinosyl-9-deoxo-9-dihydro-9a-{N'-[β-(ethoxycarbonyl)ethyl]-β-aminoethyl}-9a-aza-9a-homoerythromycin A

Starting from **Intermediate 4** and ethylacrylate and using the methods described for intermediate 6, the title compound is prepared.

### Intermediate 10

### 3-O-Decladinosyl-9-deoxo-9-dihydro-9a-[N'-(β-amidoethyl)-β-anxinoethyl]-9a-aza-9a-homoerythromycin A

Starting from **Intermediate 9** and 25% ammonia solution and using the methods described for intermediate 7, the title compound is prepared.

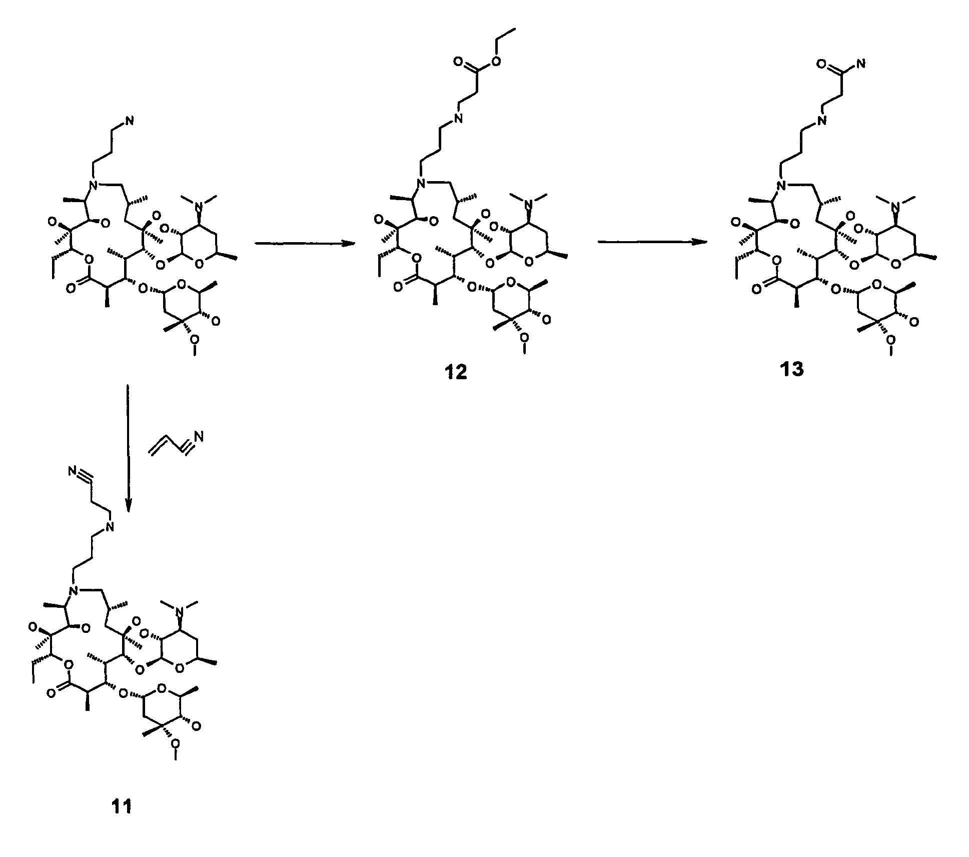

### Intermediate 11

### 9-deoxo-9-dihydro-9a-[N'-(β-cyanoethyl)-γ-aminopropyl]-9a-aza-9a-homoerythromycin A

9-Deoxo-9-dihydro-9a-γ-aminopropyl-9a-aza-9a-homoerythromycin A (6.98 g; 8.8 mmol) was dissolved in MeOH (200 ml) and acrylonitrile (581 µl; 8.8 mmol) was added. The reaction mixture was heated at 68 °C for 6 hours. Subsequently, the solvent was evaporated under reduced pressure. The crude product was purified on a silica gel column in the solvent system DCM:MeOH:NH₄OH = 90:9:1,5. to yield 4.4 g of the title compound
**MS m/z: (ES): MH⁺**= 845.3

### Intermediate 12

### 9-Deoxo-9-dihydro-9a-{N'-[β-(ethoxycarbonyl)ethyl]-γ-ammopropyl}-9a-aza-9a-homoerythromycin A

A solution of 9a-(γ-aminopropyl)-9a-aza-9-deoxo-9-dihydro-9a-homoerythromycin A (5.0 g; 6.3 mmol) and ethylacrylate (0.68 ml, 6.3 mmol) in MeOH (20 ml) was refluxed for 17 hours. The reaction mixture was evaporated to dryness, and to the residue water (50 ml) and DCM (50 ml) were added. Gradient extraction was performed at pH 7.2 and 11.2 giving after evaporation of combined organic extracts at pH 11.2 the title product (3.29 g).
**MS m/z: (ES): MH⁺**= 892.4.

### Intermediate 13

### 9-Deoxo-9-dihydro-9a-[N'-(β-amidoethyl)-γ-aminopropyl]-9a-aza-9a-homoerythromycin A

To a solution of **Intermediate 12** (3.6 g, 4 mmol) in ethanol (4 ml), a 25% ammonia solution (4 ml) was added. The reaction mixture was left at room temperature until complete conversion. The obtained crude product was purified by column chromatography in the solvent system DCM:MeOH:NH₄OH = 90:9:1.5 to yield the title product.
**MS m/z: (ES): MH⁺=** 863.3.

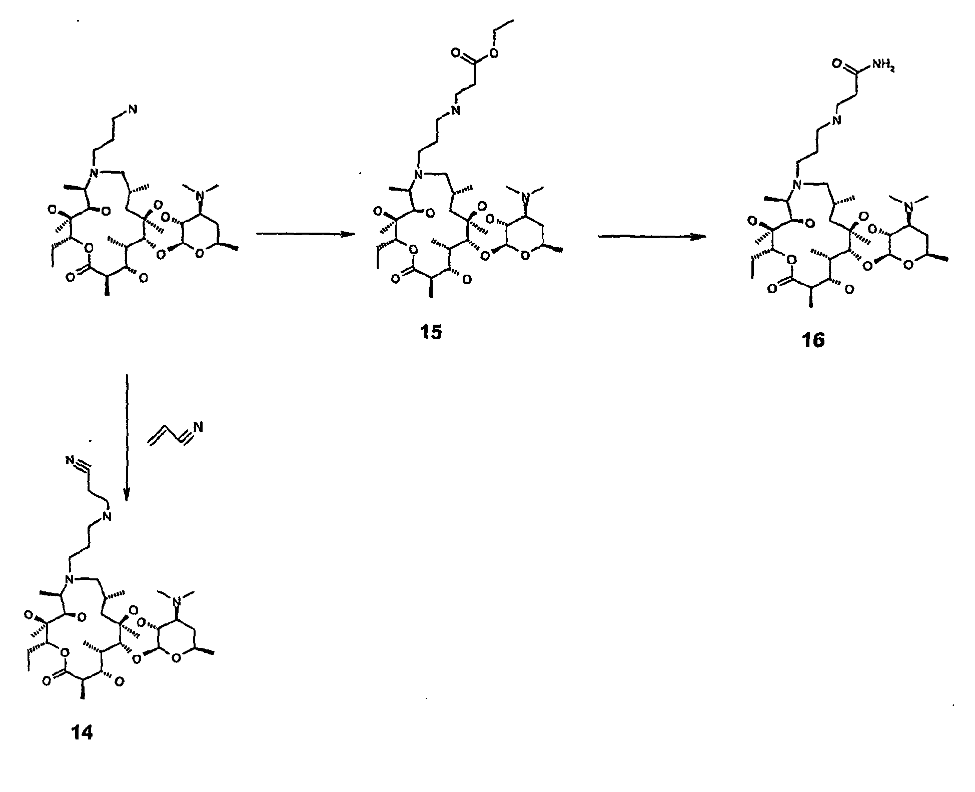

### Intermediate 14

### 3-O-Deladinosyl-9-deoxo-9-dihydro-9a-[N'-(β-cyanoethyl)-γ-aminopropyl]-9a-aza-9a-homoerythromycin A

Starting from 3-*O*-Decladinosyl-9-deoxo-9-dihydro-9a-γ-aminopropyl-9a-aza-9a-homoerythromycin according to procedure for **Intermediate 11** the title compound was prepared.
**MS m/z: (ES): MH⁺**= 687.2.

### Intermediate 15

### 3-O-Deladinosyl-9-deoxo-9-dihydro-9a-{N'-[β-(ethoxycarbonyl)ethyl]-γ-aminopropyl}-9a-aza-9a-homoerythromycin A

Starting from 3-*O*-Decladinosyl-9-Deoxo-9-dihydro-9a-γ-aminopropyl-9a-aza-9a-homoerythromycin according to procedure for **Intermediate 12** the title compound was prepared.
**MS m/z: (ES): MH⁺=** 734.3.

### Intermediate 16

### 3-O-Decladinosyl-9-deoxo-9-dihydro-9a-[N'-(β-amidoethyl)-γ-aminopropyl]-9a-aza-9a-homoerythromycin A

Starting from **Intermediate 15** according to procedure for **Intermediate 13** the title compound was prepared.
**MS m/z: (ES): MH⁺**= 705.2.

### Example 1

### 9-Deoxo-9-dihydro-9a-(N'-isopropylcarbamoyl-β-aminoethyl)-9a-aza-9a--homoerythromycin A

A mixture of **Intermediate 3** (1.0 g, 1.3 mmol) and of isopropylisocyanate (0.1 g, 1.3 mmol) in dry toluene (10 ml ) was stirred for 30 minutes at room temperature to complete the reaction. The crystals of the crude product were filtered and purified by column chromatography on silica gel using the solvent system DCM : MeOH : NH₄OH = 90 : 9 : 1.5, to give the titled compound
**MS m/z: (ES): MH⁺**=863.15.
**¹³C NMR (125 MHz, pyridine)**/δ: 177,5, 45,7, 80,3, 41,0, 83,9, 75,3, 40,4, 30,0 , 66,0, 61,7, 76,4, 75,2, 78,5, 22,1, 11,4, 103,4, 71,5, 65,9, 40,4, 30,8, 68,1, 96,7, 35,8, 73,7, 49,7, 78,8, 66,3, 16,2, 10,5, 27,7, 22,4, 9,3, 18,2, 21,9, 19,5, 21,6, 53,1; 39,6, 158,8, 42,2, 23,6.

### Example 2

### 9-Deoxo-9-dihydro-9a-[N'-(1-naphtyl)carbamoyl-β-aminoethyl]-9a-aza-9a--homoerythromycin A

A mixture of Intermediate 3 (1.0 g, 1.3 mmol) and 1-naphtylisocyanate (0.22 g, 1.3 mmol) in dry toluene (10 ml) was stirred for 30 minutes at room temperature to complete the reaction. The crystals of the crude product were filtered and purified by column chromatography on silica gel using the solvent system DCM: MeOH : NH₄OH = 90 : 9 : 1.5, to give 596 mg of the title compound..
**MS m/z: (ES): MH⁺**=947.23.
**¹³C NMR (125 MHz, pyridine)**/δ: 178,1, 46,2, 80,6), 41,6, 84,4, 75,8, 41,1, 30,4, 66,6, 61,9, 77,2, 75,8, 79,2, 22,7, 11,9, 104,0, 72,2, 66,2, 41,0, 30,7, 68,8, 97,0, 36,2, 74,1, 50,1, 79,2, 66,8, 16,5, 11,1, 28,1, 22,8, 9,9, 18,9, 22,4, 20,0, 22,0, 53,2; 40,4, 157,6, 136,7, 135,2, 129,2, 128,6, 126,9, 126,5, 126,2, 124,1, 123,3, 120,4.

### Example 3

### 9-Deoxo-9-dihydro-9a-(N'-bemylcarbamoyl-β-aminoethyl)-9a-aza-9a--homoerythromycin A

A mixture of **Intemediate 3** (1.0 g, 1.3 mmol) and benzylisocyanate (0.17 g, 1.3 mmol) in dry toluene (10 ml) was stirred for 30 minutes at room temperature to complete the reaction. The crystals of the crude product were filtered and purified by column chromatography on silica gel using the solvent system DCM-chloride : MeOH : NH₄OH = 90 : 9 : 1.5, to give the title compound.
**MS m/z: (ES): MH⁺**=911.2.
**¹³C NMR (125 MHz, pyridine)**/δ: 178,0, 46,0, 80,6, 41,4, 84,3, 75,6 , 40,1, 30,2, 66,3, 61,8, 76,9, 75,6, 78,9, 22,5, 11,8, 103,8, 71,8, 66,3, 40,8, 31,2, 68,5, 97,0, 36,1, 74,0, 50,1, 79,1, 66,6, 16,4), 10,8, 28,2, 22,8, 9,6, 18,6, 22,2, 19,8, 21,9, 53); 40,1, 159,9, 142,0, 127,3, 128,2, 129,0.

### Example 4

### 9-Deoxo-9-dihydro-9a-(N'-benzylthiocarbamoyl-β-aminoethyl)-9a-aza-9a--homoerythromycin A

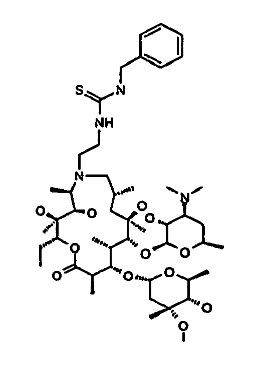

A mixture of **Intermediate 3** (1.0 g, 1.3 mmol) and benzylisothiocyanate (0.17 g, 1.3 mmol) in dry toluene (10 ml) was stirred for 30 minutes at room temperature to complete the reaction. The crystals of the crude product were filtered, wherefrom by chromatography on silica gel column using the solvent system methylene-chloride : methanol : ammonia= 90 : 9 : 1.5, the title compound was obtained.
**MS m/z: (ES): MH⁺**= 927.3.
**¹³C NMR (75 MHz, DMSO)**/δ: 176.23, 139.27, 128.09, 127.12, 126.64, 101.91, 94.91, 82.58, 77.89, 77.26, 76.49, 75.33, 74.16, 73.52, 72.64, 70.56, 67.02, 64.81, 64.51, 63.00, 59.37, 49.87, 48.71, 46.76, 44.11, 41.93, 40.35, 40.26, 40.23, 34.73, 29.94, 27.99, 26.93, 22.15, 21.34, 21.24, 20.89, 18.38, 18.13, 14.86, 10.90, 9.52, 9.38.

### Example 5

### 9-Deoxo-9-dihydro-9a-[N'-(1-naphtyl)thiocarbamoyl-β-ammoethyl-9a-aza--9a-homoerythromycin A

A mixture of **Intermediate 3** (1.0 g, 1.3 mmol) and 1-naphtylisothiocyanate (0.17 g (1.3 mmol) in dry toluene (10 ml) was stirred for 30 minutes at room temperature to complete the reaction. The crystals of the crude product were filtered and purified by column chromatography on silica gel using the solvent system DCM : MeOH : NH₄OH = 90 : 9 : 1.5, to give 625 mg of the title product.
**MS m/z: (ES): MH⁺**= 962.29.
**¹³C NMR (75 MHz, pyridine)**/δ: 183.91, 178.15, 131.46, 128.98, 128.03, 127.21, 127.05, 126.24, 126.11, 124.35, 103.73, 96.42, 84.53, 79.62, 79.14, 78.44, 76.08, 75.66, 75.26, 74.08, 71.79, 68.41, 66.57, 66.32, 50.79, 50.06, 46.01, 43.94, 42.60, 40.87, 35.94, 31.37, 29.18, 28.38, 23.08, 22.85, 22.20, 21.99, 19.74, 18.91, 15.86, 12.00, 10.72.

### Example 6

### 9-Deoxo-9-dihydro-9a-[N'-(2-trifluoromethyl)phenylcarbamoyl-β--aminoethyl]-9a-aza-9a-homoerythromycin A

According to the procedure of **Example 1** the title compound is prepared starting from Intermediate 3 and 2-(trifluoromethyl)phenylisocyanate.

### Example 7

### 9-Deoxo-9-dihydro-9a-[N'-(3-phenylpropyl)thiocarbamoyl-β-aminoethyl]--9a-aza-9a-homoerythromycin A

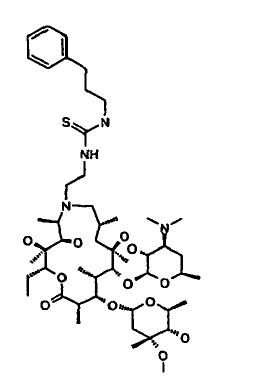

A mixture of **Intermediate 3** (1.0 g, 1.3 mmol) and 3-phenylpropylisothiocyanate (0.22 g, 1.3 mmol) in dry toluene (10 ml) was stirred for 30 minutes at room temperature to complete the reaction. The crystals of the crude product were filtered, wherefrom by chromatography on silica gel column using the solvent system methylene-chloride : methanol : ammonia= 90 : 9 : 1.5, the title compound was obtained.
**¹³C NMR (75 MHz, DMSO)**/δ: 176.25, 139.29, 128.54, 128.22, 125.98, 101.89, 94.91, 82.58, 77.87, 77.27, 76.48, 75.29, 74.16, 73.51, 72.64, 70.52, 67.00, 64.82, 64.53, 63.00, 59.47, 49.87, 48.71, 45.00, 44.12, 41.74, 40.35, 40.26, 40.22, 34.73, 29.91, 27.98, 26.93, 22.17, 21.33, 21.24, 20.89, 18.39, 18.12, 14.88, 10.90, 9.56, 9.37.
**MS m/z: (ES): MH⁺**= 955.4.

### Example 8

### 9-Deoxo-9-dihydro-9a-[N'-(β-phenylethyl)carbamoyl-β-anumoethyl]-9a-aza--9a-homoerythromycin A

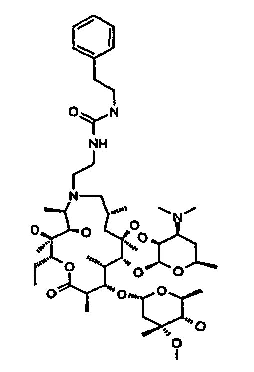

A mixture of **Intermediate 3** (1.0 g, 1.3 mmol) and β-phenylethylisocyanate (0.21 g, 1.3 mmol) in dry toluene (10 ml) was stirred for 30 minutes at room temperature to complete the reaction. The crystals of the crude product were filtered, wherefrom by chromatography on silica gel column using the solvent system methylene-chloride : methanol: ammonia= 90 : 9 : 1.5, the title compound was obtained.
**MS m/z: (ES): MH⁺**= 925.4.
**¹³C NMR (75 MHz, DMSO)**/δ: 176.43, 158.08, 139.91, 128.76, 128.41, 126.07, 102.17, 95.19, 82.76, 78.19, 77.51, 76.76, 75.46, 74.41, 73.69, 72.87, 70.81, 67.24, 65.03, 64.75, 63.00, 60.00, 51.50, 48.95, 44.36, 40.15, 40.51, 40.48, 38.19, 36.34, 34.99, 30.21, 28.33, 27.18, 22.35, 21.58, 21.45, 21.12, 18.63, 18.31, 15.18, 11.11, 9.61,9.46.

### Example 9

### 9-Deoxo-9-dihydro-9a-(N'-ethoxycarbonylmethylcarbamoyl-β-aminoethyl)--9a-aza-9a-homoerythromycin A

According to the procedure of **Example 1** the title compound is prepared starting from Intermediate 3 and ethoxycarbonylmethylisocyanate.

### Example 10

### 9-Deoxo-9-dihydro-9a-{N'-[1-(1-naphtyl)ethylcarbamoyl-β-aminoethyl}-9a--aza-9a-homoerythromycin A

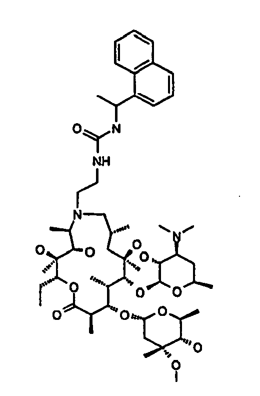

A mixture of **Intermediate 3** (1.0 g, 1.3 mmol) and 1-(1-naphtyl)ethylisocyanate (0.25 g, 1.3 mmol) in dry toluene (10 ml) was stirred for 30 minutes at room temperature to complete the reaction. The crystals of the crude product were filtered, wherefrom by chromatography on silica gel column using the solvent system methylene-chloride : methanol : ammonia= 90 : 9 : 1.5, the title compound was obtained.
**MS m/z: (ES): MH⁺**= 975.5.
**¹³C NMR (75 MHz, DMSO)**/δ: 176.21, 157.08, 141.26, 133.31, 130.27, 128.49, 126.91, 125.94, 125.37, 123.18, 121.87, 101.91, 94.98, 82.58, 77.99, 77.28, 76.55, 75.23, 74.18, 73.47, 72.65, 70.54, 67.00, 64.82, 64.53, 48.73, 44.58, 44.14, 40.35, 40.28, 40.23, 34.75, 29.95, 26.92, 22.37, 22.31, 21.34, 21.22, 21.05, 20.90, 18.41, 18.08, 14.97, 10.88, 9.38.

### Example 11

### 9-Deoxo-9-dihydro-9a-[N'-(3,4,5-trimethoxyphenyl)carbamoyl-β--aminoethyl]-9a-aza-9a-homoerythromycin A

According to the procedure of **Example 1** the title compound is prepared starting from **Intermediate 3** and 3,4,5-trimethoxyphenylisocyanate.

### Example 12

### 9-Deoxo-9-dihydro-9a-[N'-(2-naphtyl)carbamoyl-β-amwoethyl]-9a-aza-9a--homoerythromycin A

A mixture of **Intermediate 3** (1.0, 1.3 mmol) and 2-naphtylisocyanate (0.23 g, 1.3 mmol) in dry toluene (10 ml) was stirred for 30 minutes at room temperature to complete the reaction. The crystals of the crude product were filtered and purified by column chromatography on silica gel using the solvent system DCM : MeOH : NH₄OH = 90 : 9 : 1.5, to give the title compound.
**MS m/z: (ES): MR⁺** = 947.23.
**¹³C NMR (125 MHz, pyridine)**/δ: 178,2, 46,5, 81,4, 40,9, 84,2, 76,1, 40,0, 31,2, 66,8, 62,1, 77,9, 75,7, 79,8, 22,6, 11,7, 104,0, 72,3, 66,2 , 40,9, 30,6, 68,7, 97,6, 36,4, 74,1, 50,2, 79,2, 66,8, 17,1, 11,1, 28,0, 22,4, 9,7, 18,8, 22,4, 20,0, 22,0, 53,2; 39,9, 156,7, 140,0, 135,4, 130,2, 128,8, 128,4, 127,8, 126,9, 123,9, 121,1, 114,4.

### Example 13

### 9-Deoxo-9-dihydro-9a-[N'-(2,4-dichlorophemyl)carbamoyl-β-ammoethyl]-9a--aza-9a-homoerythromycin A

According to the procedure of **Example 1** the title compound is prepared starting from **Intermediate 3** and 2,4-dichlorophenylisocyanate.

### Example 14

### 3-O-Decladinosyl-9-deoxo-9-dihydro-9a-(N'-isopropylcarbamoyl-β--aminoethyl)-9a-aza-9a-homoerythromycin A

According to the procedure of **Example 1** the title compound is prepared starting from **Intermediate 4** and isopropylisocyanate.

### Example 15

### 3-O-Decladinosyl-9-deoxo-9-dihydro-9a-[N'-(1-naphtyl)carbamoyl-β-aminoethyl]-9a-aza-9a-homoerythromycin A

According to the procedure of **Example 1** the title compound is prepared starting from **Intermediate 4** and 1-naphtylisocyanate.

### Example 16

### 3-O-Decladinosyl-9-deoxo-9-dihydro-9a-(N'-benzylcarbamoyl-β--aminoethyl)-9a-aza-9a-homoerythromycin A

According to the procedure of **Example 1** the title compound is prepared starting from **Intermediate 4** and benzylisocyanate.

### Example 17

### 3-O-Decladinosyl -9-deoxo-9-dihydro-9a-(N'-benzylthiocarbamoyl-β--aminoethyl)-9a-aza-9a-homoerythromycin A

According to the procedure of **Example 1** the title compound is prepared starting from **Intermediate 4** and benzylisothiocyanate.

### Example 18

### 3-O-Decladinosyl-9-deoxo-9-dihydro-9a-[N'-(1-naphtyl)thiocarbamoyl-β-aminoethyl]-9a-aza-9a-homoerythromycin A

According to the procedure of **Example 1** the title compound is prepared starting from **Intermediate 4** and 1-naphtylisothiocyanate.

### Example 19

### 3-O-Decladinosyl-9-deoxo-9-dihydro-9a-[N'-(2-(trifluoromethyl)phenylcarbamoyl)-β-aminoethyl]-9a-aza-9a--homoerythromycin A

According to the procedure of **Example 1** the title compound is prepared starting from **Intermediate 4** and 2-(trifluoromethyl)phenylisocyanate.

### Example 20

### 3-O-Decladinosyl-9-deoxo-9-dihydro-9a-[N'-(3-phenylpropyl)thiocarbamoyl--β-aminoethyl]-9a-aza-9a-homoerythromycin A

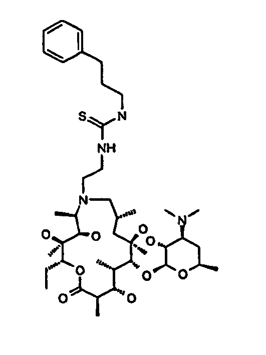

A mixture of **Intermediate 4** (1.0 g, 1.6 mmol) and 3-phenylpropylisothiocyanate 0.28 g (1.6 mmol) in dry toluene (10 ml) was stirred for 30 minutes at room temperature to complete the reaction. The crystals of the crude product were filtered, wherefrom by chromatography on silica gel column using the solvent system methylene-chloride : methanol : ammonia= 90 : 20 : 1.5, the title compound was obtained.
**MS m/z: (ES): MH⁺**= 797.3.
**¹³C NMR (75 MHz, DMSO)**/δ**:** 175.51, 141.83, 128.39, 128.38, 125.85, 103.53, 76.78, 76.39, 74.52, 73.81, 70.44, 68.54, 64.69, 43.94, 40.56, 40.49, 32.67, 30.61, 30.49, 26.76, 21.22, 16.12, 10.83, 8.52.

### Example 21

### 3-O-Decladinosyl-9-deoxo-9-dihydro-9a-[N'-(β-phenylethyl)thiocarbamoyl-β-aminoethyl]-9a-aza-9a-homoerythromycin A

According to the procedure of **Example 1** the title compound is prepared starting from **Intermediate 4** and β-phenylethylisothiocyanate.

### Example 22

### 3-O-Decladinosyl-9-deoxo-9-dihydro-9a-(N'-ethoxycarbonyLmethyl-carbamoyl-β-aminoethyl)-9a-aza-9a-homoerythromycin A

According to the procedure of **Example 1** the title compound is prepared starting from **Intermediate 4** and ethoxycarbonylmethylisocyanate.

### Example 23

### 3-O-Decladinosyl-9-deoxo-9-dihydro-9a-[N'-(2-naphtyl)carbamoyl-β--aminoethyl]-9a-aza-9a-homoerythromycin A

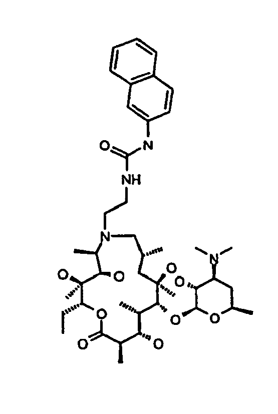

A mixture of **Intermediate 4** (1.0 g, 1.6 mmol) and 2--naphtylisocyanate (0.27 g, 1.6 mmol) in dry toluene (10 ml) was stirred for 30 minutes at room temperature to complete the reaction. The crystals of the crude product were filtered, wherefrom by chromatography on silica gel column using the solvent system methylene-chloride : methanol : ammonia= 90 : 20 : 1.5, the title compound was obtained.
**MS m/z: (ES): MH⁺**= 789.7.
**¹³C NMR (75 MHz, DMSO)**/δ: 175.11, 154.86, 137.94, 133.48, 128.32, 127.77, 127.03, 126.38, 125.82, 123.11, 119.18, 112.14, 103.05, 76.27, 75.86, 74.05, 73.29, 69.96, 68.05, 64.19, 43.50, 40.08, 30.00, 26.27, 21.03, 20.75, 17.50, 15.66, 10.35, 8.03.

### Example 24

### 3-O-Decladinosyl-9-deoxo-9-dihydro-9a-{N'-[1-(1-naphtyl)ethyl]carbamoyl-β-aminoethyl}-9a-aza-9a-homoerythromycin A

According to the procedure of **Example 1** the title compound is prepared starting from **Intermediate 4** and 1-(1-naphtyl)ethylisocyanate.

### Example 25

### 3-O-Decladinosyl-9-deoxo-9-dihydro-9a-[N'-(3,4,5-trimethoxyphenyl)-carbamoyl-β-aminoethyl]-9a-aza-9a-homoerythromycin A

According to the procedure of **Example 1** the title compound is prepared starting from **Intermediate 4** and 3,4,5-trimethoxyphenylisocyanate.

### Example 26

### 3-O-Decladinosyl-9-deoxo-9-dihydro-9a-[N'-(2,4-dichlorophenyl)carbamoyl-β-aminoethyl]-9a-aza-9a-homoerythromycin A

According to the procedure of **Example 1** the title compound is prepared starting from **Intermediate 4** and 2,4-dichlorophenylisocyanate.

### Example 27

### 9-Deoxo-9-dihydro-9a-[N'-(-cyanoethyl)-N'-isopropylcarbamoyl-β--aminoethyl]-9a-aza-9a-homoerythromycin A

According to the procedure of **Example 1** the title compound is prepared starting from **Intermediate 5** and isopropylisocyanate.

### Example 28

### 9-Deoxo-9-dihydro-9a-[N'-(β-cyanoethyl)-N'-(1-naphtyl)carbamoyl-β--aminoethyl]-9a-aza-9a-homoerythromycin A

According to the procedure of **Example 1** the title compound is prepared starting from **Intermediate 5** and 1-naphtylisocyanate.

### Example 29

### 9-Deoxo-9-dihydro-9a-[N'-(β-cyanoethyl)-N'-benzylcarbamoyl-β--aminoethyl]-9a-aza-9a-homoerythromycin A

According to the procedure of **Example 1** the title compound is prepared starting from **Intermediate 5** and benzylisocyanate.

### Example 30

### 9-Deoxo-9-dihydro-9a-[N'-(β-cyanoethyl)-N'-benzylthiocarbamoyl-β--aminoethyl]-9a-aza-9a-homoerythromycin A

According to the procedure of **Example 1** the title compound is prepared starting from **Intermediate 5** and benzylisothiocyanate.

### Example 31

### 9-Deoxo-9-dihydro-9a-[N'-(β-cyanoethyl)-N'-(1-naphtyl)thiocarbamoyl-β--aminoethyl]-9a-aza-9a-homoerythromycin A

According to the procedure of **Example 1** the title compound is prepared starting from **Intermediate 5** and 1-naphtylisothiocyanate.

### Example 32

### 9-Deoxo-9-dihydro-9a-[N'-(β-cyanoethyl)-N'-(2-(trifluoromethyl)phenyl)carbamoyl-β-aminoethyl]-9a-aza-9a--homoerythromycin A

According to the procedure of **Example 1** the title compound is prepared starting from **Intermediate 5** and 2-(trifluoromethyl)phenylisocyanate.

### Example 33

### 9-Deoxo-9-dihydro-9a-[N'-(β-cyanoethyl)-N'-(3-phenylpropyl)carbamoyl-β--aminoethyl]-9a-aza-9a-homoerythromycin A

According to the procedure of **Example 1** the title compound is prepared starting from **Intermediate 5** and 3-phenylethylisocyanate.

### Example 34

### 9-Deoxo-9-dihydro-9a-[N'-(β-cyanoethyl)-N'-(β-phenylethyl)thiocarbamoyl-β--aminoethyl]-9a-aza-9a-homoerythromycin A

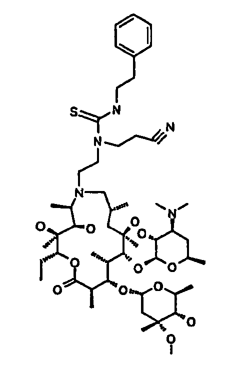

A mixture of **Intermediate 5** (0.5 g, 0.6 mmol) and β-phenylethylisothiocyanate (0.10 g, 0.6 mmol) in dry toluene (10 ml) was stirred for 30 minutes at room temperature to complete the reaction. The crystals of the crude product were filtered, wherefrom by chromatography on silica gel column using the solvent system methylene-chloride : methanol : ammonia= 90 : 9 : 1.5, the title compound was obtained.
**MS m/z: (ES): MH⁺**= 994.5.

### Example 35

### 9-Deoxo-9-dihydro-9a-[N'-(β-cyanoethyl)-N'-ethoxycarbonylmethyl- carbamoyl]-β-aminoethyl]-9a-aza-9a-homoerythromycin A

According to the procedure of **Example 1** the title compound is prepared starting from **Intermediate 5** and ethoxycarbonylmethylisocyanate.

### Example 36

### 9-Deoxo-9-dihydro-9a-{N'-(β-cyanoethyl)-N'-[1-(1-naphtyl)ethyl]carbamoyl-β--aminoethyl}-9a-aza-9a-homoerythromycin A

According to the procedure of **Example 1** the title compound is prepared starting from **Intermediate 5** and 1-(1-naphtyl)ethylisocyanate.

### Example 37

### 9-Deoxo-9-dihydro-9a-[N'-(β-cyanoethyl)-N'-(3,4,5--trimethoxyphenyl)carbamoyl-β-aminoethyl]-9a-aza-9a-homoerythromycin A

According to the procedure of **Example 1** the title compound is prepared starting from **Intermediate 5** and 3,4,5-trimethoxyphenylisocyanate.

### Example 38

### 9-Deoxo-9-dihydro-9a-[N'-(β-cyanoethyl)-N'-(2-naphtyl)carbamoyl-β--aminoethyl]-9a-aza-9a-homoerythroniycin A

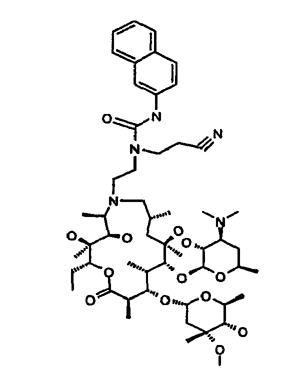

A mixture of **Intermediate 5** (0.5 g, 0.6 mmol) and 2-naphtylisocyanate (0.11 g, 0.6 mmol) in dry toluene (10 ml) was stirred for 30 minutes at room temperature to complete the reaction. The crystals of the crude product were filtered, wherefrom by chromatography on silica gel column using the solvent system methylene-chloride : methanol: ammonia= 90 : 9 : 1.5, the title compound was obtained.
**MS m/z: (ES): MH⁺**= 1000.5.

### Example 39

### 9-Deoxo-9-dihydro-9a-[N'-(β-cyanoethyl)-N'-(2,4-dichiorophenyl)carbamoyl--β-aminoethyl]-9a-aza-9a-homoerythromycin A

According to the procedure of **Example 1** the title compound is prepared starting from **Intermediate 5** and 2,4-dichlorophenylisocyanate.

### Example 40

### 3-O-Decladinosyl-9-deoxo-9-dihydro-9a-[N'-(β-cyanoethyl)-N'--isopropylcarbamoyl-β-aminoethyl]-9a-aza-9a-homoerythromycin A

According to the procedure of **Example 1** the title compound is prepared starting from **Intermediate 8** and isopropylisocyanate.

### Example 41

### 3-O-Decladinosyl-9-deoxo-9-dihydro-9a-[N'-(β-cyanoethyl)-N'-(1--naphtyl)carbamoyl-β-aminoethyl]-9a-aza-9a-homoerythromycin A

According to the procedure of **Example 1** the title compound is prepared starting from **Intermediate 8** and 1-naphtylisocyanate.

### Example 42

### 3-O-Decladmosyl-9-deoxo-9-dihydro-9a-[N'-(β-cyanoethyl)-N'--benzylcarbamoyl-β-aminoethyl]-9a-aza-9a-homoerythromycin A

According to the procedure of **Example 1** the title compound is prepared starting from **Intermediate 8** and benzylisocyanate.

### Example 43

### 3-O-Decladinosyl-9-deoxo-9-dihydro-9a-[N'-(β-cyanoethyl)-N'--benzylthiocarbamoyl-β-aminoethyl]-9a-aza-9a-homoerythromycin A

According to the procedure of **Example 1** the title compound is prepared starting from **Intermediate 8** and benzylisothiocyanate.

### Example 44

### 3-O-Decladinosyl-9-deoxo-9-dihydro-9a-[N'-(β-cyanoethyl)-N'-(1--naphtyl)thiocarbamoyl-β-aminoethyl]-9a-aza-9a-homoerythromycin A

According to the procedure of **Example 1** the title compound is prepared starting from **Intermediate 8** and 1-naphtylisothiocyanate.

### Example 45

### 3-O-Decladinosyl-9-deoxo-9-dihydro-9a-[N'-(β-cyanoethyl)-N'-(2--(trifluoromethyl)phenyl)carbamoyl-β-aminoethyl]-9a-aza-9a--homoerythromycin A

According to the procedure of **Example 1** the title compound is prepared starting from **Intermediate 8** and 2-(trifluoromethyl)phenylisocyanate.

### Example 46

### 3-O-Decladinosyl-9-deoxo-9-dihydro-9a-[N'-(β-cyanoethyl)-N'-(3-phenylpropyl)thiocarbamoyl-β-aminoethyl]-9a-aza-9a-homoerythromycin A

According to the procedure of **Example 1** the title compound is prepared starting from **Intermediate 8** and 3-phenylethylisothiocyanate.

### Example 47

### 3-O-Decladinosyl-9-deoxo-9-dihydro-9a-[N'-(β-cyanoethyl)-N'-(β--phenylethyl)thiocarbamoyl-β-aminoethyl]-9a-aza-9a-homoerythromycin A

According to the procedure of **Example 1** the title compound is prepared starting from **Intermediate 8** and β-phenylethylisothiocyanate.

### Example 48

### 3-O-Decladinosyl-9-deoxo-9-dihydro-9a-[N'-(β-cyanoethyl)-N'-ethoxy-carbonylmethylcarbamoyl-β-aminoethyl]-9a-aza-9a-homoerythromycin A

According to the procedure of **Example 1** the title compound is prepared starting from **Intermediate 8** and β-phenylethylisocyanate.

### Example 49

### 3-O-Decladinosyl-9-deoxo-9-dihydro-9a-[N'-(β-cyanoethyl)-N'-(2--naphtyl)carbamoyl-β-aminoethyl]-9a-aza-9a-homoerythromycin A

According to the procedure of **Example 1** the title compound is prepared starting from **Intermediate 8** and 2-naphtylisocyanate.

### Example 50

### 3-O-Decladinosyl-9-deoxo-9-dihydro-9a-[N'-(β-cyanoethyl)-N'-[1-(1--naphtyl)ethylcarbamoyl-β-aminoethyl]-9a-aza-9a-homoerythromycin A

According to the procedure of **Example 1** the title compound is prepared starting from **Intermediate 8** and 1-(1-naphtyl)ethylisocyanate.

### Example 51

### 3-O-Decladinosyl-9-deoxo-9-dihydro-9a-[N'-(β-cyanoethyl)-N'-(3,4,5--trimethoxyphenyl)carbamoyl-β-aminoethyl]-9a-aza-9a-homoerythromycin A

According to the procedure of **Example 1** the title compound is prepared starting from **Intermediate 8** and 3,4,5-trimethoxyphenylisocyanate.

### Example 52

### 3-O-Decladinosyl-9-deoxo-9-dihydro-9a-[N'-(β-cyanoethyl)-N'-(2,4--dichlorophenyl)carbamoyl-β-aminoethyl]-9a-aza-9a-homoerythromycin A

According to the procedure of **Example 1** the title compound is prepared starting from **Intermediate 8** and 2,4-dichlorophenylisocyanate.

### Examples 53 to 151

### General Procedure:

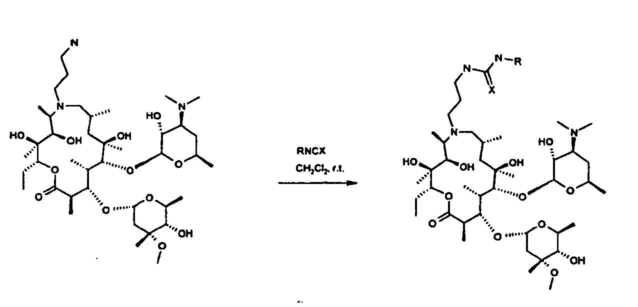

To a solution of 9a-(y-aminopropyl)-9a-aza-9-deoxo-9-dihydro-9a-homoerythromycin A (30 mg) in DCM (1 mL) was added 1.3 equivalents of the corresponding isocyanate or thioisocyanate reagents. The mixture was stirred for 1-48 hours, and then the scavenger resin (trisamine polymer bound, 3 eq.) was added. After 1 day the resin was filtered off and washed with DCM (1 mL). The solvent was evaporated giving the desired product.

The Table 1 that follows gives the structures of isocynate or thioisocynate reagent (RNCX), as well as the products of formula (I).

**Table 1**

| **Example** | **RNCX** | **Product** | **MS (ES, m/z)** | **mass/m g** | **purity %** |
|---|---|---|---|---|---|
| **Comparative Example 53** | 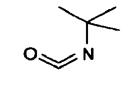 | 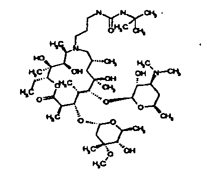 | 891,3 | 16,8 | 90,3 |
| **Comparative Example 54** | 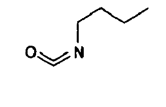 | 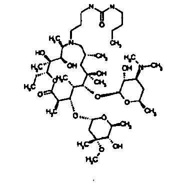 | 891,2 | 16,1 | 95,4 |
| **Comparative Example 55** | 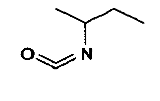 | 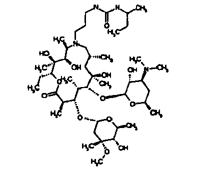 | 891,2 | 16,8 | 91 |
| **Example 56** | 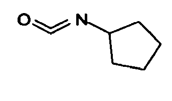 | 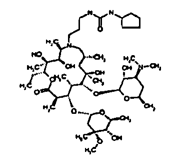 | 903,2 | 16 | 91,6 |
| **Example 57** | 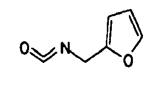 | 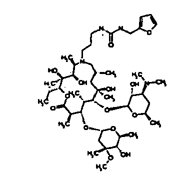 | 915,2 | 13,7 | 90,9 |
| **Example 58** | 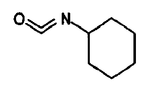 | 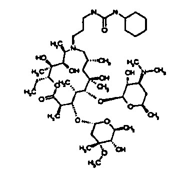 | 917,2 | 17,5 | 92,3 |
| **Example 59** | 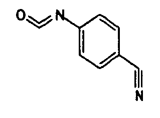 | 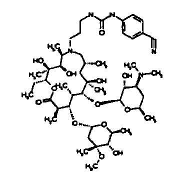 | 936,3 | 12,6 | 93,4 |
| **Comparative Example 60** | 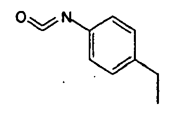 | 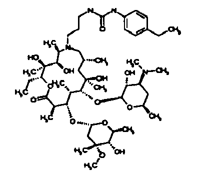 | 939,3 | 12,6 | 90,1 |
| **Comparative Example 61** | 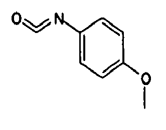 | 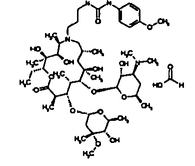 | 941,2 | 3,8 | 90 |
| **Comparative Example 62** | 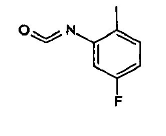 | 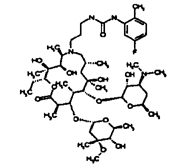 | 943,3 | 15,2 | 93,3 |
| **Example 63** | 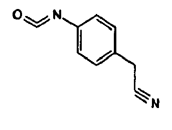 | 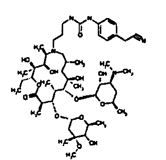 | 950,3 | 15,2 | 92 |
| **Example 64** | 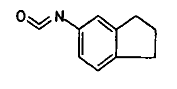 | 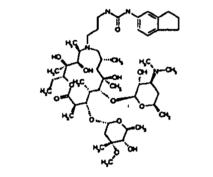 | 951,3 | 14,1 | 93,7 |
| **Example 65** | 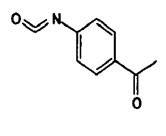 | 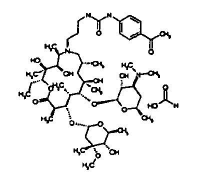 | 953,2 | 4 | 97,1 |
| **Comparative Example 66** | 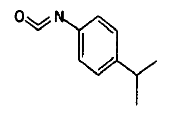 | 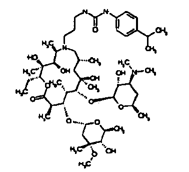 | 953,3 | 16,2 | 92,1 |
| **Comparative Example 67** | 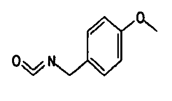 | 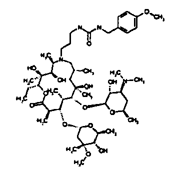 | 955,2 | 14,9 | 93,2 |
| **Example 68** | 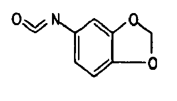 | 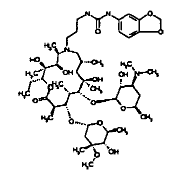 | 955,2 | 16,5 | 94,6 |
| **Example 69** | 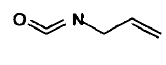 | 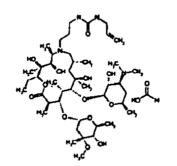 | 875,2 | 3,2 | 93,6 |
| **Comparative Example 70** | 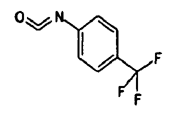 | 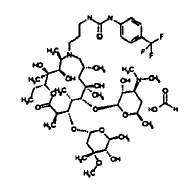 | 979,2 | 2,2 | 89,2 |
| **Example 71** | 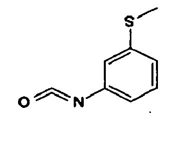 | 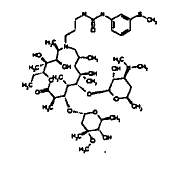 | 957,3 | 19,9 | 91,2 |
| **Example 72** | 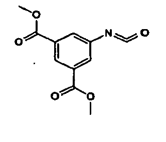 | 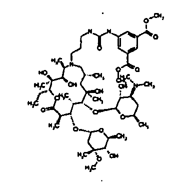 | 1027,3 | 16,2 | 94 |
| **Comparative Example 73** | 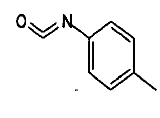 | 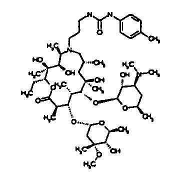 | 925,2 | 16,6 | 90 |
| **Example 74** | 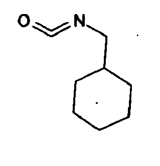 | 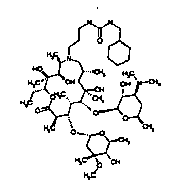 | 931,3 | 11 | 91,3 |
| **Comparative Example 75** | 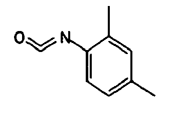 | 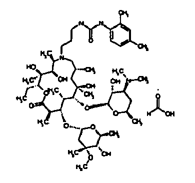 | 939,3 | 18 | 96,5 |
| **Comparative Example 76** | 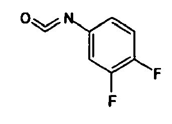 | 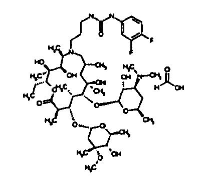 | 947,2 | 10,8 | 99,1 |
| **Comparative Example 77** | 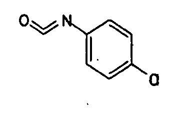 | 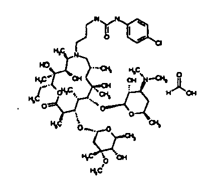 | 945,6 | 13 | 96,9 |
| **Example 78** | 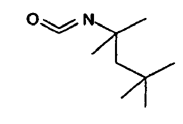 | 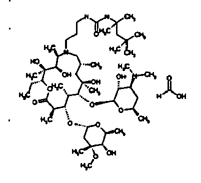 | 947,3 | 21 | 97,5 |
| **Comparative Example 79** | 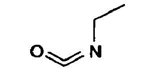 | 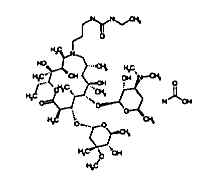 | 863,1 | 5,6 | 96,3 |
| **Comparative Example 80** | 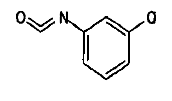 | 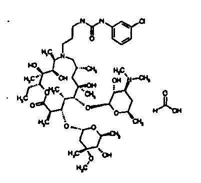 | 945,6 | 2,6 | 95,5 |
| **Comparative Example 81** | 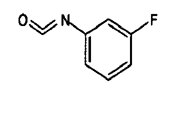 | 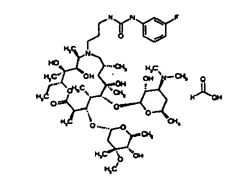 | 929,2 | 8,3 | 95,7 |
| **Example 82** | 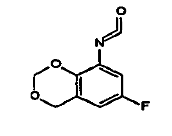 | 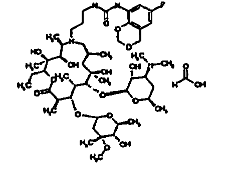 | 987,2 | 10,9 | 94,3 |
| **Example 83** | 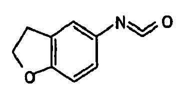 | 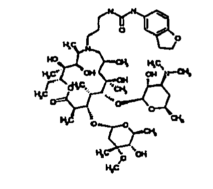 | 953,2 | 29,2 | 88,4 |
| **Example 84** | 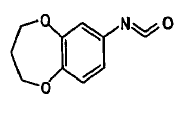 | 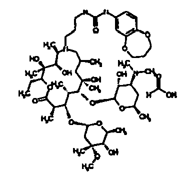 | 983,3 | 7,9 | 91,9 |
| **Example 85** | 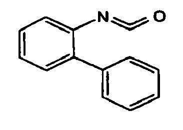 | 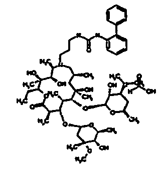 | 987,3 | 16,2 | 94,8 |
| **Example 86** | 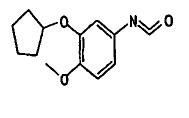 | 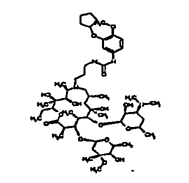 | 1025,3 | 31,6 | 91,4 |
| **Example 87** | 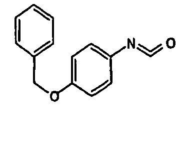 | 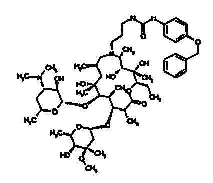 | 1017,3 | 31,2 | 92,1 |
| **Example 88** | 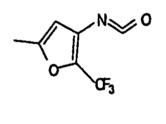 | 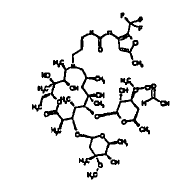 | 983,2 | 12,3 | 96,1 |
| **Example 89** | 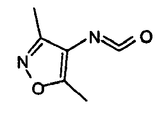 | 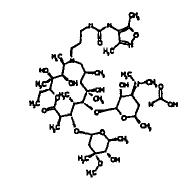 | 930,2 | 16,3 | 95 |
| **Example 90** | 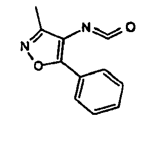 | 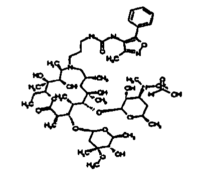 | 992,3 | 13,1 | 97,5 |
| **Example 91** | 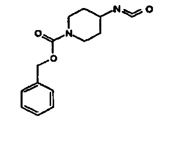 | 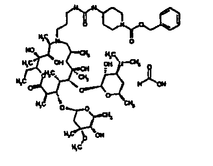 | 1052,4 | 16,6 | 99,4 |
| **Example 92** | 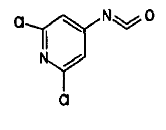 | 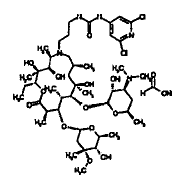 | 981,1 | 1,2 | 99,6 |
| **Example 93** | 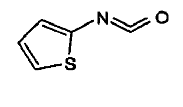 | 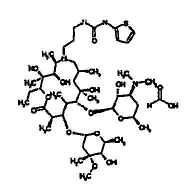 | 917,2 | 13,4 | 91,7 |
| **Example 94** | 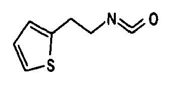 | 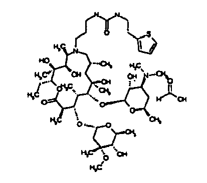 | 945,3 | 9,5 | 94,9 |
| **Example 95** | 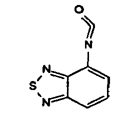 | 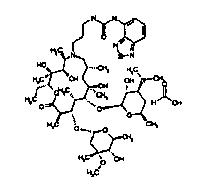 | 969,3 | 11,6 | 92,2 |
| **Comparative Example 96** | 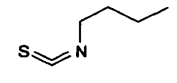 | 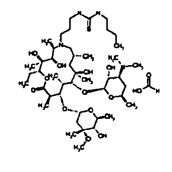 | 907,3 | 10,7 | 89,4 |
| **Comparative Example 97** | 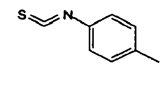 | 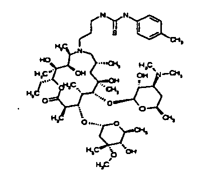 | 941,3 | 18,5 | 88,2 |
| **Example 98** | 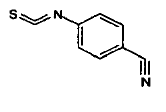 | 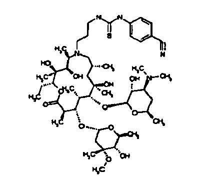 | 952,3 | 15,8 | 91,4 |
| **Comparative Example 99** | 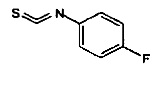 | 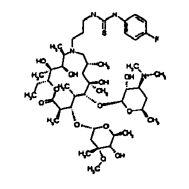 | 945,2 | 17,1 | 94,6 |
| **Example 100** | 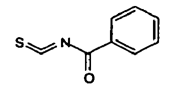 | 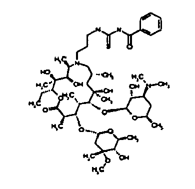 | 955,3 | 17,4 | 92,2 |
| **Comparative Example 101** | 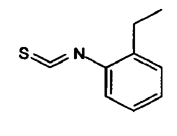 | 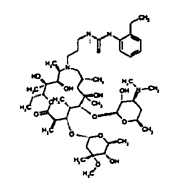 | 955,3 | 11,3 | 91,6 |
| **Comparative Example 102** | 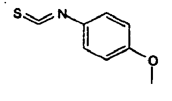 | 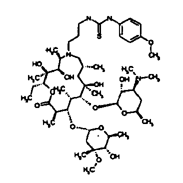 | 957,3 | 17,6 | 93,6 |
| **Comparative Example 103** | 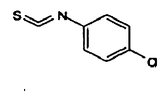 | 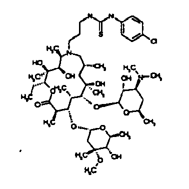 | 961,7 | 18,6 | 94,2 |
| **Comparative Example 104** | 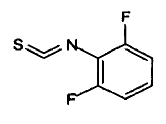 | 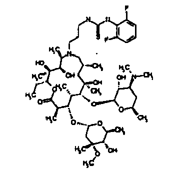 | 963,2 | 17,5 | 92,1 |
| **Example 105** | 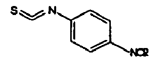 | 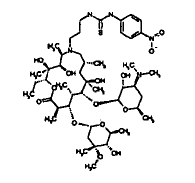 | 972,2 | 13,8 | 94,1 |
| **Example 106** | 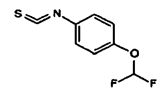 | 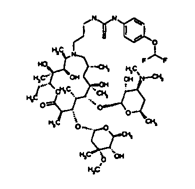 | 993,3 | 17,4 | 92,2 |
| **Comparative Example 107** | 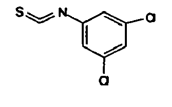 | 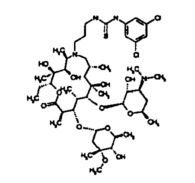 | 996,1 | 15,7 | 93,4 |
| **Example 108** | 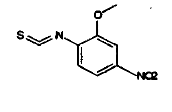 | 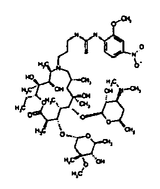 | 1002,3 | 18,8 | 96,2 |
| **Example 109** | 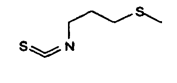 | 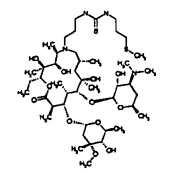 | 939,3 | 7 | 95,4 |
| **Example 110** | 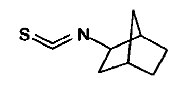 | 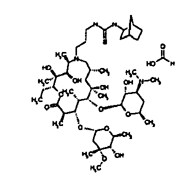 | 945,3 | 3 | 95,7 |
| **Example 111** | 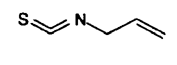 | 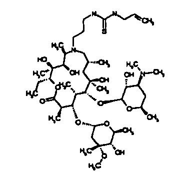 | 891,2 | 15,3 | 89,3 |
| **Example 112** | 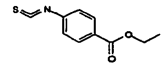 | 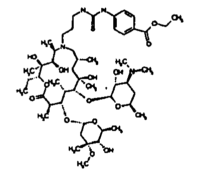 | 999,3 | 17,3 | 95,5 |
| **Example 113** | 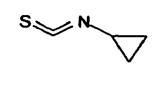 | 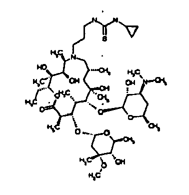 | 891,2 | 18,2 | 92,3 |
| **Comparative Example 114** | 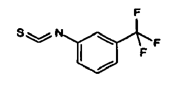 | 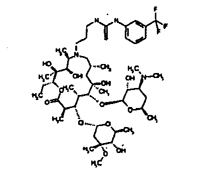 | 995,3 | 16,7 | 95,8 |
| **Example 115** | 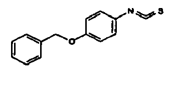 | 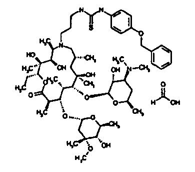 | 1033,4 | 6,6 | 97,5 |
| **Example 116** | 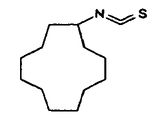 | 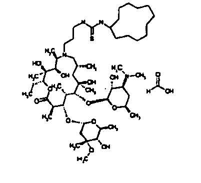 | 1017,5 | 23,3 | 91,4 |
| **Example 117** | 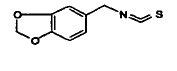 | 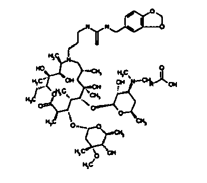 | 985,3 | 14,8 | 94,9 |
| **Example 118** | 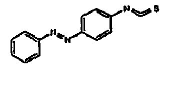 | 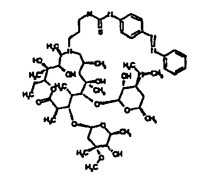 | 1031,4 | 24,5 | 90,5 |
| **Example 119** | 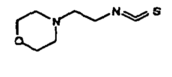 | 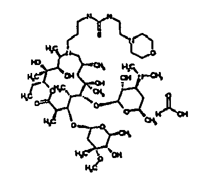 | 964,3 | 5 | 98,5 |
| **Example 120** | 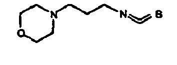 | 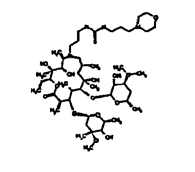 | 978,3 | 22,2 | 90,4 |
| **Example 121** | 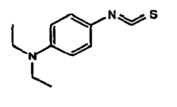 | 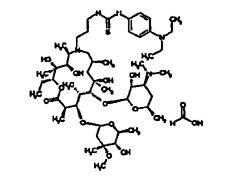 | 998,4 | 15,6 | 93,5 |
| **Example 122** | 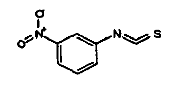 | 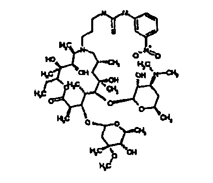 | 972,2 | 23,2 | 89,4 |
| **Example 123** | 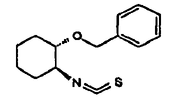 | 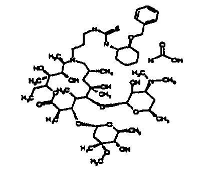 | 1039,4 | 13,3 | 94,4 |
| **Comparative Example 124** | 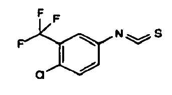 | 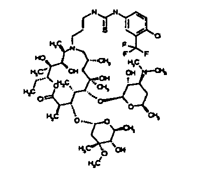 | 1029,7 | 24,4 | 90,2 |
| **Example 125** | 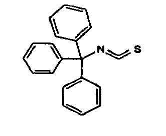 | 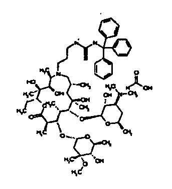 | 1093,5 | 2,8 | 98,9 |
| **Example 126** | 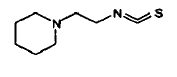 | 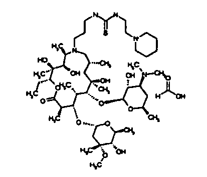 | 962,3 | 1,6 | 91,7 |
| **Example 127** | 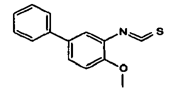 | 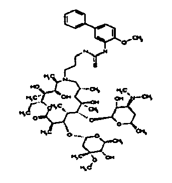 | 1033,4 | 21,5 | 92,8 |
| **Example 128** | 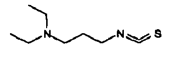 | 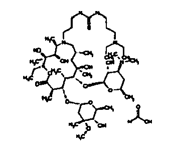 | 964,4 | 3,9 | 91,2 |
| **Example 129** | 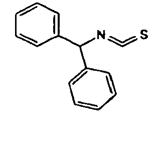 | 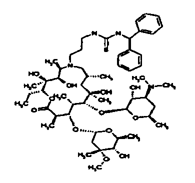 | 1017,4 | 26 | 92,1 |
| **Example 130** | 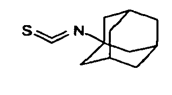 | 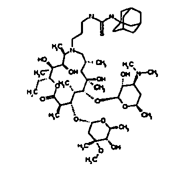 | 985,4 | 21,9 | 89,4 |
| **Comparative Example 131** | 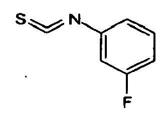 | 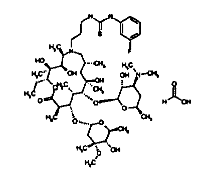 | 945,2 | 2,3 | 96,7 |
| **Comparative Example 132** | 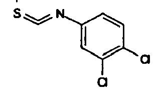 | 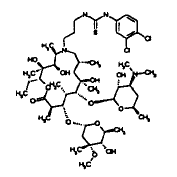 | 996,1 | 21,4 | 90,7 |
| **Comparative Example 133** | 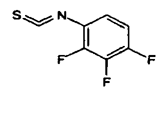 | 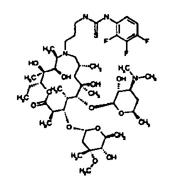 | 981,2 | 18,4 | 89,8 |
| **Example 134** | 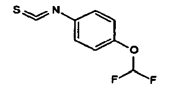 | 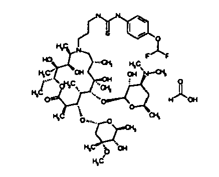 | 993,3 | 15 | 97,2 |
| **Example 135** | 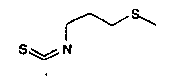 | 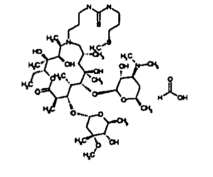 | 939,3 | 7,2 | 96,7 |
| **Comparative Example 136** | 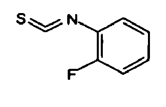 | 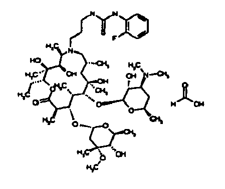 | 945,2 | 8,4 | 93,2 |
| **Comparative Example 137** | 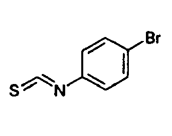 | 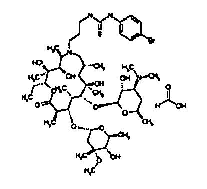 | 1006,2 | 19 | 92,2 |
| **Example 138** | 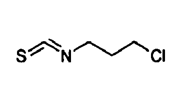 | 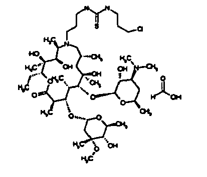 | 911,7 | 24,1 | 99,6 |
| **Example 139** | 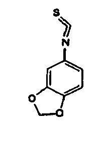 | 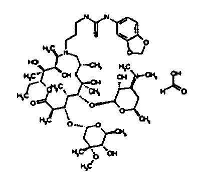 | 971,3 | 18,6 | 95,8 |
| **Example 140** | 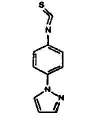 | 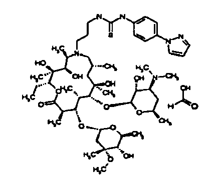 | 993,3 | 19,5 | 94,6 |
| **Example 141** | 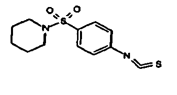 | 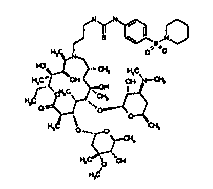 | 1074,5 | 38,2 | 91,8 |
| **Example 142** | 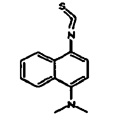 | 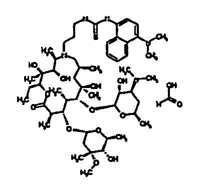 | 1020,4 | 15 | 89,1 |
| **Example 143** | 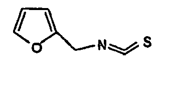 | 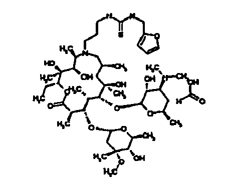 | 931,2 | 10,1 | 97,3 |
| **Example 144** | 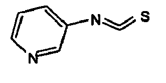 | 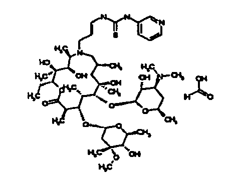 | 928,2 | 22,6 | 97,7 |
| **Example 145** | 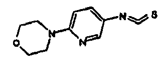 | 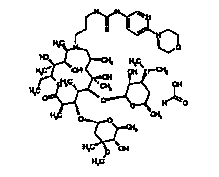 | 1013,4 | 18,1 | 99,6 |
| **Example 146** | 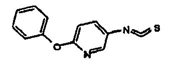 | 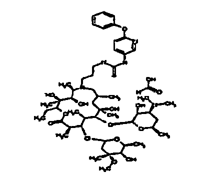 | 1020,3 | 17,9 | 97,1 |
| **Example 147** | 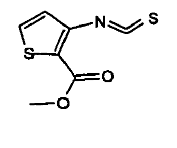 | 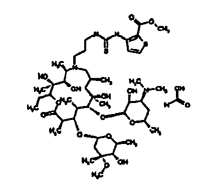 | 991,3 | 7,3 | 98,4 |
| **Example 148** | 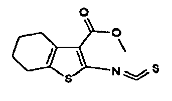 | 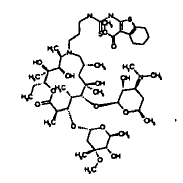 | 1045,4 | 39,5 | 89,7 |
| **Comparative Example 149** | 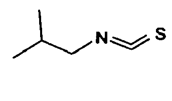 | 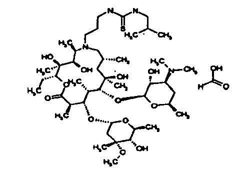 | 907,3 | 15,3 | 96,5 |
| **Example 150** | 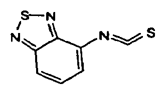 | 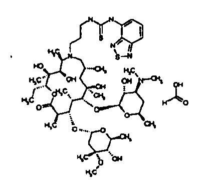 | 985,3 | 8,9 | 94,3 |
| **Example 151** | 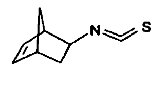 | 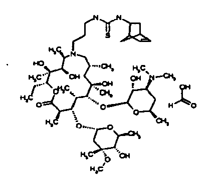 | 943,3 | 14,7 | 98,3 |

### Examples 152 to 153

### General procedure:

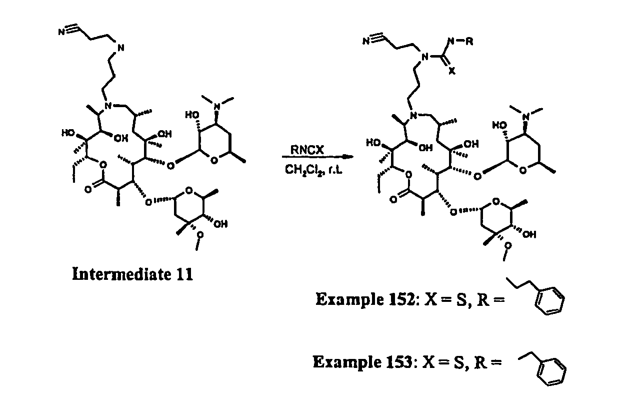

**Intermediate 11** (700 mg; 0,88 mmol) was dissolved in dry DCM (10 ml) under argon, and the 3-phenylpropylisothiocyanate **(Example 152)** or 2-phenylethylisothiocyanate **(Example 153)** (1,1 eq) was added. The reaction mixture was stirred at room temperature until conversion was completed (1-24 hours). The solvent was evaporated and the crude product purified on a silica gel column in the solvent system DCM : MeOH : NH₄OH = 90:9:1,5 to yield the desired product.

### Examples 154 to 155

### General procedure:

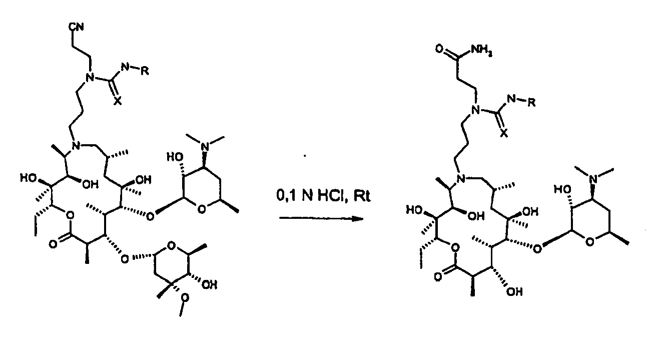

A solution of **Example 152/Example 153** (10 mg) in 0,1 N HCl (2 ml) was stirred for 24 hours. To the reaction mixture ethylacetate (5 ml) and water were added (5 ml) and the layers were separated (3 times.) The aqueous layer was adjusted to pH 9.5 with 0.1 N NaOH. and extracted with ethylacetate (4x 10 ml). These organic layers were washed with brine (2 x 20 ml), dried over K₂CO₃ and evaporated under reduced to yield the desired products.

**Table 2**

| **Example** | **Product** | **MS (ES, m/z)** | **mass/mg** | **purity %** |
|---|---|---|---|---|
| **Example 154** | 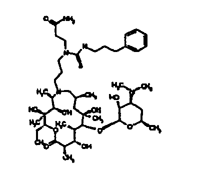 | 883.3 | 8.1 | 97.1 |
| **Example 155** | 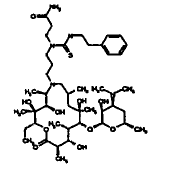 | 868.1 | 7.8 | 96.0 |

### Examples 156 to 158

### General procedure:

To the DCM solution (5 ml) of **Intermediate 3** (1 mmol), the appropriate isocyanate or isothiocyanate (1.1 mmol) was added at 0°C to room temperature and the reaction mixture stirred from 15 minutes to 48 hours. Product was isolated by extraction with DCM and purified by precipitation from EtOAc/n-hexane or by column chromatography using eluation sistem DCM/MeOH/NH₄OH = 90:9:0.5.

### Example 156

### 9-Deoxo-9-dihydro-9a-[N'-(phenyl)thiocarbamoyl-β-aminoethyl]-9a-aza-9a-homoerythromycin A

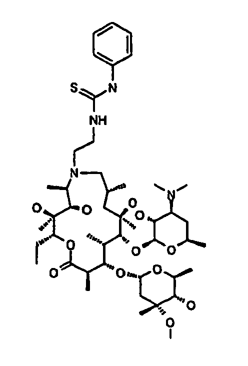

**¹³C NMR (125 MHz, pyridule)**/δ: 178.2, 46.2, 80.5, 41.9, 84.5, 75.8, 41.3, 30.1, 66.0, 61.9, 77.0, 75.7, 79.1, 22.8, 12.0, 103.8, 71.9, 66.4, 40.9, 31.2, 68.5, 97.0, 36.2, 74.1, 50.2, 79.2, 66.7, 16.4, 10.9, 28.3, 22.9, 10.4, 19.0, 22.3, 19.9, 22.1, 51.5, 44.1, 182.6, 140.8, 129.6, 125.3, 124.9.
**MS m/z: (ES): MH⁺**= 913.23.

### Example 157

### 9-Deoxo-9-dihydro-9a-[N'-(phenyl)carbamoyl-β-aminoethyl]-9a-aza-9a-homoerythromycin A

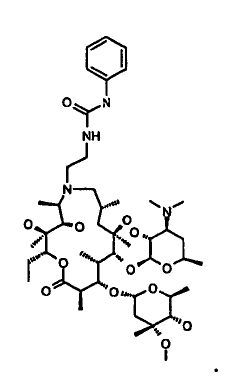

**¹³C NMR (125 MHz, pyridine)**/δ: 178.1, 46.3, 81.1, 41.1, 84.3, 76.0, 40.3, 30.9, 67.0, 62.0, 77.6, 75.7, 79.5, 22.6, 11.7, 104.0, 72.2, 66.3, 40.9, 30.7, 68.7, 97.4, 36.3, 74.1, 50.2, 79.2, 66.8, 16.8, 11.1, 28.0, 22.5, 9.7, 18.8, 22,4, 20.0, 22.0, 53.1; 44.0, 156.7, 142.3, 129.3, 121.9, 119.3.
**MS m/z: (ES): MH⁺**= 897.17.

### Example 158

### 9-Deoxo-9-dihydro-9a-[N'-(ethyl)carbamoyl-β-ammo-ethyl]-9a-aza-9a-homoerythromycin A

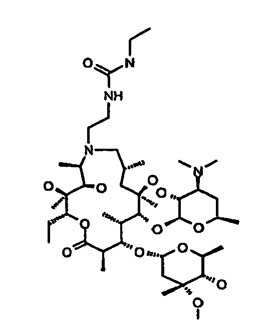

**¹³C NMR (125 MHz, pyridine)**/δ: 178.0, 46.2, 80.8, 41.4, 84.4, 75.7, 40.9, 30.4, 66.0, 62.1, 76.9, 75.7, 79.9, 22.6, 11.9, 103.8, 72.0, 66.4, 40.9, 31.2, 68.5, 97.2, 36.2, 74.1, 50.2, 79.2, 66.7, 16.6, 11.0, 28.2, 22.8, 9.7, 18.7, 22.3, 19.9, 22.0, 53.4; 40.1, 159.9, 35.9, 16.4.
**MS m/z: (ES): MH⁺**= 849.12.

### Example 159

### 9-Deoxo-9-dihydro-9a-[N'-(phenylethyl)thiocarbamoyl-β-antinoethyl]-9a-aza-9a-homoerythromycin A

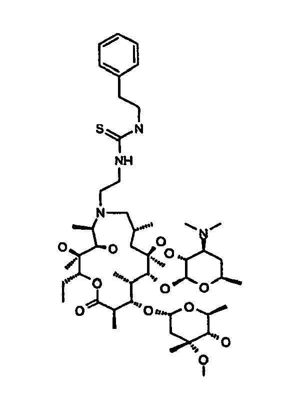

To a solution of **Intermediate 3** (0.2 g, 0.257 mmol) in DCM (10 ml) 2-phenylethyl-isothiocyanate (0.045 ml, 0.302 mmol) was added. The reaction mixture was stirred 24 hours at room temperature. To the reaction mixture, water was added (20 ml). The layers were separated and the water layer was extracted with DCM (2 x 10 ml). Combined organic layers were washed with brine (2 x 10 ml), dried over K₂CO₃ and evaporated under reduced pressure. The crude product was purified by precipitation from EtOAc/n-hexane yielding the title compound (0.134 g).
**¹³C NMR (75 MHz, DMSO)**/δ: 176.25, 139.29, 128.54, 128.22, 125.98, 101.89, 94.91, 82.58, 77.87, 77.27, 76.48, 75.29, 74.16, 73.51, 72.64, 70.52, 67.00, 64.82, 64.53, 63.00, 59.47, 49.87, 48.71, 45.00, 44.12, 41.74, 40.35, 40.26, 40.22, 34.73, 29.91, 27.98, 26.93, 22.17, 21.33, 21.24, 20.89, 18.39, 18.12, 14.88, 10.90, 9.53, 9.37.
**MS m/z: (ES): MH⁺**= 941.4.

### Examples 160 to 165

### General procedure

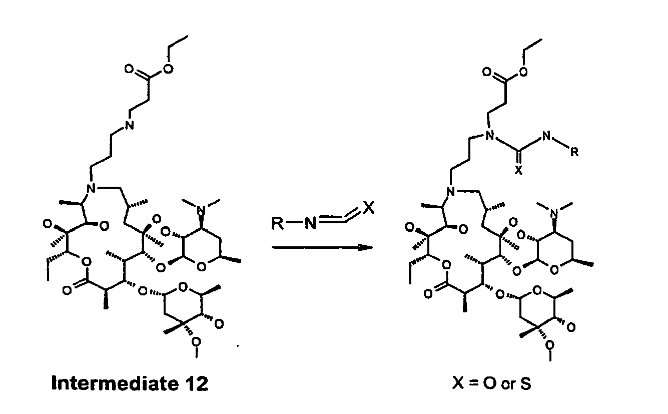

A mixture of **Intermediate 12** (1.12 mmol) and the appropriate isocyanate/isothiocyanate (1.12 mol) in dry DCM (15 ml) was stirred for 1-4 hours at room temperature and than evaporated to dryness. The crude product was purified by column chromatography using eluation sistem DCM/MeOH/N₄OH = 90:9:0.5.

### Example 160

### 9-Deoxo-9-dihydro-9a-{N'-[β-(ethoxycarbonyl)ethyl]-N'-[β-(phenylethyl)thiocarbamoyl]-γ-aminopropyl}-9a-aza-9a-homoerythromycin A

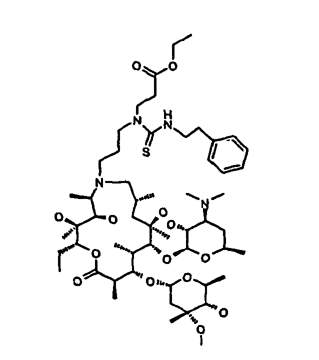

According to the general procedure starting from **Intermediate 12** and 2-phenylethylisothiocynate the title compound was obtained (22 mg).
**¹³C NMR (75 MHz, DMSO)**/δ: 180.1, 176.6, 171.3, 139.6, 128.5, 128.5, 128:3, 128.3, 126.0, 102.0, 95.0, 82.6, 77.9, 77.4, 76.5, 75.0, 74.4, 73.5, 72.7, 70.7, 67.2, 64.9, 64.7, 62.3, 60.0, 59.5, 48.8, 48.4, 47.6, 46.7, 46.5, 44.1, 40.6, 40.6, 40.3, 34.9, 34.7, 31.8, 29.9, 27.6, 27.1, 24.6, 22.4, 21.3, 21.3, 20.9, 18.4, 18.3, 14.7, 14.0, 10.9, 9.5,9.4.

### Example 161

### 9-Deoxo-9-dihydro-9a-{N'-[β-(ethoxycarbonyl)ethyl]-N'-[γ-(phenylpropyl)thiocarbamoyl]-γ-aminopropyl}-9a-aza-9a-homoerythromycin A

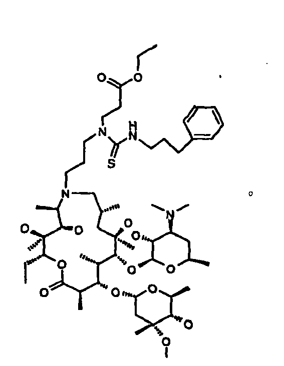

According to the general procedure starting from Intermediate 12 and phenylpropylisothiocynate the title compound was obtained (379 mg).
**¹³C NMR (75 MHz, DMSO)**/δ: 180.5, 176.6, 171.6, 141.9, 128.2, 128.2, 128.2, 128.2, 125.6, 102.0, 95.0, 82.7, 77.8, 77.4, 76.6, 75.0, 74.6, 73.6, 72.8, 70.8, 67.2, 65.0, 64.6, 62.4, 60.0, 59.4, 48.7, 48.2, 47.6, 46.6, 44.9, 44.1, 40.7, 40.7, 40.4, 34.8, 32.8, 32.0, 30.5, 30.0, 27.7, 27.7, 24.7, 22.4, 21.5, 21.4, 21.0, 18.5, 18.3, 14.8, 14.2, 11.0, 9.5, 9.3.

### Example 162

### 9-Deoxo-9-dihydro-9a-{N'-[β-(ethoxycarbonyl)ethyl]-N'-bemylthiocarbamoyl-γ-aminopropyl]-9a-aza-9a-homoerythromycin A

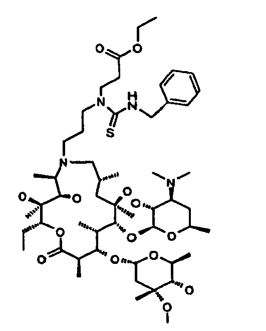

According to the general procedure starting from **Intermediate 12** and benzylisothiocyanate the title compound was obtained (22 mg).
**¹³C NMR (75 MHz, DMSO)**/δ: 180.7, 176.3, 171.2, 139.9, 128.0, 128.0, 126.8, 126.8, 126.3, 120.1, 94.9, 82.7, 77.9, 77.4, 76.6, 75.0, 74.3, 73.5, 72.7, 70.7, 67.2, 65.0, 64.7, 62.5, 60.0, 59.6, 48.8, 48.4, 48.3, 47.8, 46.8, 40.6, 40.6, 40.2, 39.8, 34.8, 32.0, 30.0, 27.0, 27.8, 24.8, 22.4, 21.4, 21.3, 20.9, 18.4, 18.3, 14.7, 14.0, 10.9, 9.5, 9.3.

### Example 163

### 9-Deoxo-9-dihydro-9a-{N'-[β-(ethoxycarbonyl)ethyl]-N'-[1-(1-naphtylethyl]carbamoyl-γ-aminopropyl}-9a-aza-9a-homoerythromycin A

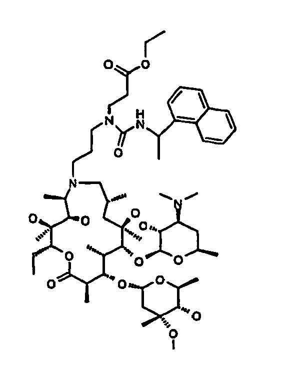

According to the general procedure starting from **Intermediate 12** and 1-naphtylethylisocynate the title compound was obtained (68 mg).
**MS m/z: (ES): MH⁺**=1089.8

### Example 164

### 9-Deoxo-9-dihydro-9a-{N'-[β-(ethoxycarbonyl)ethyl]-N'-(2,4-dichlorophenyl)carbamoyl-γ-aminopropyl}-9a-aza-9a-homoerythromycin A

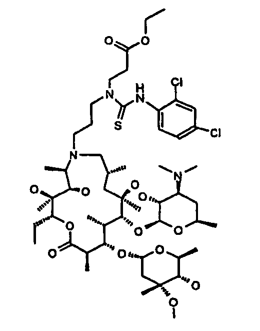

According to the general procedure starting from **Intermediate 12** and 2,4-dichlorophenylisocyanate the title compound was obtained.
**MS m/z: (ES): MH⁺**= 1112.5

### Example 165

### 9-Deoxo-9-dihydro-9a-{N'-[β-(ethoxycarbonyl)ethyl]-N'-(2-naphtyl)carbamoyl-γ-aminopropyl}-9a-aza-9a-homoerythromycin A

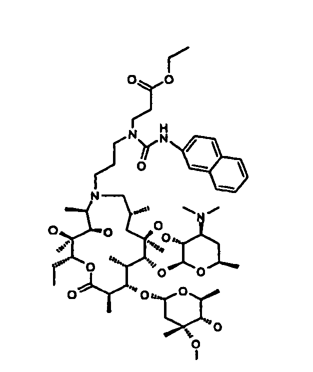

According to the general procedure starting from **Intermediate 12** and 2-naphtylisocyanate the title compound was obtained.
**MS m/z: (ES): MH⁺**= 1065.6.

### Examples 166 to 170

### General procedure

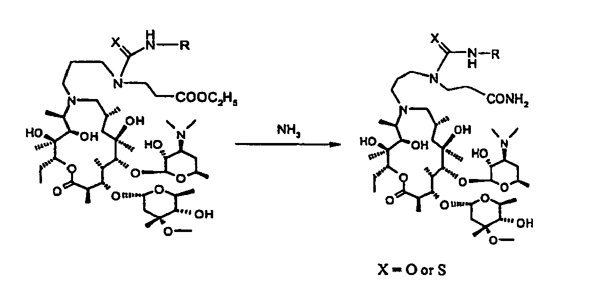

To a solution of the corresponding 9a-{N'-[β-(ethoxycarbonyl)ethyl]-N'-substituted carbamoyl- or thiocarmamoyl-γ-aminopropyl}-9-deoxo-9-dihydro-9a-aza-9a-homoerythromycine A compounds in ethanol (4 ml), a 25 % ammonia solution (4 ml) was added. The reaction mixture was left at room temperature until complete conversion and then evaporated to dryness. Obtained crude product was purified by column chromatography in the solvent system DCM:MeOH:NH₄OH = 90:9:1.5 to yield the corresponding 9a-[N'-β-(amido)ethyl] compounds.

### Example 166

### 9-Deoxo-9-dihydro-9a-{N'-(β-amidoethyl)-N'-[β-(phenylethyl)thiocarbamoyl]-γ-ammopropyl}-9a-aza-9a-homoerythromycin A

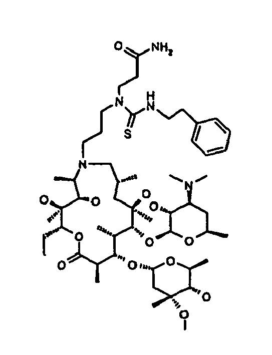

According to the general procedure starting from **Example 160** the title compound (94 mg) was obtained.
**MS m/z: (ES): MH⁺**= 1026.4.

### Example 167

### 9-Deoxo-9-dihydro-9a-{N'-(β-amidoethyl)-N'-[γ-(phenylpropyl)thiocarbamoyl]-γ-aminopropyl}-9a-aza-9a-homoerythromycin A

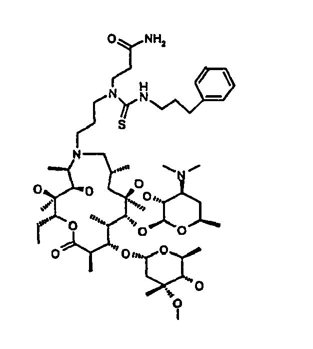

According to the general procedure starting from Example 161 the title compound (122 mg) was obtained.
**MS m/z: (ES): MH⁺**= 1041.4.

### Example 168

### 9-Deoxo-9-dihydro-9a-{N'-(β-amidoethyl)-N'-benzylthiocarbamoyl-γ-aminopropyl}-9a-aza-9a-homoerythromycin A

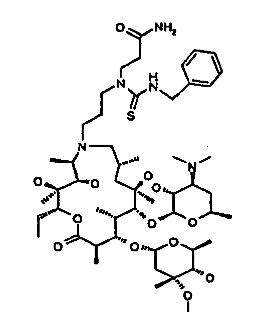

According to the general procedure starting from **Example 162** the title compound (102 mg) was obtained.
**MS m/z: (ES): MH⁺=** 1012.6.

### Example 169

### 9-Deoxo-9-dihydro-9a-[N'-(β-amidoethyl)-N'-(2,4-dichlorophenyl)carbamoyl-γ-aminopropyl]-9a-aza-9a-homoerythromycin A

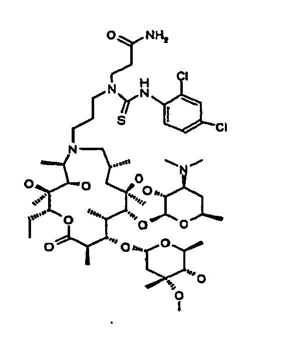

According to the general procedure starting from **Example 164** the title compound (112 mg) was obtained.
**MS m/z: (ES): MH⁺**= 1050.6.

### Example 170

### 9-Deoxo-9-dihydro-9a-{N'-(β-anfdoethyl)-N'-(2-naphtylcarbamoyl)-γ-aminopropyl}-9a-aza-9a-homoerythromycin A

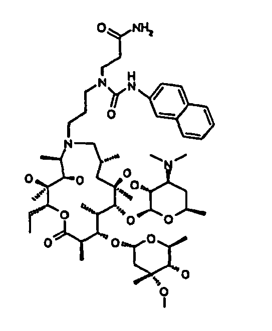

According to the general procedure starting from **Example 165** the title compound (90 mg) was obtained.
**MS m/z: (ES): MH**⁺= 1032.3.

### Examples 171 to 176

### General procedure

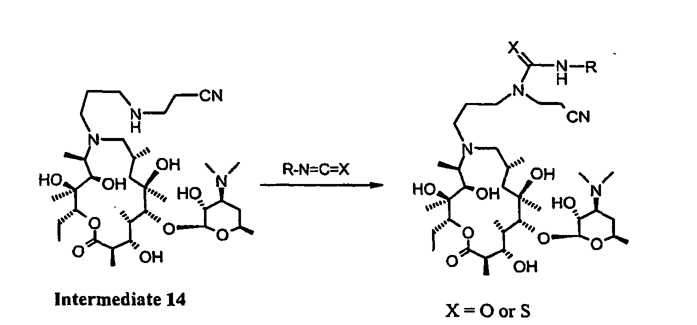

A mixture of **Intermediate 14** (1.45 mmol) and the corresponding isocyanate or isothiocyanate (1.45 mmol) in dry DCM (15 ml) was stirred from 1 to 4 hours at room temperature and then evaporated to dryness. Obtained crude product was purified by column chromatography in the solvent system DCM:MeOH = 90:1 to yield the corresponding 3-*O*-Decladinosyl-9a-[N'-(β-cyanoethyl)-N'-substituted carbamoyl- or thiocarmamoyl-γ-aminopropyl]-9-deoxo-9-dihydro-9a-aza-9a-homoerythromycine A compound.

### Example 171

### 3-O-Decladinosyl-9-deoxo-9-dihydro-9a-{N'-(β-cyanoethyl)-N'-[β-(phenylethyl)thiocarbaruoyl]-γ-aminopropyl}-9a-aza-9a-homoerythromycin A

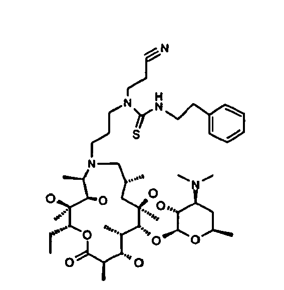

According to the general procedure starting from **Intermediate 14** and ethylphenylisothiocyanate the title compound was obtained (133.5 mg).
**MS m/z: (ES): MH⁺**= 850.5.

### Comparative Example 172

### 3-O-Decladinosyl-9-deoxo-9-dihydro-9a-{N'-(β-cyanoethyl)-N'-[3-(phenylpropyl)thiocarbamoyl]-γ-aminopropyl}-9a-aza-9a-homoerythromycin A

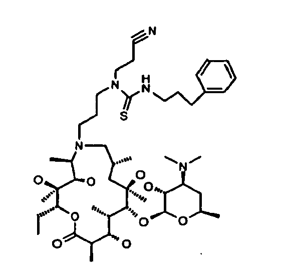

According to the general procedure starting from **Intermediate 14** and propylphenylisothiocyanate the title compound was obtained (227.6 mg).
**MS m/z: (ES): MH⁺**= 864.5.

### Comparative Example 173

### 3-O-Decladinosyl-9-deoxo-9-dihydro-9a-[N'-(β-cyanoethyl)-N'-benzylthiocarbamoyl-γ-aminopropyl]-9a-aza-9a-homoerythromycin A

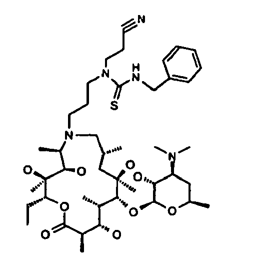

According to the general procedure starting from **Intermediate 14** and benzylisothiocyanate the title compound was obtained (177 mg).
**MS m/z: (ES): MH⁺**= 836.5.

### Comparative Example 174

### 3-O-Decladinosyl-9-deoxo-9-dihydro-9a-{N'-(β-cyanoethyl)-N'-[(2,4-dichlorophenyl)carbamoyl]-γ-aminopropyl}-9a-aza-9a-homoerythromycin A

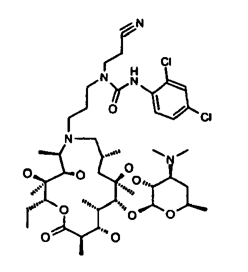

According to the general procedure starting from **Intermediate 14** and 2,4-dichlorophenylisocynate the title compound was obtained (140 mg).
**MS m/z: (ES): MH⁺**= 874.4.

### Comparative Example 175

### 3-O-Decladinosyl-9-deoxo-9-dihydro-9a-{N'-(β-cyanoethyl)-N'-(2-naphtyl)carbamoyl]-γ-aminopropyl}-9a-aza-9a-homoerythromycin A

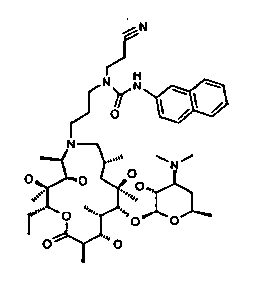

According to the general procedure starting from **Intermediate 14** and 2-naphtylisocyanate the title compound was obtained (111 mg).
**MS m/z: (ES): MH⁺**= 856.4.

### Comparative Example 176

### 3-O-Decladinosyl-9-deoxo-9-dihydro-9a-{N'-(β-cyanoethyl)-N'-[1-(1-naphtylethyl)carbamoyl]-γ-aminopropyl}-9a-aza-9a-homoerythromycin A

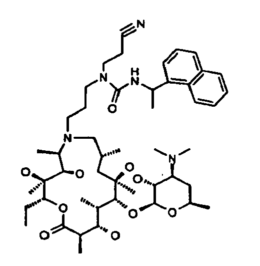

According to the general procedure starting from **Intermediate 14** and 1-(1-naphtyl)ethylisocynate the title compound was obtained (148 mg).
**MS m/z: (ES): MH⁺**= 884.5.

### Examples 177 to 201

### General procedure

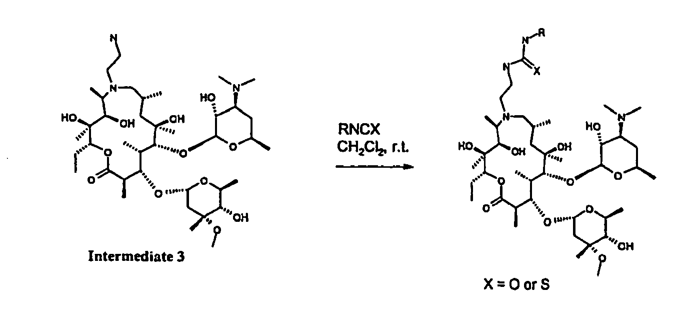

To a solution of **Intermediate 3** (30 mg, 0.04 mmol) in DCM (1 ml) was added 1.3 equivalents of the iso-/isothiocyanate reagent. The mixture was stirred from 1 to 48 hours, and a scavenger resin (trisamine polymer bound, 3 eq.) was added. After 1 day the resin was filtered off and washed with DCM (1 ml). The solvent was evaporated giving the desired product. The products are purified, if necessary, using preparative LC/MS.
The table that follows gives the structures of iso-/isothiocynate reagent, as well as the products of formula (I).

| **Example** | **RNCX** | **Product** | **MS (ES, m/z)** | **purity %** |
|---|---|---|---|---|
| Example 177 | 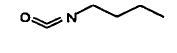 | 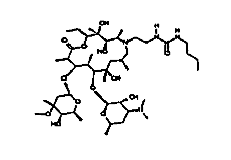 | 877.5 | 91.2 |
| Example 178 | 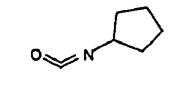 | 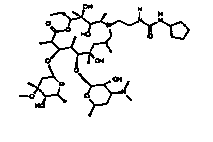 | 889,5 | 95.1 |
| Example 179 | 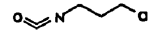 | 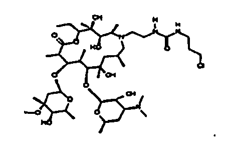 | 897.4 | 82.1 |
| Example 180 | 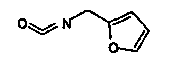 | 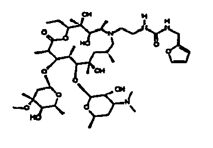 | 901.4 | 59.6 |
| Example 181 | 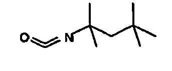 | 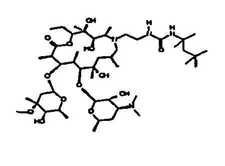 | 933.5 | 99.1 |
| Example 182 | 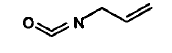 | 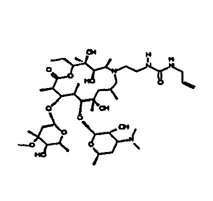 | 861.4 | 70.2 |
| Example 183 | 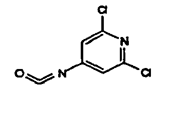 | 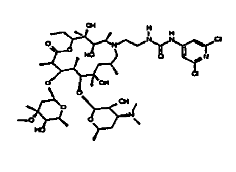 | 967.3 | 49.2 |
| Example 184 | 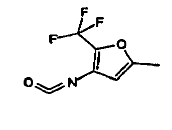 | 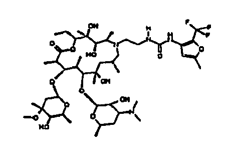 | 970.4 | 90.7 |
| Example 185 | 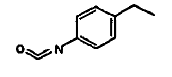 | 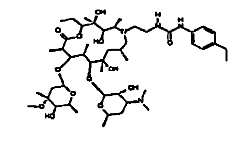 | 925.5 | 97.2 |
| Example 186 | 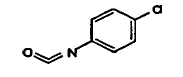 | 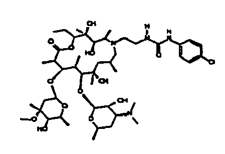 | 931.4 | 93.1 |
| Example 187 | 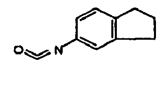 | 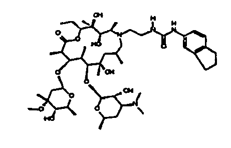 | 937.5 | 95.3 |
| Example 188 | 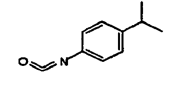 | 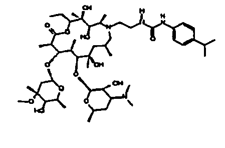 | 939.6 | 95 |
| Example 189 | 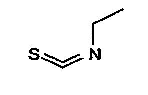 | 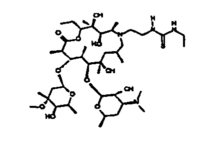 | 865.4 | 90.2 |
| Example 190 | 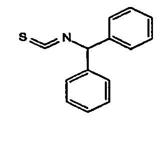 | 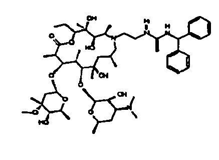 | 1003.6 | 91.5 |
| Example 191 | 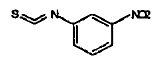 | 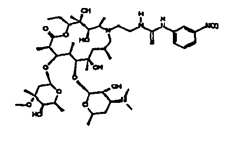 | 958.4 | 96 |
| Example 192 | 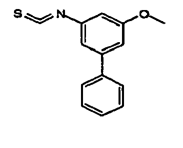 | 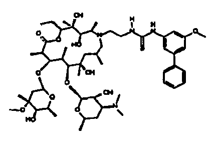 | 1019.4 | 91.6 |
| Example 193 | 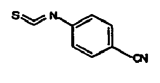 | 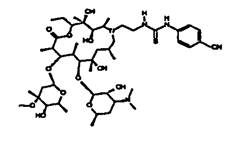 | 938.5 | 97.5 |
| Example 194 | 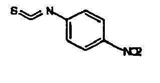 | 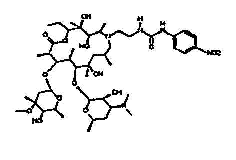 | 958.3 | 96.5 |
| Example 195 | 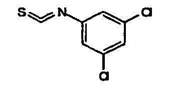 | 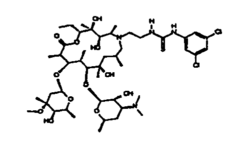 | 981.4 | 98.1 |
| Example 196 | 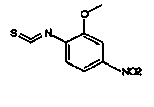 | 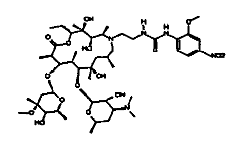 | 988.4 | 94.8 |
| Example 197 | 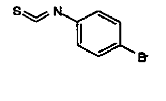 | 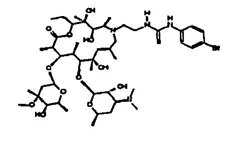 | 992.6 | 96.7 |
| Example 198 | 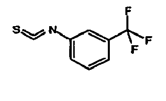 | 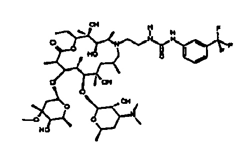 | 981.6 | 96.1 |
| Example 199 | 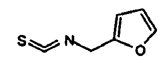 | 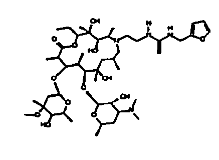 | 917.4 | 94.7 |
| Example 200 | 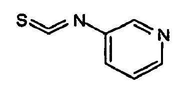 | 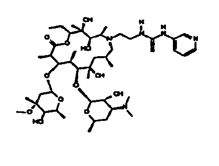 | 914.5 | 97.4 |
| Example 201 | 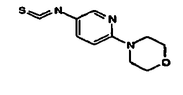 | 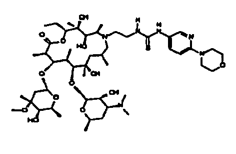 | 999.6 | 97.2 |

### Example 202

### 9-Deoxo-9-dihydro-9a-{N'-[β-(ethoxycarbonyl)ethyl]-N'-(2-naphtyl)carbamoyl-β-aminoethyl}-9a-aza-9a-homoerythromycin A

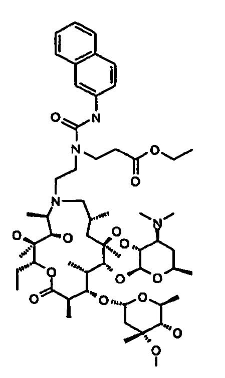

According to procedure for **Example 159** starting from **Intermediate 6** and 2-naphtyl-isocyanate the title compound was obtained.
**MS m/z: (ES): MH⁺**= 1047.7.

### Example 203

### 9-Deoxo-9-dihydro-9a-{N'-(β-amidoethyl)-N'-(2-naphtyl)carbamoyl-β-aminoethyl}-9a-aza-9a-homoerythromycin A

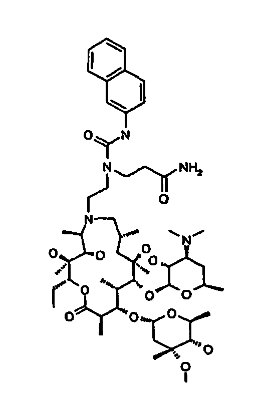

According to procedure for **Example 159** starting from **Intermediate 7** and 2-naphtyl-isocyanate the title compound was obtained.
**MS m/z: (ES): MH⁺**= 1018.6.

### Example 204

### In Vitro Assay

The *in vitro* potency of the compounds has been compared with that of azithromycin to better gauge compounds suitable for further testing. As described above, parasites were exposed for 72 hours.. Data from the *in vitro* potency tests of azithromycin against the three parasite strains have been averaged, and these numbers have been used for identification of "active" macrolide compounds. These average IC_{50S} against the TM91C235, D6, and W2 strains were 1621.2 ng/mL, 96.9 ng/mL, and 1759.2 ng/mL, respectively.

Preliminary testing of the compounds of present invention showed that they exhibit antimalarial activity comparable to azithromycin or better.

Table 3: IC₅₀ Values of macrolide compounds, in comparison with azithromycin, tested against the three parasite strains (TM91C235, D6, and W2) with different patterns of resistance.

| Compound | IC₅₀ (ng/mL) | | |
|---|---|---|---|
| | TM91C235 | D6 | W2 |
| azithromycin | 1621.2 | 796.9 | 1759.2 |
| Example 1 | 619.1 | NA | 1777.0 |
| Example 2 | 39.9 | 134.4 | 74.4 |
| Example 3 | 154.7 | 292.0 | 261.7 |
| Example 12 | 28.9 | 71.3 | 43.2 |
| Example 53 | 712.7 | NA | 365.8 |
| Example 55 | 1047.6 | NA | 435.4 |
| Example 56 | 556.0 | NA | 250.5 |
| Example 57 | 453.5 | NA | 262.7 |
| Example 58 | 377.0 | NA | 169.4 |
| Example 59 | 193.0 | 361.6 | 92.0 |
| Example 60 | 93.3 | 212.4 | 69.3 |
| Example 61 | 435.9 | NA | 252.5 |
| Example 62 | 110.3 | 373.5 | 50.2 |
| Example 63 | 542.5 | NA | 141.3 |
| Example 64 | 91.5 | 252.6 | 70.0 |
| Example 65 | 397.7 | NA | 708.4 |
| Example 66 | 74.6 | 214.1 | 67.6 |
| Example 67 | 462.2 | NA | 194.7 |
| Example 68 | 253.5 | 797.8 | 105.9 |
| Example 70 | 86.3 | 163.9 | 76.5 |
| Example 71 | 131.2 | 318.2 | ND |
| Example 72 | 422.0 | 665.3 | ND |
| Example 73 | 128.4 | 305.4 | ND |
| Example 74 | 132.6 | 567.6 | ND |
| Example 75 | 228.9 | 478.6 | ND |
| Example 76 | 126.4 | 297.6 | ND |
| Example 77 | 89.6 | 186.5 | ND |
| Example 78 | 181.8 | 266.8 | ND |
| Example 80 | 116.7 | 451.1 | 185.6 |
| Example 81 | 159.7 | 565.3 | 277.9 |
| Example 82 | 227.1 | 293.3 | 316.3 |
| Example 84 | 61.8 | 28.9 | 42.0 |
| Example 85 | 141.7 | 262.0 | 208.8 |
| Example 86 | 141.6 | 456.5 | 206.5 |
| Example 87 | 148.5 | 303.8 | 178.0 |
| Example 88 | 329.8 | 371.1 | 318.5 |
| Example 90 | 510.5 | 972.1 | 762.4 |
| Example 91 | 259.4 | 697.6 | 303.4 |
| Example 96 | 363.7 | 624.6 | 179.0 |
| Example 97 | 154.5 | 310.6 | 67.1 |
| Example 98 | 74.5 | 125.9 | 47.6 |
| Example 99 | 131.0 | 302.3 | 68.8 |
| Example 100 | 275.3 | 284.2 | 102.2 |
| Example 101 | 240.5 | 425.0 | 91.9 |
| Example 102 | 200.5 | 459.9 | 73.7 |
| Example 103 | 103.4 | 180.5 | 51.4 |
| Example 104 | 181.5 | 490.0 | 83.1 |
| Example 105 | 23.7 | 18.4 | 20.1 |
| Example 106 | 120.3 | 214.9 | 62.6 |
| Example 109 | 332.3 | 843.3 | 190.0 |
| Example 110 | 111.0 | 246.7 | 46.1 |
| Example 111 | 458.5 | NA | 246.1 |
| Example 112 | 106.6 | 121.2 | 70.9 |
| Example 113 | 865.9 | NA | 605.2 |
| Example 115 | 172.2 | 269.1 | ND |
| Example 116 | 149.0 | 281.3 | ND |
| Example 117 | 223.3 | 329.0 | ND |
| Example 118 | 99.3 | 110.8 | ND |
| Example 121 | 311.6 | 799.3 | 714.3 |
| Example 123 | 137.3 | 699.9 | 297.2 |
| Example 124 | 12.2 | 55.9 | 57.5 |
| Example 125 | 36.4 | 142.2 | 42.2 |
| Example 127 | 57.1 | 60.0 | 117.3 |
| Example 129 | 56.0 | 222.1 | ND |
| Example 130 | 150.0 | 544.7 | 206.9 |
| Example 131 | 114.6 | 506.7 | 198.1 |
| Example 132 | 83.9 | 295.1 | 228.5 |
| Example 133 | 72.3 | 440.8 | 161.6 |
| Example 134 | 99.7 | 398.4 | 279.1 |
| Example 136 | 121.4 | 978.1 | 396.1 |
| Example 137 | 182.3 | 274.4 | 177.7 |
| Example 140 | 105.2 | 166.3 | 113.7 |
| Example 141 | 73.1 | 187.5 | 57.0 |
| Example 142 | 191.8 | 347.6 | 186.5 |
| Example 143 | 234.4 | 321.9 | 438.7 |
| Example 146 | 141.5 | 229.6 | 116.5 |
| Example 147 | 342.7 | 469.4 | 388.7 |
| Example 148 | 425.6 | 862.2 | 239.7 |
| Example 149 | 414.3 | 656.7 | 472.0 |
| Example 150 | 104.6 | 181.4 | 91.0 |
| Example 151 | 180.9 | 298.9 | 170.8 |
| Example 154 | 325.9 | NA | 395.9 |
| Example 158 | 102.8 | 167.4 | 404.6 |
| Example 160 | 68.3 | 153.4 | ND |
| Example 161 | 54.0 | 141.6 | ND |
| Example 162 | 69.3 | 104.2 | ND |
| Example 163 | 67.9 | 134.7 | ND |
| Example 166 | 160.8 | 458.2 | 229.8 |
| Example 167 | 57.2 | 126.9 | ND |
| Example 168 | 310.3 | 510.8 | 357.0 |
| Example 169 | 147.5 | 226.6 | 188.0 |
| Example 170 | 128.0 | 543.3 | ND |

| | | | |
|---|---|---|---|
| NA: less active than azithromycin; ND: not determined | | | |

## Claims

1. A compound of formula (I), 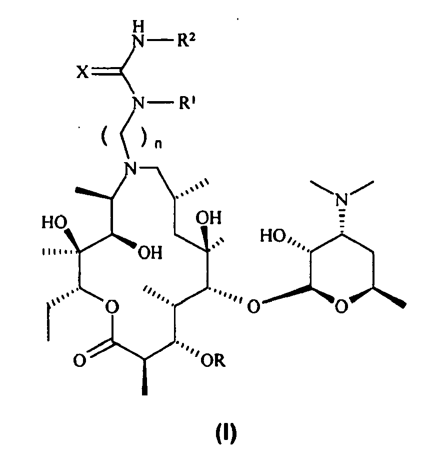 wherein
R represents H or cladinosyl group of formula (II); 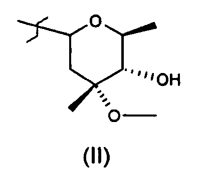
R¹ represents H, β-cyanoethyl, β-amidoethyl or β-(C₁₋₄alkoxycarbonyl)ethyl;
R² represents
a) C₁₋₁₂ alkyl, wherein C₁₋₁₂ alkyl is
i) uninterrupted or interrupted by 1-3 bivalent radical groups selected from -O-, -S- and -N(R³)- ; and/or
ii) linear or branched, and unsubstituted or substituted by 1-3 groups selected from halogen, OH; NH₂; N-(C₁-C₄)alkylamino; N,N-di(C₁-C₄-alkyl)amino, CN, NO₂; C(O)OC₁₋₄alkylaryl, (C₁-C₄-alkyl)-thio; a C₃₋₁₄ membered carbocycle optionally substituted with one or more substituents selected from halogen, CN, C₁₋₄alkyl unsubstituted or substituted with 1 to 3 halogen, O(C₁₋₄alkyl) optionally substituted with 1 to 3 halogen, S(C₁-C₄-alkyl), O(C₃-₆ cycloalkyl), O(C₁₋₄alkylaryl), C₁₋₄alkylcyano, C(O)C₁₋₄alkyl, C₁₋₄ alkyloxycarbonyl, optionally substituted aryl, optionally substituted
X represents O or S;
n is 2 or 3;
wherein C₃₋₁₄ membered carbocycle means any stable 3, 4, 5, 6, 7, 8, 9, 10, 11, 12, 13 or 14-membered monocyclic, bicyclic or tricyclic ring, any of which may be saturated, unsaturated, or aromatic, fused or bridged, recognizing that rings with certain numbers of members cannot be bicyclic or tricyclic, and that when the ring is bridged, the substituents recited for the ring may also be present on the bridge;
provided that when R¹ is H or β-cyanoethyl and n is 3, R² cannot be:
a) uninterrupted and unsubstituted linear or branched C₁₋₄alkyl;
b) -(CH₂)ₘ-Ar wherein m is 1-3 and wherein Ar is a C₃₋₁₀ membered monocyclic or fused bicyclic aromatic carbocycle which is optionally substituted with one or more substituents selected from halogen, unsubstituted O(C₁₋₄alkyl) or C₁₋₄alkyl unsubstituted or substituted with 1 to 3 halogen;
c) -(CH₂)ₘ-Ar wherein m is 0 and wherein Ar is a C₃₋₁₀ membered monocyclic or fused bicyclic aromatic carbocycle which is unsubstituted or substituted by 1 to 3 substituents selected from halogen, unsubstituted O(C₁₋₄alkyl) or C₁₋₄alkyl unsubstituted or substituted with 1 to 3 halogen; or
d) ethoxycarbonyl methyl
or a pharmaceutically acceptable salt, solvate or ester thereof.

2. The compound of claim 1, wherein
R represents H or cladinosyl group of formula (II) 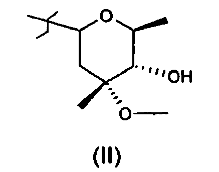
R¹ represents H, β-cyanoethyl, β-amidoethyl or β-(C₁₋₄alkoxycarbonyl)ethyl;
R² represents
a) C₁₋₁₂ alkyl, wherein C₁₋₁₂ alkyl is linear or branched, and unsubstituted or substituted by 1-3 groups selected from halogen, N,N-di(C₁-C₄-alkyl) amino, C(O)OC₁₋₄alkylaryl, (C₁-C₄-alkyl)thio or; phenyl, naphthyl, aryl, furyl, cycloalkyl, thiophenyl, 3,4-methylenedioxyphenyl, morpholinyl, or piperidinyl optionally substituted with one or more substituents selected from halogen, C₁₋₄alkyl unsubstituted or substituted with 1 to 3 halogen,O(C₁₋₄alkyl), O(C₃₋₆ cycloalkyl), O(C₁₋₄alkylaryl), C(O)C₁₋₄alkyl, C₁₋₄ alkyloxycarbonyl, optionally substituted aryl, optionally substituted heteroaryl, C(O)C₁₋₄alkylaryl, C(O)OC₁₋₄alkylaryl;
b) an unsubstituted C₂₋₆ alkenyl; or
c) C₁₋₁₂ cycloalkyl, adamantyl, norbornyl, norbornenyl, phenyl, indanyl, or naphthyl; any of which is unsubstituted or substituted by 1-3 groups selected from halogen; CN; C₁₋₄alkyl unsubstituted or substituted with 1 to 3 of halogen or CN group; O(C₁₋₄alkyl) optionally substituted with 1 to 3 halogen; S(C₁-C₄-alkyl); O(C₃₋₆ cycloalkyl); O(C₁₋₄alkylaryl); C(O)C₁₋₄alkyl; C₁₋₄ alkyloxycarbonyl; aryl; heteroaryl; NO₂; N,N-di(C₁-C₄-alkyl)amino, diazoaryl; and piperidinylsulfonamido; or
d) dihydrobenzofuranyl, C₁₋₃ alkylenedioxyphenyl, benzopyranyl, furyl, isoxazolyl, piperidinyl, pyridinyl, thiophenyl, benzothiadiazolyl, tetrahydrobenzothiophenyl, optionally substituted by 1-3 groups selected from halogen; C₁₋₄alkyl unsubstituted or substituted with 1 to 3 halogen; C₁₋₄ alkyloxycarbonyl; optionally substituted aryl; optionally substituted heteroaryl; C(O)OC₁₋₄alkylaryl; or phenoxy;
e) C(O)aryl;
X represents O or S; and
n is 2 or 3.

3. The compound of claim 2, wherein,
R represents H or cladinosyl group of formula (II) 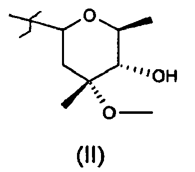
R¹ represents H, β-cyanoethyl, β-amidoethyl or β-(C₁₋₄alkoxycarbonyl)ethyl;
R² represents
a) 3-phenylpropyl, β-phenylethyl, ethoxycarbonylmethyl, isopropyl, 1-(1-naphthyl)-ethyl, t-butyl, n-butyl, sec-butyl, benzyl, 2-furylmethyl, 4-methoxybenzyl, cyclohexylmethyl, ethyl, 2-(2-methyl-5,5-dimethyl)-pentyl, 2-(2-thiophenyl)-ethyl, 3-thiomethylpropyl, 3,4-methylenedioxyphenylmethyl, N-morpholinylethyl, N-morpholinylpropyl, trityl, N-piperidinylethyl, 3-diethylaminopropyl, diphenylmethyl, 3-chloropropyl, isobutyl; or
b) 2-propenyl; or
c) cyclopentyl, cyclopropyl, cyclododecyl, norbornyl, norbomenyl, 2-benzyloxycyclohexyl, adamantyl, phenyl, 1-naphthyl, 4-chlorophenyl, 2-trifluoromethylphenyl, 3,4,5-trimethoxyphenyl, 2-naphthyl, 2,4-dichlorophenyl, 4-cyanophenyl, cyclohexyl, 4-ethylphenyl, 4-methoxyphenyl, 2-methyl-5-fluorophenyl, 4-cyanomethylphenyl, indanyl, 4-acetylphenyl, 2-phenylphenyl, 3-thiomethylphenyl, 3,5-dimethoxycarbonylphenyl, 4-methylphenyl, 2,4-dimethylphenyl, 3,4-difluorophenyl, 3-chlorophenyl, 3-fluorophenyl, 3-cyclopentoxy-4-methoxyphenyl, 4-benzyloxyphenyl, 2-ethylphenyl, 2,6-difluorophenyl, 4-nitrophenyl, 3,5-dichlorophenyl, 2-methoxy-4-nitrophenyl, ethoxycarbonylphenyl, 2-trifluoromethylphenyl, 4-phenylazophenyl, 4-diethylaminophenyl, 3-nitrophenyl, 3-chloro-4-trifluoromethylphenyl, 3,4-dichlorophenyl, 2,3,4-trifluorophenyl, 4-bromophenyl, 4-diazolylphenyl, 4-piperadylsulfonamidophenyl, 1-(4-dimethylamino)-naphthyl, 4-isopropylphenyl, 4-difluoromethoxyphenyl, or 2-methoxy-5-phenylphenyl; or
d) 3,4-methylenedioxyphenyl, 6-fluorobenzo-1,3-pyranyl, dihydrobenzofuranyl, 3,4-propylenedioxyphenyl, 3-(2-trifluoromethyl-5-methyl)-furyl, 4-(3,5-dimethyl)-isoxazole, 4-(3-phenyl-5-methyl)-isoxazole, benzyloxycarbonylpiperidinyl, 4-(2,6-dichloro)-pyridinyl, 2-thiophenyl, benzothiadiazolyl, 3-(2-methoxycarbonyl)-thiophenyl, 2-(3-methoxycarbonyl)-tetrahydrobenzothiophenyl, pyridinyl, 5-(2-morpholinyl)-pyridinyl, 5-(2-phenoxy)-pyridinyl; or
e) C(O)aryl;
X represents O or S; and
n is 2 or 3.

4. Process for the preparation of the compound of formula (I), 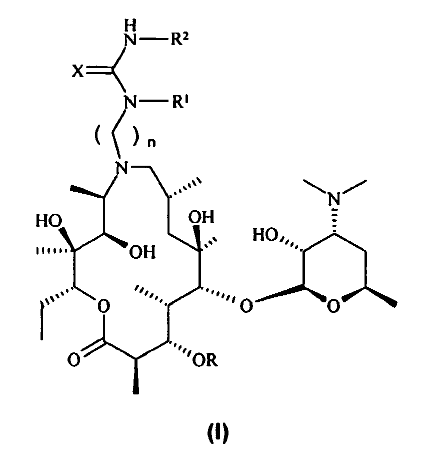 wherein
R represents H or cladinosyl group of formula (II); 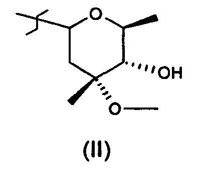
R¹ represents H, β-cyanoethyl, β-amidoethyl or β-(C₁₋₄alkoxycarbonyl)ethyl;
R² represents
a) C₁₋₁₂ alkyl, wherein C₁₋₁₂ alkyl is
i) uninterrupted or interrupted by 1-3 bivalent radical groups selected from - O-, -S- and -N(R³)- ; and/or
ii) unsubstituted or substituted by 1-3 groups selected from halogen; OH; NH₂; N-(C₁-C₄)alkylamino; N,N-di(C₁-C₄-alkyl)amino; CN, NO₂; C(O)OC₁₋₄alkylaryl; a C₃₋₁₄ membered carbocycle optionally substituted with one or more substituents selected from halogen, CN, C₁₋₄alkyl unsubstituted or substituted with 1 to 3 halogen, O(C₁₋₄alkyl) optionally substituted with 1 to 3 halogen, S(C₁-C₄-alkyl), O(C₃₋₆ cycloalkyl), Q(C₁₋₄alkylaryl), C₁₋₄alkylcyano, C(Q)C₁₋₄alkyl, C₁₋₄ alkyloxycarbonyl, optionally substituted aryl, optionally substituted heteroaryl, C(O)C₁₋₄alkylaryl, C(O)OC₁₋₄alkylaryl, NO₂, diazoaryl, sulfo- 5 or 6 membered carbocyclic or heterocyclic ring, C₁₋₄alkyl-C(O)-O-C₁₋₄alkyl and C₁₋₄alkylO-C(O)-NR³; a C₃₋₁₄ membered saturated, unsaturated or aromatic heterocycle containing 1 to 3 heteroatoms selected from the group nitrogen, oxygen, sulphur optionally substituted with halogen, CN, C₁₋₄alkyl unsubstituted or substituted with 1 to 3 halogen, O(C₁₋₄alkyl) optionally substituted with 1 to 3 halogen, S(C₁-C₄-alkyl), O(C₃₋₆ cycloalkyl), O(C₁₋₄alkylaryl), C₁₋₄alkylcyano, C(O)C₁₋₄alkyl, C(O)OC₁₋₄alkylaryl; C₁₋₄ alkyloxycarbonyl, optionally substituted aryl, optionally substituted heteroaryl, C(O)C₁₋₄alkylaryl, NO₂, diazoaryl, sulfo- 5 or 6 membered carbocyclic or heterocyclic ring, C₁₋₄alkyl-C(O)-O-C₁₋₄alkyl and C₁₋₄alkylO-C(O)-NR³; or
b) C₂₋₆ alkenyl containing 0, 1, 2, or 3 heteroatoms selected from O, S, or N, optionally substituted with one or more substituents selected from halogen; CN; NO_{2,}; OH; NH₂; N-(C₁-C₄)alkylamino; N,N-di(C₁-C₄-alkyl)amino; optionally substituted aryl; optionally substituted heteroaryl; or
c) C₃₋₁₄ membered carbocycle which is unsubstituted or substituted by 1-3 groups selected from halogen; OH; CN; C₁₋₄alkyl unsubstituted or substituted with 1 to 3 halogen or CN group; O(C₁₋₄alkyl) optionally substituted with 1 to 3 halogen; S(C₁-C₄-alkyl); O(C₃-₆ cycloalkyl); O(C₁₋₄alkylaryl); C(O)C₁₋₄alkyl; C(O)QC₁₋₄alkylaryl; C₁₋₄ alkyloxycarbonyl; optionally substituted aryl; optionally substituted heteroaryl; C(O)C₁₋₄alkylaryl; NO₂; diazoaryl; sulfo- 5 or 6 membered carbocyclic or heterocyclic ring; C₁₋₄alkyl-C(O)-O-C₁₋₄alkyl and C₁₋₄alkylO-C(O)-NR³; or
d) C₃₋₁₄ membered saturated, unsaturated or aromatic heterocycle containing 1 to 3 heteroatoms selected from the group nitrogen, oxygen, sulphur optionally substituted by 1-3 groups selected from halogen; CN; C₁₋₄alkyl unsubstituted) or substituted with 1 to 3 halogen; Q(C₁₋₄alkyl) optionally substituted with 1 to 3 halogen; S(C₁₋C₄-alkyl); O(C₃₋₆ cycloalkyl); O(C₁₋₄alkylaryl); C₁₋₄alkylcyano; C(O)C₁₋₄alkyl; C₁₋₄ alkyloxycarbonyl; optionally substituted aryl; optionally substituted heteroaryl; C(O)C₁₋₄alkylaryl; C(O)OC₁₋₄alkylaryl; NO₂; diazoaryl; 5 or 6 membered carbocyclic or heterocyclic ring; sulfo- 5 or 6 membered carbocyclic or heterocyclic ring; C₁₋₄alkyl-C(O)-O-C₁₋₄alkyl and C₁₋₄alkylO-C(O)-NR³;
e) C(O)aryl;
R³ represents H or C₁₋₄ alkyl;
X represents O or S;
n is 2 or 3;
wherein C₃₋₁₄ membered carbocycle means any stable 3, 4, 5, 6, 7, 8, 9, 10, 11, 12, 13 or 14-membered monocyclic, bicyclic or tricyclic ring, any of which may be saturated, unsaturated, or aromatic, fused or bridged, recognizing that rings with certain numbers of members cannot be bicyclic or tricyclic, and that when the ring is bridged, the substituents recited for the ring may also be present on the bridge;
provided that when R¹ is H or β-cyanoethyl and n is 3, R² cannot be
a) uninterrupted and unsubstituted linear or branched C₁₋₄alkyl;
b) -(CH₂)ₘ-Ar wherein m is 1-3 and wherein Ar is a C₃₋₁₀ membered monocyclic or fused bicyclic aromatic carbocycle which is optionally substituted with one or more substituents selected from halogen, unsubstituted O(C₁₋₄alkyl) or C₁₋₄alkyl unsubstituted or substituted with 1 to 3 halogen;
c) -(CH₂)ₘ-Ar wherein m is 0 and wherein Ar is a C₃₋₁₀ membered monocyclic, or fused bicyclic aromatic carbocycle which is unsubstituted or substituted by 1 to 3 substituents selected from halogen, unsubstituted Q(C₁₋₄alkyl) or C₁₋₄alkyl unsubstituted or substituted with 1 to 3 halogen; or
d) ethoxycarbonylmethyl
or a pharmaceutically acceptable salt, solvate or ester thereof, wherein
a compound of formula **(III)** 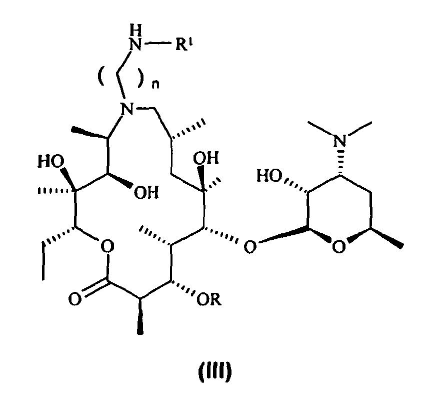 is reacted with an isocyanate or a thioisocyanate of formula **(IV),**
R²-N=C=X **(IV)**
in an aprotic solvent selected from toluene, xylene and dichloromethane, at a temperature from about 0° to 110°C.

5. Use of a compound according to claim 1 or a pharmaceutically acceptable salt, solvate or ester thereof in the manufacture of a medicament for the therapeutic and/or prophylactic treatment of malaria.

6. Use as claimed in claim 5, wherein the subject has been infected with *Plasmodium falciparum*.

7. Use as claimed in claim 5, wherein the subject has been infected with *P. vivax*.

8. Use as claimed in claim 5, wherein the subject has been infected with *P. ovale*.

9. Use as claimed in claim 5, wherein the subject has been infected with *P. ma*/*ariae*.

10. Use as claimed in claim 5, wherein the medicament is administered after the subject has been exposed to the malaria parasite.

11. Use as claimed in claim 5, wherein the medicament is administered before the subject travels to a country where malaria is endemic.

12. Use as claimed in claim 11, wherein the malaria is caused by a drug-resistant malarial strain.

13. Use as claimed in claim 12, wherein the drug-resistant malarial strain is resistant to at least one of chloroquine, mefloquine, halofantrine, artemisinin, atovaquone/proguanil, doxycycline or primaquine.

14. Use as claimed in claim 5, wherein the therapeutic and/or prophylactic treatment is in a human.

15. A pharmaceutical preparation comprising the compound of claim 1 or a pharmaceutically acceptable salt, solvate or ester thereof, and at least one pharmaceutically acceptable carrier.

16. The pharmaceutical preparation of claim 15, wherein the preparation is for the treatment of malarial infections.

## Patentansprüche

1. Eine Verbindung der Formel (I) 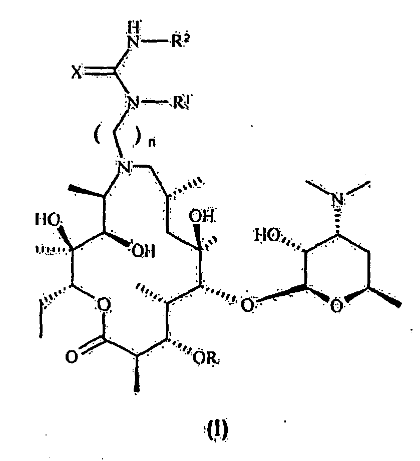 wobei
R H oder einen Cladinosylrest der Formel (II) darstellt; 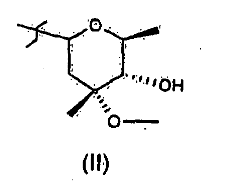
R¹ H, β-Cyanoethyl, β-Amidoethyl oder β-(C₁₋₄-Alkoxycarbonyl)ethyl darstellt;
R²
a) C₁₋₁₂-Alkyl, wobei C₁₋₁₂-Alkyl.
i) ununterbrochen oder unterbrochen durch 1 bis 3 bivalente radikalische Reste, ausgewählt aus -O-, -S- und -N(R³)-; und/oder
ii) linear oder verzweigt und unsubstituiert oder substituiert mit 1 bis 3 Resten, ausgewählt aus Halogen, OH; NH₂; N-(C₁-C₄)-Alkylamino; N,N-Di(C₁-C₄-alkyl)amino, CN, NO₂; C(O)OC₁₋₄-Alkylaryl, (C₁-C₄-Alkyl)thio; einem C₃₋₁₄-gliedrigen Carbocyclus, gegebenenfalls substituiert mit einem oder mehreren Substituenten, ausgewählt aus Halogen, CN, C₁₋₄-Alkyl, unsubstituiert oder substituiert mit 1 bis 3 Halogenatomen, O(C₁₋₄-Alkyl), gegebenenfalls substituiert mit 1 bis 3 Halogenatomen, S(C₁-C₄-Alkyl), O(C₃₋₆-Cycloalkyl), O(C₁₋₄-Alkylaryl), C₁₋₄-Alkylcyano, C(O)C₁₋₄-Alkyl, C₁₋₄-Alkyloxycarbonyl, gegebenenfalls substituiertem Aryl, gegebenenfalls substituiertem Heteroaryl, C(O)C₁₋₄-Alkylaryl, C(O)OC₁₋₄-Alkylaryl, NO₂, Diazoaryl, einem sulfonierten 5- oder 6-gliedrigen carbocyclischen oder heterocyclischen Ring, C₁₋₄-Alkyl-C(O)-O-C₁₋₄-alkyl und C₁₋₄-Alkyl-O-C(0)-NR³; einem C₃₋₁₄-gliedrigen gesättigten, ungesättigten oder aromatischen Heterocyclus mit 1 bis 3 Heteroatomen, ausgewählt aus der Gruppe Stickstoff, Sauerstoff, Schwefel, gegebenenfalls substituiert mit Halogen, CN, C₁₋₄-Alkyl, unsubstituiert oder substituiert mit 1 bis 3 Halogenatomen, O(C₁₋₄-Alkyl), gegebenenfalls substituiert mit 1 bis 3 Halogenatomen, S(C₁-C₄-Alkyl), O(C₃₋₆-Cycloalkyl), O(C₁₋₄-Alkylaryl), C₁₋₄-Alkylcyano, C(O)C₁₋₄-Alkyl, C(O)OC₁₋₄-Alkylaryl; C₁₋₄-Alkyloxycarbonyl, gegebenenfalls substituiertem Aryl, gegebenenfalls substituiertem Heteroaryl, C(O)C₁₋₄-Alkylaryl, NO₂, Diazoaryl, einem sulfonierten 5- oder 6- gliedrigen carbocyclischen oder heterocyclischen Ring, C₁₋₄-Alkyl-C(O)-O-C₁₋₄-alkyl und C₁₋₄-AlkylO-C(O)-NR³; ist oder
b) C₂₋₆-Alkenyl, das 0, 1, 2 oder 3 Heteratome, ausgewählt aus O, S oder N, enthält, gegebenenfalls substituiert mit einem oder mehreren Substituenten, ausgewählt aus Halogen; CN; NO₂; OH; NH₂; N-(C₁-C₄)-Alkylamino; N,N-Di(C₁-C₄-alkyl)amino; gegebenenfalls substituiertem Aryl; gegebenenfalls substituiertem Heteroaryl; oder
c) einen C₃₋₁₄-gliedrigen Carbocyclus, unsubstituiert oder substituiert mit 1 bis 3 Resten, ausgewählt aus Halogen; OH; CN; C₁₋₄-Atkyl, unsubstituiert oder substituiert mit 1 bis 3 Halogenatomen oder einem CN-Rest; O(C₁₋₄Alkyl), gegebenenfalls substituiert mit 1 bis 3 Halogenatomen; S(C₁-C₄-Alkyl); O(C₃₋₆-Cycloalkyl); O(C₁₋₄-Alkylaryl); C(O)C₁₋₄-Alkyl; C(O)OC₁₋₄-Alkylaryl; C₁₋₄-Alkyloxycarbonyl; gegebenenfalls substituiertem Aryl; gegebenenfalls substituiertem Heteroaryl; C(O)C₁₋₄-Alkylaryl; NO₂; N-(C₁-C₄)-Alkylamino; N,N-Di(C₁-C₄-alkyl)aminodiazoaryl; einem sulfonierten 5- oder 6-gliedrigen carbocyclischen oder heterocyclischen Ring; C₁₋₄-Alkyl-C(O)-O-C₁₋₄-alkyl und C₁₋₄-AlkylO-C(O)-NR³; oder
d) einen C₃₋₁₄-gliedrigen gesättigten, ungesättigten oder aromatischen Heterocyclus mit 1 bis 3 Heteroatomen, ausgewählt aus der Gruppe Stickstoff, Sauerstoff, Schwefel, gegebenenfalls substituiert mit 1 bis 3 Resten, ausgewählt aus Halogen; CN; C₁₋₄-Alkyl, unsubstituiert oder substituiert mit 1 bis 3 Halogenatomen; O(C₁₋₄-Alkyl), gegebenenfalls substituiert mit 1 bis 3 Halogenatomen; S(C₁-C₄-Alkyl); O(C₃₋₆-Cycloalkyl); O(C₁₋₄-Alkylaryl); C₁₋₄-Alkylcyano; C(O)C₁₋₄-Alkyl; C₁₋₄-Alkyloxycarbonyl; gegebenenfalls substituiertem Aryl, gegebenenfalls substituiertem Heteroaryl; C(O)C₁₋₄-Alkylaryl, C(O)OC₁₋₄-Alkylaryl; NO₂; Diazoaryl; einem 5- oder 6-gliedrigen carbocyclischen oder heterocyclischen Ring; einem sulfonierten 5- oder 6-gliedrigen carbocyclischen oder heterocyclischen Ring; C₁₋₄-Alkyl-C(O)-O-C₁₋₄-alkyl und C₁₋₄-AlkylO-C(O)-NR³;
e) C(O)Aryl;
darstellt;
R³ H oder C₁₋₄-Alkyl darstellt;
X O oder S darstellt;
n 2 oder 3 ist;
wobei ein C₃₋₁₄-gliedriger Carbocyclus irgendeinen stabilen 3-, 4-, 5-, 6-, 7-, 8-, 9-, 10-, 11-, 12-, 13- oder 14-gliedrigen monocyclischen, bicyclischen oder tricyclischen Ring bedeutet, von denen jeder gesättigt, ungesättigt oder aromatisch, kondensiert oder verbrückt sein kann, unter Berücksichtigung dessen, dass Ringe mit einer bestimmten Anzahl an Gliedern nicht bicyclisch und tricyclisch sein können, und dass wenn der Ring verbrückt ist, die für den Ring angegebenen Substituenten auch an der Verbrückung vorliegen können;
mit der Maßgabe, dass wenn R¹ H oder β-Cyanoethyl ist und n 3 ist, R² nicht:
a) ununterbrochenes und unsubstituiertes lineares oder verzweigtes C₁₋₄-Alkyl;
b) -(CH₂)ₘ,-Ar, wobei m 1 bis 3 ist und wobei Ar ein C₃₋₁₀-gliedriger monocyclischer oder kondensierter bicyclischer aromatischer Carbocyclus ist, der gegebenenfalls mit einem oder mehreren Substituenten, ausgewählt aus Halogen, unsubstituiertem O(C₁₋₄-Alkyl) oder C₁₋₄-Alkyl, unsubstituiert oder substituiert mit 1 bis 3 Halogenatomen, substituiert ist;
c) -(CH₂)ₘ-Ar, wobei m 0 ist und wobei Ar ein C₃₋₁₀-gliedriger monocyclischer oder kondensierter bicyclischer aromatischer Carbocyclus ist, der unsubstituiert oder mit bis 3 Substituenten, ausgewählt aus Halogen, unsubstituiertem O(C₁₋₄-Alkyl) oder C₁₋₄-Alkyl, unsubstituiert oder substituiert mit 1 bis 3 Halogenatomen, substituiert ist; oder
d) ethoxycarbonylmethyl
sein kann
oder ein pharmazeutisch verträgliches Salz, Solvat oder ein pharmazeutisch verträglicher Ester davon.

2. Die Verbindung nach Anspruch 1, wobei
R H oder einen Cladinosylrest der Formel (II) darstellt 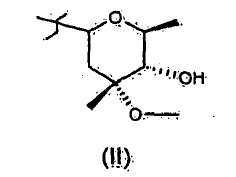
R¹ H, β-Cyanoethyl, β-Amidoethyl oder β-(C₁₋₄-Alkoxycarbonyl)ethyl darstellt;
R²
a) C₁₋₁₂-Alkyl, wobei C₁₋₁₂-Alkyl linear oder verzweigt und unsubstituiert oder substituiert mit 1 bis 3 Resten, ausgewählt aus Halogen, N,N-Di(C₁-C₄-alkyl)amino, C(O)OC₁₋₄-Alkylaryl, (C₁-C₄-Alkyl)thio; oder Phenyl, Naphthyl, Aryl, Furyl, Cycloalkyl, Thiophenyl, 3,4-Methylendioxyphenyl, Morpholinyl oder Piperidinyl, gegebenenfalls substituiert mit einem oder mehreren Substituenten, ausgewählt aus Halogen, C₁₋₄-Alkyl, unsubstituiert oder substituiert mit 1 bis 3 Halogenatomen, O(C₁₋₄-Alkyl), O(C₃₋₆-Cycloalkyl), O(C₁₋₄-Alkylaryl), C(O)C₁₋₄-Alkyl, C₁₋₄-Alkyloxycarbonyl, gegebenenfalls substituiertem Aryl, gegebenenfalls substituiertem Heteroaryl, C(O)C₁₋₄-Alkylaryl, C(O)OC₁₋₄-Alkylaryl, ist;
b) ein unsubstituiertes C₂₋₆-Alkenyl; oder
c) C₁₋₁₂-Cycloalkyl, Adamantyl, Norbomyl, Norbomenyl, Phenyl, Indanyl oder Naphthyl; von denen jeder unsubstituiert oder mit 1 bis 3 Resten, ausgewählt aus Halogen; CN; C₁₋₄-Alkyl, unsubstitutiert oder substituiert mit 1 bis 3 Halogenatomen oder CN-Resten, O(C₁₋₄-Alkyl), gegebenenfalls substituiert mit 1 bis 3 Halogenatomen; S(C₁-C₄-Alkyl); O(C₃₋₆-Cycloalkyl), O(C₁₋₄-Alkylaryl), C(O)C₁₋₄-Alkyl; C₁₋₄-Alkyloxycarbonyl; Aryl; Heteroaryl; NO₂; N,N-Di(C₁-C₄-alkyl)amino; Diazoaryl; und Piperidinylsulfonamido, substituiert ist; oder
d) Dihydrobenzofuranyl, C₁₋₃-Alkylendioxyphenyl, Benzopyranyl, Furyl, Isoxazolyl, Piperidinyl, Pyridinyl, Thiophenyl, Benzothiadiazolyl, Tetrahydrobenzothiophenyl, gegebenenfalls substituiert mit 1 bis 3 Resten, ausgewählt aus Halogen; C₁₋₄-Alkyl, unsubstituiert oder substituiert mit 1 bis 3 Halogenatomen; C₁₋₄-Alkyloxycarbanyl; gegebenenfalls substituiertem Aryl; gegebenenfalls substituiertem Heteroaryl; C(O)OC₁₋₄-Alkylaryl; oder Phenoxy;
e) C(O)Aryl;
darstellt;
X O oder S darstellt; und
n 2 oder 3 ist.

3. Die Verbindung nach Anspruch 2, wobei
R H oder einen Cladinosylrest der Formel (II) darstellt 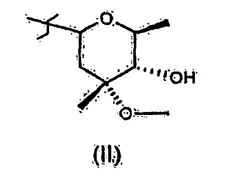
R¹ H, β-Cyanoethyl, β-Amidoethyl oder β-(C₁₋₄-Alkoxycarbonyl)ethyl darstellt;
R²
a) 3-Phenylpropyl, β-Phenylethyl, Ethoxycarbonylmethyl, Isopropyl, 1-(1-Naphthyl)-ethyl, t-Butyl, n-Butyl, sek.-Butyl, Benzyl, 2-Furylmethyl, 4-Methoxybenzyl, Cyclohexylmethyl, Ethyl, 2-(2-Methyl-5,5-dimethyl)pentyl, 2-(2-Thiophenyl)-ethyl, 3-Thiomethylpropyl, 3,4-Methylendioxyphenylmethyl, N-Morpholinylethyl, N-Morpholinylpropyl, Trityl, N-Piperidinylethyl, 3-Diethylaminopropyl, Diphenylmethyl, 3-Chlorpropyl, Isobutyl; oder
b) 2-Propenyl; oder
c) Cyclopentyl, Cyclopropyl, Cyclododecyl, Norbornyl, Norbomenyl, 2-Benzyloxycyclohexyl, Adamantyl, Phenyl, 1-Naphthyl, 4-Chlorphenyl, 2-Trifluormethylphenyl, 3,4,5-Trimethoxyphenyl, 2-Naphthyl, 2,4-Dichlorphenyl, 4-Cyanophenyl, Cyclohexyl, 4-Ethylphenyl, 4-Methoxyphenyl, 2-Methyl-5-fluorphenyl, 4-Cyanomethylphenyl, Indanyl, 4-Acetylphenyl, 2-Phenylphenyl, 3-Thiomethylphenyl, 3,5-Dimethoxycarbonylphenyl, 4-Methylphenyl, 2,4-Dimethylphenyl, 3,4-Difluorphenyl, 3-Chlorphenyl, 3-Fluorphenyl, 3-Cyclopentoxy-4-methoxyphenyl, 4-Benzyloxyphenyl, 2-Ethylphenyl, 2,6-Difluorphenyl, 4-Nitrophenyl, 3,5-Dichlorphenyl, 2-Methoxy-4-nitrophenyl, Ethoxycarbonylphenyl, 2-Trifluormethylphenyl, 4-Phenylazophenyl, 4-Diethylaminophenyl, 3-Nitrophenyl, 3-Chlor-4-trifluormethylphenyl, 3,4-Dichlorphenyl, 2,3,4-Trifluorphenyl, 4-Bromphenyl, 4-Diazolylphenyl, 4-Piperadylsulfonamidophenyl, 1-(4-Dimethylamino)naphthyl, 4-Isopropylphenyl, 4-Difluormethoxyphenyl oder 2-Methoxy-5-phenylphenyl; oder
d) 3,4-Methylendioxyphenyl, 6-Fluorbenzo-1,3-pyranyl, Dihydrobenzofuranyl, 3,4-Propylendioxyphenyl, 3-(2-Trifluormethyl-5-methyl)furyl, 4-(3,5-Dimethyl)-isoxazol, 4-(3-Phenyl-5-methyl)isoxazol, Benzyloxycarbonylpiperidinyl, 4-(2,6-Dichlor)pyridinyl, 2-Thiophenyl, Benzothiadiazolyl, 3-(2-Methoxycarbonyl)-thiophenyl, 2-(3-Methoxycarbonyl)tetrahydrobenzothiophenyl, Pyridinyl, 5-(2-Morpholinyl)pyridinyl, 5-(2-Phenoxy)pyridinyl; oder
e) C(O)Aryl;
darstellt;
X O oder S darstellt; und
n 2 oder 3 ist.

4. Verfahren zur Herstellung der Verbindung der Formel (I) 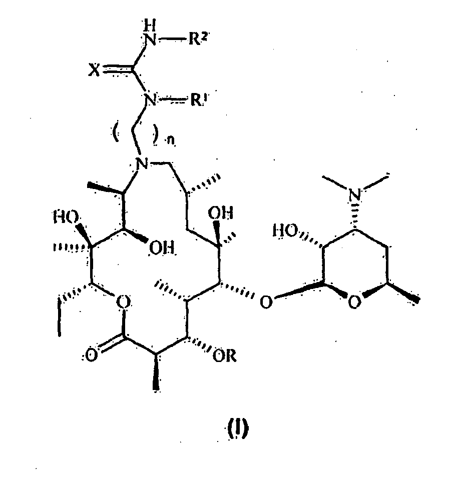 wobei
R H oder einen Cladinosylrest der Formel (II) darstellt; 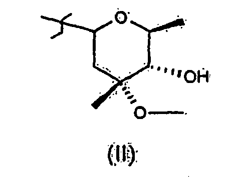
R¹ H, β-Cyanoethyl, β-Amidoethyl oder β-(C₁₋₄-Alkoxycarbonyl)ethyl darstellt;
R²
a) C₁₋₁₂-Alkyl, wobei C₁₋₁₂-Alkyl
i) ununterbrochen oder unterbrochen durch 1 bis 3 bivalente radikalische Reste, ausgewählt aus -O-, -S- und -N(R³)-; und/oder
ii) unsubstituiert oder substituiert mit 1 bis 3 Resten, ausgewählt aus Halogen; OH; NH₂; N(C₁-C₄)-Alkylamino; N,N-Di(C₁-C₄-alkyl)amino; CN, NO₂; C(O)OC₁₋₄-Alkylaryl; einem C₃₋₁₄-gliedrigen Carbocyclus, gegebenenfalls substituiert mit einem oder mehreren Substituenten, ausgewählt aus Halogen, CN, C₁₋₄-Alkyl, unsubstituiert oder substituiert mit 1 bis 3 Halogenatomen, O(C₁₋₄-Alkyl), gegebenenfalls substituiert mit 1 bis 3 Halogenatomen, S(C₁-C₄-Alkyl), O(C₃₋₆-Cycloalkyl), O(C₁₋₄-Alkylaryl), C₁₋₄-Alkylcyano, C(O)C₁₋₄-Alkyl, C₁₋₄-Alkyloxycarbonyl, gegebenenfalls substituiertem Aryl, gegebenenfalls substituiertem Heteroaryl, C(O)C₁₋₄-Alkylaryl, C(0)OC₁₋₄-Alkylaryl, NO₂, Diazoaryl, einem sulfonierten 5- oder 6-gliedrigen carbocyclischen oder heterocyclischen Ring, C₁₋₄-Alkyl-C(O)-O-C₁₋₄-Alkyl und C₁₋₄-AlkylO-C(O)-NR³; einem C₃₋₁₄-gliedrigen gesättigten, ungesättigten oder aromatischen Heterocyclus mit 1 bis 3 Heteroatomen, ausgewählt aus der Gruppe Stickstoff, Sauerstoff, Schwefel, gegebenenfalls substituiert mit Halogen, CN, C₁₋₄-Alkyl, unsubstituiert oder substituiert mit 1 bis 3 Halogenatomen, O(C₁₋₄-Alkyl), gegebenenfalls substituiert mit 1 bis 3 Halogenatomen, S(C₁-C₄-Alkyl), O(C₃₋₆-Cycloalkyl), O(C₁₋₄-Alkylaryl), C₁₋₄-Alkylcyano, C(O)C₁₋₄-Alkyl, C(O)OC₁₋₄-Alkylaryl; C₁₋₄-Alkyloxycarbonyl, gegebenenfalls substituiertem Aryl, gegebenenfalls substituiertem Heteroaryl, C(O)C₁₋₄-Alkylaryl, NO₂, Diazoaryl, einem sulfonierten 5- oder 6-gliedrigen carbocyclischen oder heterocyclischen Ring; C₁₋₄-Alkyl-C(O)-O-C₁₋₄-alkyl und C₁₋₄-AlkylO-C(O)-NR, ist; oder
b) C₂₋₆-Alkenyl, das 0, 1, 2 oder 3 Heteroatome, ausgewählt aus O, S oder N, enthält, gegebenenfalls substituiert mit einem oder mehreren Substituenten, ausgewählt aus Halogen; CN; NO₂; OH; NH₂; N-(C₁-C₄)-Alkylamino; N,N-Di(C₁-C₄-alkyl)amino; gegebenenfalls substituiertem Aryl; gegebenenfalls substituiertem Heteroaryl; oder
c) einen C₃₋₁₄-gliedrigen Carbocyclus, der unsubstituiert oder mit 1 bis 3 Resten substituiert ist, ausgewählt aus Halogen; OH; CN; C₁₋₄-Alkyl, unsubstituiert oder substituiert mit 1 bis 3 Halogenatomen oder CN-Resten; O(C₁₋₄-Alkyl), gegebenenfalls substituiert mit 1 bis 3 Halogenatomen; S(C₁-C₄-Alkyl); O(C₃₋₆-Cycloalkyl); O(C₁₋₄-Alkylaryl); C(O)C₁₋₄-Alkyl; C(O)OC₁₋₄-Alkylaryl; C₁₋₄-Alkyloxycarbonyl; gegebenenfalls substituiertem Aryl; gegebenenfalls substituiertem Heteroaryl; C(O)C₁₋₄-Alkylaryl; NO₂; Diazoaryl; einem sulfonierten 5- oder 6-gliedrigen carbocyclischen oder heterocyclischen Ring, C₁₋₄-Alkyl-C(O)-O-C₁₋₄-alkyl und C)₁₋₄-AlkylO-C(O)-NR³, oder
d) einen C₃₋₁₄-gliedrigen gesättigten, ungesättigten oder aromatischen Heterocyclus mit 1 bis 3 Heteroatomen, ausgewählt aus der Gruppe Stickstoff, Sauerstoff, Schwefel, gegebenenfalls substituiert mit 1 bis 3 Resten, ausgewählt aus Halogen; CN; C₁₋₄-Alkyl, unsubstituiert oder substituiert mit 1 bis 3 Halogenatomen; O(C₁₋₄-Alkyl), gegebenenfalls substituiert mit 1 bis 3 Halogenatomen; S(C₁-C₄-Alkyl); O(C₃₋₆-Cycloalkyl); O(C₁₋₄-Alkylaryl); C₁₋₄-Alkylcyano; C(O)C₁₋₄-Alkyl; C₁₋₄-Alkyloxycarbonyl; gegebenenfalls substituiertem Aryl, gegebenenfalls substituiertem Heteroaryl; C(O)C₁₋₄-Alkylaryl; C(O)OC₁₋₄-Alkylaryl; NO₂; Diazoaryl; einem 5- oder 6-gliedrigen carbocyclischen oder heterocyclischen Ring; einem sulfonierten, 5- oder 6-gliedrigen carbocyclischen oder heterocyclischen Ring; C₁₋₄-Alkyl-C(O)-O-C₁₋₄-alkyl und C₁₋₄-AlkylO-C(O)-NR³;
e) C(O)Aryl;
darstellt;
R³ H oder C₁₋₄-Alkyl darstellt;
X O oder S darstellt;
n 2 oder 3 ist;
wobei ein C₃₋₁₄-gliedriger Carbocyclus irgendeinen stabilen 3-, 4-, 5-, 6-, 7-, 8-, 9-, 10-, 11-, 12-, 13- oder 14-gliedrigen monocyclischen, bicyclischen oder tricyclischen Ring bedeutet, von denen jeder gesättigt, ungesättigt oder aromatisch, kondensiert oder verbrückt sein kann, unter Berücksichtigung dessen, dass Ringe mit einer bestimmten Anzahl an Gliedern nicht bicyclisch oder tricyclisch sein können, und dass wenn der Ring verbrückt ist, die für den Ring angegebenen Substituenten auch an der Verbrückung vorliegen können;
mit der Maßgabe, dass wenn R¹ H oder β-Cyanoethyl ist und n 3 ist, R² nicht
a) ununterbrochenes und unsubstituiertes lineares oder verzweigtes C₁₋₄-Alkyl;
b) -(CH₂)ₘ-Ar, wobei m 1 bis 3 ist und wobei Ar ein C₃₋₁₀-gliedriger monocyclischer oder kondensierter bicyclischer aromatischer Carbocyclus ist, der gegebenenfalls mit einem oder mehreren Substituenten, ausgewählt aus Halogen, unsubstituiertem 0(C₁₋₄-Alkyl) oder C₁₋₄-Alkyl, unsubstituiert oder substituiert mit 1 bis 3 Halogenatomen, substituiert ist;
c) -(CH₂)ₘ-Ar, wobei m 0 ist und wobei Ar ein C₃₋₁₀-gliedriger monocyclischer oder kondensierter bicyclischer aromatischer Carbocyclus ist, der unsubstituiert oder mit 1 bis 3 Substituenten, ausgewählt aus Halogen, unsubstituiertem O(C₁₋₄-Alkyl) oder C₁₋₄-Alkyl, unsubstituiert oder substituiert mit 1 bis 3 Halogenatomen, substituiert ist; oder
d) ethoxycarbonylmethyl
sein kann
oder ein pharmazeutisch verträgliches Salz, Solvat oder ein pharmazeutisch verträglicher Ester davon, wobei
eine Verbindung der Formel (III) 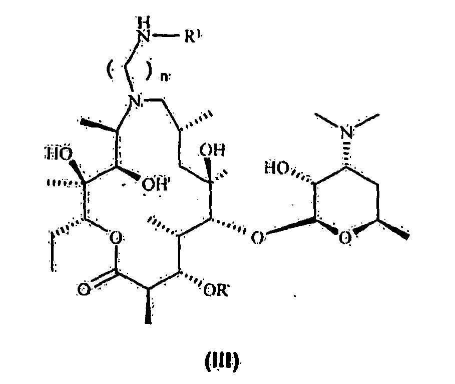 mit einem Isocyanat oder einem Thioisocyanat der Formel (IV)
R²-N=C=X. **(IV)**
in einem aprotischen Lösungsmittel, ausgewählt aus Toluol, Xylol und Dichlormethan, bei einer Temperatur von etwa 0°C bis 110°C umgesetzt wird.

5. Verwendung einer Verbindung nach Anspruch 1 oder eines pharmazeutisch verträglichen Salzes, Solvats oder Esters davon bei der Herstellung eines Medikaments zur therapeutischen und/oder prophylaktischen Behandlung von Malaria.

6. Verwendung nach Anspruch 5, wobei der Patient mit *Plasmodium falciparum* infiziert wurde.

7. Verwendung nach Anspruch 5, wobei der Patient mit *P. vivax* infiziert wurde.

8. Verwendung nach Anspruch 5, wobei der Patient mit *P. ovale* infiziert wurde.

9. Verwendung nach Anspruch 5, wobei der Patient mit *P. malariae* infiziert wurde.

10. Verwendung nach Anspruch 5, wobei das Medikament verabreicht wird, nachdem der Patient dem Malaria-Parasiten ausgesetzt war.

11. Verwendung nach Anspruch 5, wobei das Medikament verabreicht wird, bevor der Patient in ein Land reist, in dem Malaria endemisch ist.

12. Verwendung nach Anspruch 11, wobei die Malaria durch einen Arzneistoff-resistenten Malariastamm hervorgerufen wird.

13. Verwendung nach Anspruch 12, wobei der Arzneistoff-resistente Malariastamm gegen mindestens eines aus Chloroquin, Mefloquin, Halofantrin, Artemisinin, Atovaquon/Proguanil, Doxycyclin oder Primachin resistent ist.

14. Verwendung nach Anspruch 5, wobei die therapeutische und/oder prophylaktische Behandlung an einem Menschen erfolgt.

15. Ein Arzneimittel, umfassend die Verbindung nach Anspruch 1 oder ein pharmazeutisch verträgliches Salz, Solvat oder einen pharmazeutisch verträglichen Ester davon und mindestens einen pharmazeutisch verträglichen Träger.

16. Das Arzneimittel nach Anspruch 15, wobei das Arzneimittel zur Behandlung von Malariainfektionen bestimmt ist.

## Revendications

1. Composé de formule (I) 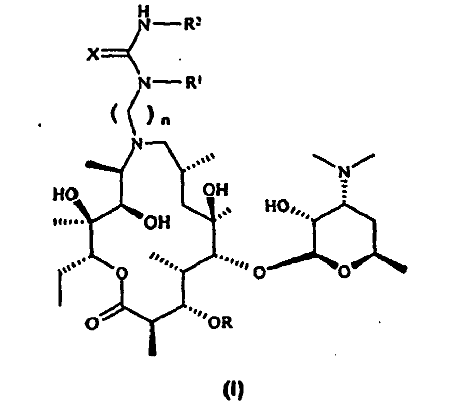 dans laquelle
R représente H ou un groupe cladinosyle de formule (II) : 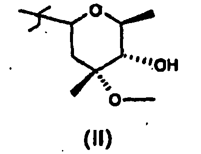
R¹ représente H, un groupe β-cyanoéthyle, β-amidoéthyle ou β-((alkoxy en C₁ à C₄)carbonyl)éthyle ;
R² représente
a) un groupe alkyle en C₁ à C₁₂, ledit groupe alkyle en C₁ à C₁₂ étant
i) non interrompu ou interrompu avec 1 à 3 groupes consistant en radicaux bivalents choisis entre des groupes -O-, -S- et -N(R³)- ; et/ou
ii) linéaire ou ramifié, et non substitué ou substitué avec 1 à 3 groupes choisis entre des groupes halogéno, OH; NH₂; N- (alkyle en C₁ à C₄)amino; N, N-di (alkyle en C₁ à C₄)amino, CN, NO₂ ; C(O)O-(alkyle en C₁ à C₄)aryle, (alkyle en C₁ à C₄)-thio ; un carbocycle en C₃ à C₁₄ facultativement substitué avec un ou plusieurs substituants choisis entre des substituants halogéno, CN, alkyle en C₁ à C₄ non substitué ou substitué avec 1 à 3 substituants halogéno, O(alkyle en C₁ à C₄) facultativement substitué avec 1 à 3 substituants halogéno, S(alkyle en C₁ à C₉), O(cycloalkyle en C₃ à C₆), O((alkyle en C₁ à C₄) aryle), (alkyle en C₁ à C₄)cyano, C(O) (alkyle en C₁ à C₄), (alkyle en C₁ à C₄)oxycarbonyle, aryle facultativement substitué, hétéroaryle facultativement substitué, C(O)(alkyle en C₁ à C₄)aryle, C(O)O(alkyle en C₁ à C₄)aryle, NO₂, diazoaryle, sulfo-(noyau carbocyclique ou hétérocyclique penta- ou hexagonal), (alkyle en C₁ à C₄)-C(O)-O-(alkyle en C₁ à C₄) et (alkyle en C₁ à C₉)O-C(O)-NR³; un hétérocycle en C₃ à C₁₄ saturé, insaturé ou aromatique contenant 1 à 3 hétéroatomes choisis dans le groupe comprenant l'azote, l'oxygène et le soufre facultativement substitué, avec un substituant halogéno, CN, alkyle en C₁ à C₉ non substitué ou substitué avec 1 à 3 substituants halogéno, O(alkyle en C₁ à C₄) facultativement substitué avec 1 à 3 substituants halogéno, S(alkyle en C₁ à C₄), O(cycloalkyle en C₃ à C₆), O((alkyle en C₁ à C₄)aryle), (alkyle en C₁ à C₄)-cyano, C(O)(alkyle en C₁ à C₄), C(O)O(alkyle en C₁ à C₄) aryle; (alkyle en C₁ à C₄)oxycarbonyle, aryle facultativement substitué, hétéroaryle facultativement substitué, C(O)(alkyle en C₁ à C₄)aryle; NO₂ diazoaryle, sulfo-(noyau carbocyclique ou hétérocyclique penta- ou hexagonal), (alkyle en C₁ à C₄)C(O)-O-(alkyle en C₁ à C₄) et (alkyle en C₁ à C₄)O-C(O)-NR³; ou
b) un groupe alcényle en C₂ à C₆ contenant 0, 1, 2 ou 3 hétéroatomes choisis entre 0, S et N, facultativement substitué avec un ou plusieurs substituants choisis entre des substituants halogéno; CN ; NO₂; OH ; NH₂; N-(alkyle en C₁ à C₄)amino; N,N-di(alkyle en C₁ à C₄)-amino ; aryle facultativement substitué ; hétéroaryle facultativement substitué; ou
c) un carbocycle en C₃ à C₁₄ qui est non substitué ou substitué avec 1 à 3 groupes choisis entre des groupes halogéno ; OH ; CN ; alkyle en C₁ à C₄ non substitué ou substitué avec 1 à 3 substituants halogéno ou groupes CN ; O(alkyle en C₁ à C₄) facultativement substitué avec 1 à 3 substituants halogéno ; S(alkyle en C₁ à C₄) ; O(cycloalkyle en C₃ à C₆) ; O((alkyle en C₁ à C₄)aryle) ; C(O)(alkyle en C₁ à C₄) ; C(O)O(alkyle en C₁ à C₄)aryle ; (alkyle en C₁ à C₄)oxycarbonyle ; aryle facultativement substitué ; hétéroaryle facultativement substitué ; C(O)(alkyle en C₁ à C₄)aryle ; NO₂ ; N-(alkyle en C₁ à C₄)amino ; N,N-di(alkyle en C₁ à C₄)amino ; diazoaryle ; sulfo-(noyau carbocyclique ou hétérocyclique penta- ou hexagonal) ; (alkyle en C₁ à C₄)C(O)-O-(alkyle en C₁ à C₄) et (alkyle en C₁ à C₄)O-C(O)-NR³ ; ou
d) un hétérocycle en C₃ à C₁₄ saturé, insaturé ou aromatique contenant 1 à 3 hétéroatomes choisis dans le groupe comprenant l'azote, l'oxygène et le soufre facultativement substitué avec 1 à 3 groupes choisis entre des groupes halogéno ; CN ; alkyle en C₁ à C₄ non substitué ou substitué avec 1 à 3 substituants halogéno ; O(alkyle en C₁ à C₄) facultativement substitué avec 1 à 3 substituants halogéno ; S(alkyle en C₁ à C₄) ; O(cycloalkyle en C₃ à C₆) : O((alkyle en C₁ à C₄)aryle) ; (alkyle en C₁ à C₄)cyano ; C(O)(alkyle en C₁ à C₄) ; (alkyle en C₁ à C₄)oxycarbonyle ; aryle facultativement substitué ; hétéroaryle facultativement substitué ; C(O)(alkyle en C₁ à C₄)aryle ; C(O)O-(alkyle en C₁ à C₄)aryle ; NO₂ ; diazoaryle ; un noyau carbocyclique ou hétérocyclique penta- ou hexagonal ; sulfo-(noyau carbocyclique ou hétérocyclique penta- ou hexagonal) ; (alkyle en C₁ à C₄)-C(O)-O-(alkyle en C₁ à C₄) et (alkyle en C₁ à C₄)C)-C(O)-NR³ ;
e) un groupe C(O)aryle ;
R³ représente H ou un groupe alkyle en C₁ à C₄ ;
X représente 0 ou S ;
n est égal à 2 ou 3
où l'expression "carbocyle en C₃ à C₁₄" désigne n'importe quel noyau monocyclique, bicyclique ou tricyclique stable de 3,4,5,6,7,8,9,10,11,12,13 ou 14 membres, n'importe lequel de ces noyaux pouvant être saturé, insaturé ou aromatique, condensé ou ponté, en sachant que des noyaux avec certains nombres de membres ne peuvent être bicycliques ou tricycliques et que lorsque le noyau est ponté, les substituants indiqués pour le noyau peuvent également être présents sur le pont ;
sous réserve que, lorsque R¹ représente H ou un groupe β-cyanoéthyle et n est égal à 3 ; R² ne peut être :
a) un groupe alkyle en C₁ à C₄ linéaire ou ramifié non interrompu et non substitué :
b) un groupe -(CH₂)ₘ-Ar dans lequel m a une valeur de 1 à 3 et dans lequel Ar représente un carbocycle aromatique monocyclique ou bicyclique condensé en C₃ à C₁₀, qui est facultativement substitué avec un ou plusieurs substituants choisis entre des substituants halogéno, O(alkyle en C₁ à C₄) non substitué et alkyle en C₁ à C₄ non substitué ou substitué avec 1 à 3 substituants halogéno ;
c) un groupe -(CH₂)ₘ-Ar dans lequel m est égal à 0 et dans lequel Ar représente un carbocycle aromatique monocyclique ou bicyclique condensé en C₃ à C₁₀, qui est non substitué ou substitué avec 1 à 3 substituants choisis entre des substituants halogéno, O(alkyle en C₁ à C₄) non substitué et alkyle en C₁ à C₄ non substitué ou substitué avec 1 à 3 substituants halogéno ; ou
d) éthoxycarbonylméthyle,
ou un de ses sels, produits de solvatation ou esters pharmaceutiquement acceptables.

2. Composé suivant la revendication 1, dans lequel
R représente H ou un groupe cladinosyle de formule (II) 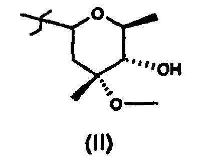
R¹ représente H, un groupe β-cyanoéthyle, β-amidoéthyle ou β-((alkoxy en C₁ à C₄)carbonyl)éthyle ;
R² représente
a) un groupe alkyle en C₁ à C₁₂, ledit groupe alkyle en C₁ à C₁₂ étant
linéaire ou ramifié, et non substitué ou substitué avec 1 à 3 groupes choisis entre des groupes halogéno, N,N-di(alkyle en C₁ à C₄)amino, C(O)O-(alkyle en C₁ à C₄)aryle, (alkyle en C₁ à C₄)thio ; ou phényle, naphtyle, aryle, furyle, cycloalkyle, thiophényle, 3,4-méthylènedioxyphényle, morpholinyle ou pipéridinyle facultativement substitué avec un ou plusieurs substituants choisis entre des substituants halogéno, alkyle en C₁ à C₄ non substitué ou substitué avec 1 à 3 substituants halogéno, O(alkyle en C₁ à C₄), O(cycloalkyle en C₃ à C₆), O((alkyle en C₁ à C₄)aryle), C(O)(alkyle en C₁ à C₄), (alkyle en C₁ à C₄)oxycarbonyle, aryle facultativement substitué, hétéroaryle facultativement substitué, C(O)(alkyle en C₁ à C₄)aryle, C(O)O-(alkyle en C₁ à C₄) aryle;
b) un groupe alcényle en C₂ à C₆ non substitué ; ou
c) un groupe cycloalkyle en C₁ à C₁₂, adamantyle, norbornyle, norbornényle, phényle, indanyle ou naphtyle ; n'importe lequel de ces groupes étant non substitué ou substitué avec 1 à 3 groupes choisis entre des groupes halogéno ; CN ; alkyle en C₁ à C₄ non substitué ou substitué avec 1 à 3 substituants halogéno ou groupes CN ; O(alkyle en C₁ à C₄) facultativement substitué avec 1 à 3 substituants halogéno ; S(alkyle en C₁ à C₄) ; O(cycloalkyle en C₃ à C₆) ; O((alkyle en C₁ à C₄)aryle) ; C(O)(alkyle en C₁ à C₄) ; (alkyle en C₁ à C₄)-oxycarbonyle ; aryle ; hétéroaryle : NO₂ ; N,N-di-(alkyle en C₁ à C₄)amino, diazoaryle ; et pipéridinyl-sulfonamido ; ou
d) un groupe dihydrobenzofurannyle, (alkylène en C₁ à C₃)dioxyphényle, benzopyrannyle, furyle, isoxazolyle, pipéridinyle, pyridinyle, thiophényle, benzothiadiazolyle, tétrahydrobenzothiophényle, facultativement substitué avec 1 à 3 groupes choisis entre des groupes halogéno ; alkyle en C₁ à C₄ non substitué ou substitué avec 1 à 3 substituants halogéno ; (alkyle en C₁ à C₄)-oxycarbonyle ; aryle facultativement substitué : hétéroaryle facultativement substitué ; C(O)O(alkyle en C₁ à C₄)aryle ; ou phénoxy ;
e) un groupe C(O)aryle ;
X représente O ou S ; et
n est égal à 2 ou 3.

3. Composé suivant la revendication 2, dans lequel
R représente H ou un groupe cladinosyle de formule (II) 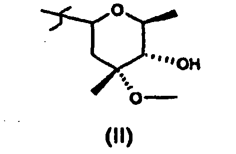
R¹ représente H, un groupe β-cyanoéthyle, β-amidoéthyle ou β-((alkoxy en C₁ à C₄)carbonyl)éthyle ;
R² représente
a) un groupe 3-phénylpropyle, β-phényléthyle, éthoxycarbonylméthyle, isopropyle, 1-(1-naphtyl)éthyle, tertiobutyle, n-butyle, sec.-butyle, benzyle, 2-furylméthyle, 4-méthoxybenzyle, cyclohexylméthyle, éthyle, 2-(2-méthyl-5,5-diméthyl)-pentyle, 2-(2-thiophényl)-éthyle, 3-thiométhylpropyle, 3,4-méthylènedioxyphénylméthyle, N-morpholinyléthyle, N-morpholinylpropyle, trityle, N-pipéridinyléthyle, 3-diéthylaminopropyle, diphénylméthyle, 3-chloropropyle, isobutyle ; ou
b) un groupe 2-propényle ; ou
c) un groupe cyclopentyle, cyclopropyle, cyclododécyle, norbornyle, norbornényle, 2-benzyloxycyclohexyle, adamantyle, phényle, 1-naphtyle, 4-chlorophényle, 2-trifluorométhylphényle, 3,4,5-triméthoxyphényle, 2-naphtyle, 2,4-dichlorophényle, 4-cyanophényle, cyclohexyle, 4-éthylphényle, 4-méthoxyphényle, 2-méthyl-5-fluorophényle, 4-cyanométhylphényle, indanyle, 4-acétylphényle, 2-phénylphényle, 3-thiométhylphényle, 3,5-diméthoxycarbonylphényle, 4-méthylphényle, 2,4-diméthylphényle, 3,4-difluorophényle, 3-chlorophényle, 3-fluorophényle, 3-cyclopentoxy-4-méthoxyphényle, 4-benzyloxyphényle, 2-éthylphényle, 2,6-difluorophényle, 4-nitrophényle, 3,5-dichlorophényle, 2-méthoxy-4-nitrophényle, éthoxycarbonylphényle, 2-trifluorométhylphényle, 4-phénylazophényle, 4-diéthylaminophényle, 3-nitrophényle, 3-chloro-4-trifluorométhylphényle, 3,4-dichlorophényle, 2,3,4-trifluorophényle, 4-bromophényle, 4-diazolylphényle, 4-pipéradylsulforiamidophényle, 1-(4-diméthylamino)-naphtyle, 4-isopropylphényle, 4-difluorométhoxyphényle ou 2-méthoxy-5-phénylphényle ; ou
d) un groupe 3,4-méthylènedioxyphényle, 6-fluorobenzo-1,3-pyrannyle, dihydrobenzofurannyle, 3,4-propylènedioxyphényle, 3-(2-trifluorométhyl-5-méthyl)-furyle, 4-(3,5-diméthyl)-isoxazole, 4-(3-phényl-5-méthyl)-isoxazole, benzyloxycarbonylpipéridinyle, 4-(2,6-dichloro)-pyridinyle, 2-thiophényle, benzothiadiazolyle, 3-(2-méthoxycarbonyl)-thiophényle, 2-(3-méthoxycarbonyl)-tétrahydrobenzothiophényle, pyridinyle, 5-(2-morpholinyl)-pyridinyle, 5-(2-phénoxy)-pyridinyle ; ou
e) un groupe C(O)aryle ;
X représente 0 ou S ; et
n est égal à 2 ou 3.

4. Procédé pour la préparation du composé de formule (I) 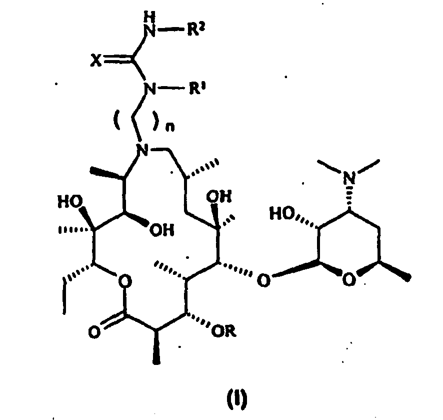 dans laquelle
R représente H ou un groupe cladinosyle de formule (II) : 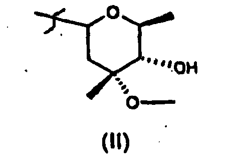
R¹ représente H, un groupe β-cyanoéthyle, β-amidoéthyle ou β-((alkoxy en C₁ à C₄)carbonyl)éthyle ;
R² représente
a) un groupe alkyle en C₁ à C₁₂, ledit groupe alkyle en C₁ à C₁₂ étant
i) non interrompu ou interrompu avec 1 à 3 groupes consistant en radicaux bivalents choisis entre des groupes -O-, -S- et -N(R³)- ; et/ou
ii) non substitué ou substitué avec 1 à 3 groupes choisis entre des groupes halogéno ; OH ; NH₂ ; N-(alkyle en C₁ à C₄)amino ; N,N-di(alkyle en C₁ à C₄)amino ; CN, NO₂ ; C(O)O-(alkyle en C₁ à C₄)-aryle ; un carbocycle en C₃ à C₁₄ facultativement substitué avec un ou plusieurs substituants choisis entre des substituants halogéno, CN, alkyle en C₁ à C₄ non substitué ou substitué avec 1 à 3 substituants halogéno, O(alkyle en C₁ à C₄) facultativement substitué avec 1 à 3 substituants halogéno, S(alkyle en C₁ à C₄), O(cycloalkyle en C₃ à C₆), O((alkyle en C₁ à C₄)aryle), (alkyle en C₁ à C₄)cyano, C(O)(alkyle en C₁ à C₄), (alkyle en C₁ à C₄)oxycarbonyle, aryle facultativement substitué hétéroaryle facultativement substitué, C(O)(alkyle en C₁ à C₄)aryle, C(O)O(alkyle en C₁ à C₄)aryle, NO₂, diazoaryle, sulfo-(noyau carbocyclique ou hétérocyclique penta- ou hexagonal), (alkyle en C₁ à C₄)-C(O)-O-(alkyle en C₁ à C₄) et (alkyle en C₁ à C₄)O-C(O)-NR³ ; un hétérocycle en C₃ à C₁₄ saturé, insaturé ou aromatique contenant 1 à 3 hétéroatomes choisis dans le groupe comprenant l'azote, l'oxygène et le soufre facultativement substitué avec un substituant halogéno, CN, alkyle en C₁ à C₄ non substitué ou substitué avec 1 à 3 substituants halogéno, O(alkyle en C₁ à C₄) facultativement substitué avec 1 à 3 substituants halogéno, S(alkyle en C₁ à C₄), O(cycloalkyle en C₃ à C₆), O((alkyle en C₁ à C₄)aryle), (alkyle en C₁ à C₄)cyano, C(O)(alkyle en C₁ à C₄), C(O)O-(alkyle en C₁ à C₄)aryle ; (alkyle en C₁ à C₄)-oxycarbonyle, aryle facultativement substitué, hétéroaryle facultativement substitué, C(O)(alkyle en C₁ à C₄)aryle, NO₂, diazoaryle, sulfo-(noyau carbocyclique ou hétérocyclique penta- ou hexagonal), (alkyle en C₁ à C₄)C(O)-O-(alkyle en C₁ à C₄) et (alkyle en C₁ à C₄)O-C(O)-NR³ ; ou
b) un groupe alcényle en C₂ à C₆ contenant 0, 1, 2 ou 3 hétéroatomes choisis entre O, S et N, facultativement substitué avec un ou plusieurs substituants choisis entre des substituants halogéno ; CN ; NO₂ ; OH ; NH₂ ; N-(alkyle en C₁ à C₄)amino ; N,N-di(alkyle en C₁ à C₄)-amino ; aryle facultativement substitué ; hétéroaryle facultativement substitué ; ou
c) un carbocycle en C₃ à C₁₄ qui est non substitué ou substitué avec 1 à 3 groupes choisis entre des groupes halogéno ; OH ; CN ; alkyle en C₁ à C₄ non substitué ou substitué avec 1 à 3 substituants halogéno ou groupes CN ; O(alkyle en C₁ à C₄) facultativement substitué avec 1 à 3 substituants halogéno ; S(alkyle en C₁ à C₄) ; O(cycloalkyle en C₃ à C₆) ; O((alkyle en C₁ à C₄)aryle) ; C(O)(alkyle en C₁ à C₄) ; C(O)O(alkyle en C₁ à C₄)aryle ; (alkyle en C₁ à C₄)oxycarbonyle ; aryle facultativement substitué ; hétéroaryle facultativement substitué ; C(O)(alkyle en C₁ à C₄)aryle ; NO₂ ; diazoaryle ; sulfo-(noyau carbocyclique ou hétérocyclique penta- ou hexagonal) ; (alkyle en C₁ à C₄)C(O)-O-(alkyle en C₁ à C₄) et (alkyle en C₁ à C₄)OC(O)-NR³ ; ou
d) un hétérocycle en C₃ à C₁₄ saturé, insaturé ou aromatique contenant 1 à 3 hétéroatomes choisis dans le groupe comprenant l'azote, l'oxygène et le soufre facultativement substitué avec 1 à 3 groupes choisis entre des groupes halogéno ; CN ; alkyle en C₁ à C₄ non substitué ou substitué avec 1 à 3 substituants halogéno ; O(alkyle en C₁ à C₄) facultativement substitué avec 1 à 3 substituants halogéno ; S(alkyle en C₁ à C₄) ; O(cycloalkyle en C₃ à C₆) : O((alkyle en C₁ à C₄)aryle) ; (alkyle en C₁ à C₄)cyano ; C(O)(alkyle en C₁ à C₄) ; (alkyle en C₁ à C₄)oxycarbonyle ; aryle facultativement substitué ; hétéroaryle facultativement substitué ; C(O)(alkyle en C₁ à C₄)aryle ; C(O)O-(alkyle en C₁ à C₄)aryle ; NO₂ ; diazoaryle ; un noyau carbocyclique ou hétérocyclique penta- ou hexagonal ; sulfo-(noyau carbocyclique ou hétérocyclique penta- ou hexagonal) ; (alkyle en C₁ à C₄)-C(O)-O-(alkyle en C₁ à C₄) et (alkyle en C₁ à C₄)O-C(O)-NR³ ;
e) un groupe C(O)aryle ;
R³ représente H ou un groupe alkyle en C₁ à C₄ ;
X représente O ou S ;
n est égal à 2 ou 3
où l'expression "carbocyle en C₃ à C₁₄" désigne n'importe quel noyau monocyclique, bicyclique ou tricyclique stable de 3,4,5,6,7,8,9,10,11,12,13 ou 14 membres, n'importe lequel de ces noyaux pouvant être saturé, insaturé ou aromatique, condensé ou ponté, en sachant que des noyaux avec certains nombres de membres ne peuvent être bicycliques ou tricycliques et que lorsque le noyau est ponté, les substituants indiqués pour le noyau peuvent également être présents sur le pont ;
sous réserve que, lorsque R¹ représente H ou un groupe β-cyanoéthyle et n est égal à 3 ; R² ne peut être :
a) un groupe alkyle en C₁ à C₄ linéaire ou ramifié non interrompu et non substitué ;
b) un groupe -(CH₂)ₘ-Ar dans lequel m a une valeur de 1 à 3 et dans lequel Ar représente un carbocycle aromatique monocyclique ou bicyclique condensé en C₃ à C₁₀, qui est facultativement substitué avec un ou plusieurs substituants choisis entre des substituants halogéno, O(alkyle en C₁ à C₄) non substitué et alkyle en C₁ à C₄ non substitué ou substitué avec 1 à 3 substituants halogéno ;
c) un groupe -(CH₂)ₘ-Ar dans lequel m est égal à 0 et dans lequel Ar représente un carbocycle aromatique monocyclique ou bicyclique condensé en C₃ à C₁₀, qui est non substitué ou substitué avec 1 à 3 substituants choisis entre des substituants halogéno, O(alkyle en C₁ à C₄) non substitué et alkyle en C₁ à C₄ non substitué ou substitué avec 1 à 3 substituants halogéno ; ou
d) éthoxycarbonylméthyle
ou d'un de ses sels, produits de solvatation ou esters pharmaceutiquement acceptables, dans lequel un composé de formule (III) 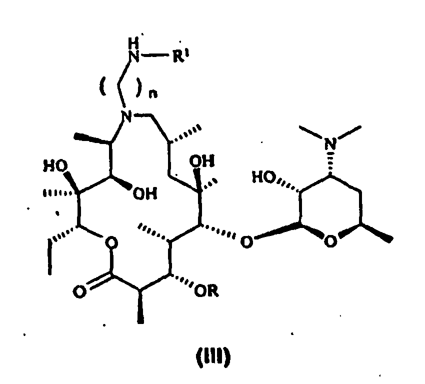 est amené à réagir avec un isocyanate ou un thioisocyanate de formule (IV)
**R²-N=C=X** **(IV)**
dans un solvant aprotique choisi entre le toluène, le xylène et le dichlorométhane, à une température d'environ 0°C à 110°C.

5. Utilisation d'un composé suivant la revendication 1 ou d'un de ses sels, produits de solvatation ou esters pharmaceutiquement acceptables dans la production d'un médicament destiné au traitement thérapeutique et/ou prophylactique du paludisme.

6. Utilisation suivant la revendication 5, dans laquelle le sujet a été infecté par *Plasmodium falciparum.*

7. Utilisation suivant la revendication 5, dans laquelle le sujet a été infecté par *P. vivax.*

8. Utilisation suivant la revendication 5, dans laquelle le sujet a été infecté par *P. ovale.*

9. Utilisation suivant la revendication 5, dans laquelle le sujet a été infecté par *P. malariae*.

10. Utilisation suivant la revendication 5, dans laquelle le médicament est administré après exposition du sujet au parasite du paludisme.

11. Utilisation suivant la revendication 5, dans laquelle le médicament est administré avant que le sujet ne voyage dans un pays où le paludisme est endémique.

12. Utilisation suivant la revendication 11, dans laquelle le paludisme est provoqué par une souche d'agent du paludisme résistante aux médicaments.

13. Utilisation suivant la revendication 12, dans laquelle la souche d'agent du paludisme résistante aux médicaments est résistante à au moins un des médicaments consistant en chloroquine, méfloquine, halofantrine, artémisinine, atovaquone/proguanil, doxycycline et primaquine.

14. Utilisation suivant la revendication 5, dans laquelle le traitement thérapeutique et/ou prophylactique est effectué chez un être humain.

15. Préparation pharmaceutique comprenant le composé de la revendication 1 ou un de ses sels, produits de solvatation ou esters pharmaceutiquement acceptables et au moins un support pharmaceutiquement acceptable.

16. Préparation pharmaceutique suivant la revendication 15, qui est destinée au traitement d'infections par le parasite du paludisme.
